# EUROPÄISCHE PATENTSCHRIFT

(11) **EP 0 975 616 B1**
(45) Veröffentlichungstag und Bekanntmachung des Hinweises auf die Patenterteilung: **13.02.2002**
(21) Anmeldenummer: 98913750.0
(22) Anmeldetag: 02.04.1998
(51) Int. Cl.: C07D 303/36, C07D 203/12, C07C 251/60, C07C 271/28, C07C 251/42, A01N 43/04, A01N 43/34, A01N 47/20, A01N 37/18, A01N 37/10

(54) **SUBSTITUIERTE BENZYLOXYIMINO-VERBINDUNGEN**
SUBSTITUTED BENZYL OXIMINO COMPOUNDS
COMPOSES DU TYPE BENZYLO-OXYMINO SUBSTITUES

(30) Priorität: 18.04.1997 DE 19716237
(43) Veröffentlichungstag der Anmeldung: 02.02.2000
(73) Patentinhaber: BASF AKTIENGESELLSCHAFT, 67056 Ludwigshafen (DE)
(72) Erfinder: GRAMMENOS, Wassilios, D-67063 Ludwigshafen (DE); SAUTER, Hubert, D-68167 Mannheim (DE); BAYER, Herbert, D-68159 Mannheim (DE); GROTE, Thomas, D-67105 Schifferstadt (DE); GYPSER, Andreas, D-68159 Mannheim (DE); KIRSTGEN, Reinhard, D-67434 Neustadt (DE); MÜLLER, Bernd, D-67227 Frankenthal (DE); PTOCK, Arne, D-67067 Ludwigshafen (DE); RÖHL, Franz, D-67105 Schifferstadt (DE); GÖTZ, Roland, D-91541 Rothenburg (DE); LORENZ, Gisela, D-67434 Neustadt (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(74) Vertreter: Fitzner, Uwe, Dr.
(86) Internationale Anmeldenummer: EP9801942
(87) Internationale Veröffentlichungsnummer: WO9847886

(56) Entgegenhaltungen:
- EP-A- 0 129 889
- EP-A- 0 463 488
- WO-A-93/15046
- CH-A- 592 641
- H. LANGHALS ET AL: CHEM. BER., Bd. 114, Nr. 12, 1981, Seiten 3831-3854, XP002073304
- C. SANTELLI: TETRAHEDRON LETT., Bd. 21, Nr. 30, 1980, Seiten 2893-2896, XP002073305
- "BEILSTEINS HANDBUCH DER ORGANISCHEN CHEMIE, 4. Auflage, 3. und 4. Ergänzungswerk, Band 18, Teil 5" 1976 , SPRINGER-VERLAG , BERLIN, DE XP002073306 siehe Seite 4364, Formel VII-VIII und Seite 4365, Zeilen 3-16

## Beschreibung

Die vorliegende Erfindung betrifft Benzyloxyimino-Verbindungen der Formel I, 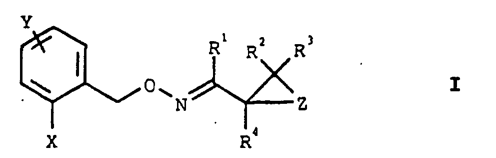 in der die Substituenten die folgenden Bedeutungen haben:
- X: N(COOCH₃)-OCH₃, C(COOCH₃)=CH-OCH₃, C(COOCH₃)=N-OCH₃, C(CONHCH₃)=N-OCH₃ oder C(COOCH₃)=CH-CH₃;
- Y: Wasserstoff, Halogen, C₁-C₄-Alkyl, C₁-C₄-Halogenalkyl oder C₁-C₄-Alkoxy;
- Z: Sauerstoff, C(R^{a})₂ oder NR^{a}, wobei
R^{a} für Wasserstoff oder C₁-C₄-Alkyl steht und im Fall C(R^{a})₂ gleich oder verschieden sein können;
- R¹: Halogen, C₁-C₄-Alkyl, C₁-C₄-Halogenalkyl oder C₁-C₄-Alkoxy;
- R2: Wasserstoff, Halogen, C₁-C₄-Alkyl, C₁-C₄-Alkoxy oder gegebenenfalls durch übliche Gruppen substituiertes Phenyl;
- R3: Wasserstoff, Halogen, C₁-C₄-Alkyl oder C₁-C₄-Alkoxy;
- R⁴: C₁-C₄-Alkyl oder
Phenyl, das gegebenenfalls 1-3 Substituenten aus der folgenden Gruppe trägt: Halogen, C₁-C₄-Alkyl, C₁-C₄-Halogenalkyl, C₁-C₄-Alkoxy, C₁-C₄-Halogenalkoxy oder C(R^{b})=N-OR^{c}, wobei
Rb für Wasserstoff oder C₁-C₄-Alkyl und
R^{c} für C₁-C₄-Alkyl, C₃-C₄-Alkenyl oder C₃-C₄-Alkinyl steht.

Zusätzlich betrifft die Erfindung Verfahren und Zwischenprodukte zur Herstellung der Verbindungen I, sowie Mittel und die Verwendung der Verbindungen I zur Bekämpfung von Schadpilzen und tierischen Schädlingen.

Phenylcarbamate mit orthoständiger Methoximino-Gruppe werden in WO-A 93/15,046 offenbart. α-Phenylacrylsäure- und α-Phenyl-α-methoximinoessigsäurederivate mit orthoständiger Methoximino-Gruppierung sind aus EP-A 426 460, EP-A 460 575, EP-A 472 300, EP-A 585 751, WO-A 92/13,830, WO-A 92/18,487, WO-A 92/18,494, JP-A 05/201,946 und JP-A 05/255,012 bekannt. Die dort beschriebenen Verbindungen tragen in der Orthoposition zu der X entsprechenden Gruppe eine CH₂O-N=CR'R"-Gruppierung. Die allgemeinen Definitionen der Reste R' und R" umfassen die Definition des Restes R¹ in Formel I und lassen zusätzlich Cycloalkyl und z. T. Heterocyclyl als Substituent zu.

Durch EP-A 463 488 und WO-A 90/07,493 werden solche α-Phenylacrylsäure- und α-Phenyl-α-methoximinoessigsäurederivate beispielhaft offenbart, in denen die CH₂O-N=CR'R"-Gruppierung als Substituenten R' und R" Alkyl- und Cyclopropyl-, Oxiranyl-, bzw. Aziridinyreste trägt.

Die in den vorstehend genannten Schriften beschriebenen Verbindungen sind als Pflanzenschutzmittel gegen Schadpilze und z. T. gegen tierische Schädlinge geeignet.

Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die substituierten Benzyloxyimino-Verbindungen der Formel I gefunden. Weiterhin wurden Zwischenprodukte und Verfahren zur Herstellung der Verbindungen I, sowie die Verwendung der Verbindungen I und diese enthaltene Mittel zur Bekämpfung von Schadpilzen und tierischen Schädlingen gefunden. Die fungizide Wirkung ist bevorzugt.

Die Verbindungen der Formel I unterscheiden sich von den aus den oben genannten Schriften EP-A 463 488 und WO-A 90/07,493 bekannten Verbindungen in der Substitution der Methoximinogruppe durch den Rest R⁴, der nicht Wasserstoff sein kann. Die Verbindungen der Formel I weisen eine gegenüber den bekannten Verbindungen erhöhte Wirksamkeit gegen Schadpilze und tierische Schädlinge auf.

Die Verbindungen der Formel I können an sich nach den in EP-A 463 488 und WO-A 90/07,493 beschriebenen Methoden erhalten werden.

Insbesondere erhält man die Verbindungen der Formel I, in denen Z für Sauerstoff und C(R^{a})₂ steht, dadurch, daß man ein Oxim der Formel II mit einer Benzylverbindung der Formel III umsetzt.

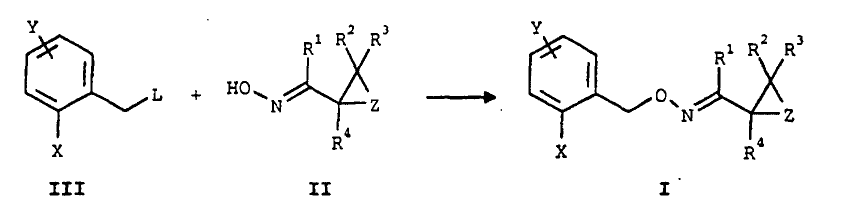

In Formel III steht L für eine nucleophil austauschbare Gruppe, beispielsweise Halogen, wie Fluor, Chlor, Brom oder Jod, insbesondere Chlor oder Brom, oder Alkyl- oder Arylsulfonate, wie Mesylat oder Tosylat.
1. Die Umsetzung des Oxims II mit der Benzylverbindung III erfolgt in bekannter Weise bei Temperaturen von -10°C bis 80°C, vorzugsweise 0°C bis 65°C, in einem inerten organischen Lösungsmittel in Gegenwart einer Base [vgl. EP-A 463 488].
   Die Edukte werden im allgemeinen in äquimolaren Mengen miteinander umgesetzt. Es kann für die Ausbeute vorteilhaft sein, II in einem Überschuß bezogen auf III einzusetzen.
   Die für die Herstellung der Verbindungen I benötigten Benzylverbindungen der Formel III sind aus der Literatur bekannt [EP-A 420 091; EP-A 513 580; WO-A 93/15,046; WO-A 94/05,620] und können gemäß der zitierten Literatur hergestellt werden.
   Die für die Herstellung der Verbindungen I, in denen Z für Sauerstoff steht, benötigten Ausgangsstoffe IIA können auf dem folgenden Weg hergestellt werden. 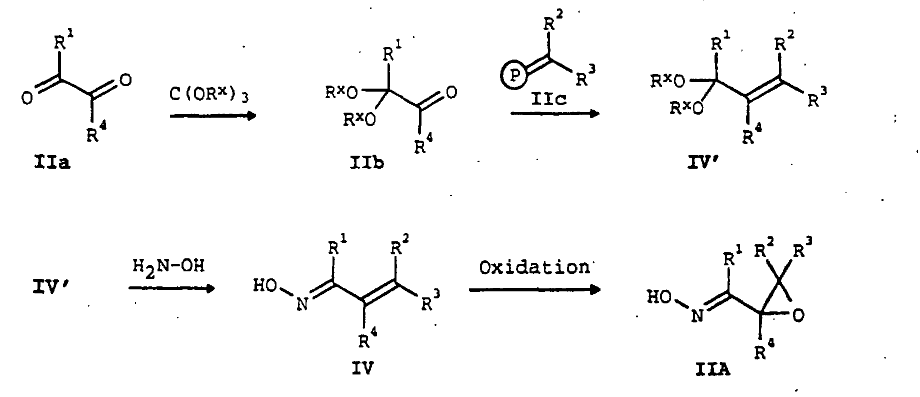
2A. In dem voranstehenden Reaktionsschema steht R^{x} für einen C₁-C₁₀-Alkylrest und Ⓟ in der Formel IIc für einen Phosphoranylrest, wie beispielsweise Triphenylphosphoranyl. Die Acetalisierung des α,β-Diketons IIa zu IIb erfolgt üblicherweise bei Temperaturen von -20°C bis 120°C, vorzugsweise 0°C bis 85°C, in einem inerten organischen Lösungsmittel in Gegenwart einer Säure [vgl. Khim.-Farm. Zh., S. 606 (1986); J. Chem. Res. Miniprint, S. 2528 (1982)].
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Toluol, Methanol, Ethanol und Dimethylacetamid. Es können auch Gemische der genannten Lösungsmittel verwendet werden.
   Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung.
   Die Säuren werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, C(OR^{x})₃ in einem Überschuß bezogen auf IIa einzusetzen.
   Diketone IIa sind kommerziell erhältlich oder in der Literatur bekannt [J. Am. Chem. Soc., S. 12588 (1996); J. Chem. Soc. Chem. Commun., S. 692 (1987); Khim.-Farm. Zh., S. 606 (1986)] oder können gemäß der zitierten Literatur hergestellt werden.
2B. Die Verbindung IIb wird mit einem Phosphoran der Formel IIc in einer Wittig-Reaktion in das α,β-ungesättigte Acetal IV' übergeführt.
   Die Wittig-Reaktion erfolgt üblicherweise bei Temperaturen von -78°C bis 85°C, vorzugsweise -10°C bis 65°C, in einem inerten organischen Lösungsmittel in Gegenwart einer Base [vgl. EP-A 513 5801.
   Geeignete Lösungsmittel sind aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylsulfoxid, Dimethylformamid und Dimethylacetamid, besonders bevorzugt Tetrahydrofuran, tert. Butyl-methylether, Dimethylformamid und Dimethylacetamid. Es können auch Gemische der genannten Lösungsmittel verwendet werden.
   Als Basen kommen allgemein anorganische Verbindungen wie Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, metallorganische Verbindungen, insbesondere Alkalimetallalkyle wie Methyllithium, Butyllithium und Phenyllithium, Alkylmagnesiumhalogenide wie Methylmagnesiumchlorid, sowie Alkalimetall- und Erdalkalimetallalkoholate wie Natriummethanolat, Natriummethanolat, Kaliummethanolat, Kalium-tert.-Butanolat und Dimethoxymagnesium, in Betracht. Besonders bevorzugt werden Kalium-tert. Butylat, Natrium-tert. Butylat, Natriummethanolat, Kaliummethanolat und Natriumhydroxid.
   Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, IIc in einem Überschuß bezogen auf IIb einzusetzen.
   Die Phosphorane der Formel IIc sind kommerziell erhältlich oder in der Literatur bekannt [vgl. Liebigs Ann. Chem., S. 708 (1967); J. Org. Chem., S 4860 (1983)] oder können gemäß der zitierten Literatur hergestellt werden.
2C. Das α,β-ungesättigte Acetal der Formel IV' wird mit Hydroxylamin, oder einem seiner Säureadditionssalze, zu dem Oxim der Formel IV üblicherweise bei Temperaturen von -10°C bis 86°C, vorzugsweise 0°C bis 65°C, in einem inerten organischen Lösungsmittel gegebenenfalls in Gegenwart einer Base [vgl. Journal of Organic Chemistry, S. 759 (1993); Helv. Chim. Acta, S. 1546 (1960)] umgesetzt. Geeignete Lösungsmittel sind Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, besonders bevorzugt Methanol und Ethanol. Es können auch Gemische der genannten Lösungsmittel verwendet werden.
   Als Basen kommen allgemein organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin, sowie bicyclische Amine in Betracht. Besonders bevorzugt werden Triethylamin und Pyridin.
   Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, Hydroxylamin in einem Überschuß bezogen auf IV' einzusetzen.
2D. Das α,β-ungesättigte Oxim der Formel IV wird durch Oxidation in das Oxiran IIA übergeführt.
   Die Epoxidierung erfolgt üblicherweise bei Temperaturen von -78°C bis 45°C, vorzugsweise -20°C bis 30°C, in einem inerten organischen Lösungsmittel [vgl. Houben-Weyl, Methoden der organischen Chemie, Band 4/1a, 4. Auflage, S. 182 - 239, Georg Thieme Verlag, Stuttgart 1981].
   Als Oxidationsmittel kommen sauerstoffübertragende Verbindungen wie anorganische Peroxide, wie beispielsweise Wasserstoffperoxid, tert. Butylhydroperoxid oder organische Persäuren, wie z. B. Perbenzoesäure, 3-Chlor-perbenzoesäure oder Peressigsäure in Betracht. Sie werden im allgemeinen in äquimolaren Mengen miteinander umgesetzt. Es kann für die Ausbeute vorteilhaft sein, sie in einem 3- bis 5-fachen Überschuß bezogen auf die Verbindung IV einzusetzen.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, sowie Dimethylsulfoxid, Dimethylformamid und Dimethylacetamid, besonders bevorzugt Toluol, Methylenchlorid und Chlorbenzol. Es können auch Gemische der genannten Lösungsmittel verwendet werden.
   Die für die Herstellung der Verbindungen I, in denen Z für C(R^{a})₂ steht, benötigten Ausgangsstoffe IIB können auf den folgenden literaturbekannten Wegen hergestellt werden.
3. Das Cyclopropylcarbonsäurederivat der Formel IIf' [zur Herstellung siehe z. B. EP-A 459 285; Anal. Chem. **24**, S. 623 (1952), Journal of Organic Chemistry **31,** S. 1379 (1966)] kann zur Darstellung des Ketons der Formel IIf mit metallorganischen Reagenzien Mt-R¹, insbesondere Grignard- oder Lithiumverbindungen, umgesetzt werden [vgl. Bull. Chem. Soc. Jap. **68**, S. 341 (1995); J. Am. Chem. Soc. **76**, S. 2244 (1954); Chem. Ber. **114**, S. 3831 (1981); Helv. Chim. Acta **42**, S. 2394 (1959)]. 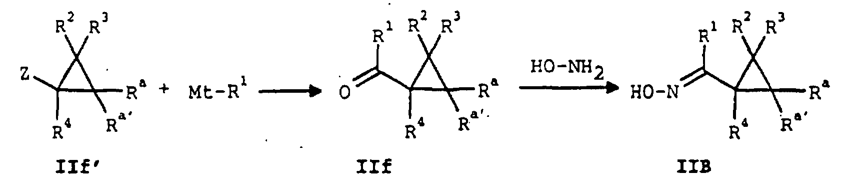 Z = CN oder 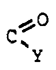 Y = Halogen, Mesylat, Tosylat, Alkoxy Weiterhin erhält man Verbindungen der Formel I, in denen Z für Sauerstoff steht, dadurch, daß man eine Benzylverbindung der Formel III mit einem Oxim der Formel IV zu einem Oximether der Formel V umsetzt, der anschließend durch Oxidation in eine Verbindung der Formel I übergeführt wird. 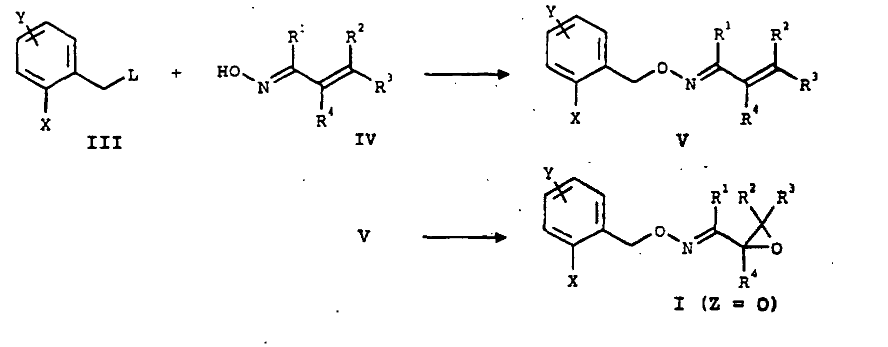
4. Die Herstellung des Oximethers V erfolgt in an sich bekannter Weise, üblicherweise bei Temperaturen von -20°C bis 85°C, vorzugsweise 0°C bis 65°C, in einem inerten organischen Lösungsmittel in Gegenwart einer Base [vgl. EP-A 463 488].
4A. Ein Syntheseweg für das für die Herstellung der Verbindungen V benötigte Oxim IV ist bereits bei der Synthese der Verbindungen IIA beschrieben worden.
4B. Die Oxidation des ungesättigten Oximethers V zu der Verbindung der Formel I, in der Z für Sauerstoff steht, erfolgt analog der Oxidation der Verbindung IV zu dem Oxiran IIA.
   Die Oxidation erfolgt üblicherweise bei Temperaturen von -78°C bis 45°C, vorzugsweise -20°C bis 30°C, in einem inerten organischen Lösungsmittel.
   Verbindungen der Formel I, in denen Z für NH steht, erhält man, in dem man Verbindungen der Formel I, in denen z für Sauerstoff steht, mit Metallaziden umsetzt. 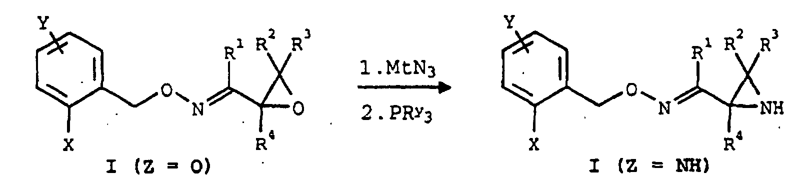
5. Diese Umsetzung erfolgt üblicherweise bei Temperaturen von -20°C bis 120°C, vorzugsweise -20°C bis 100°C, in einem inerten organischen Lösungsmittel [vgl. Journal of Heterocycl. Chemistry, S. 473 (1991) und Tetrahedron S. 5287 (1991)].
   In dem voranstehenden Reaktionsschema steht Mt für ein Ion aus der Gruppe der Alkali-, Erdalkali- oder Übergangsmetalle, oder Trialkylsilyl, beispielsweise Natrium oder Trimethylsilyl; R^{y} steht für einen C₁-C₁₀-Alkoxy-, insbesondere einen Ethoxyrest oder Aryl-, beispielsweise einen Phenylrest.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Ketone wie Aceton, Methylethylketon, Diethylketon und tert.-Butylmethylketon, sowie Dimethylsulfoxid, Dimethylformamid und Dimethylacetamid, oder Wasser, besonders bevorzugt Aceton, Dichlormethan, Toluol und Wasser/Aceton-Gemisch. Es können auch Gemische der genannten Lösungsmittel verwendet werden.
   Die Edukte werden im allgemeinen in äquimolaren Mengen miteinander umgesetzt. Es kann für die Ausbeute vorteilhaft sein, MtN₃ in einem Überschuß bezogen auf I (Z = O) einzusetzen.
   Darüber hinaus erhält man Verbindungen der Formel I, in denen Z für NH steht, in dem man Verbindungen der Formel V mit Ethyl-1-azido-N-(methylsulfonyl)formamidat umsetzt und die erhaltene Verbindung V' anschließend hydrolysiert. 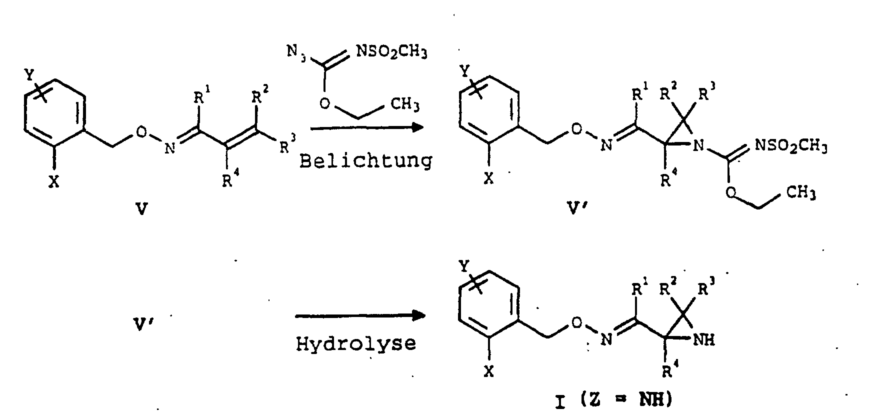
6A. Geeignete Reaktionsbedingungen für die Umsetzung des Oximethers V mit Ethyl-1-azido-N-(methylsulfonyl)formamidat sind in Journal of Organic Chemistry, S. 3945 (1989) genannt.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ketone wie Aceton, Methylethylketon, Diethylketon und tert.-Butylmethylketon, sowie Dimethylsulfoxid, Dimethylformamid und Dimethylacetamid, besonders bevorzugt Cyclohexan, Methylenchlorid, Toluol und Aceton. Es können auch Gemische der genannten Lösungsmittel verwendet werden.
   Die Edukte werden im allgemeinen in äquimolaren Mengen miteinander umgesetzt. Es kann für die Ausbeute vorteilhaft sein, Ethyl-1-azido-N-(methylsulfonyl)formamidat in einem 3-bis 10-fachen Überschuß bezogen auf V einzusetzen.
6B. Das für die Herstellung der Verbindung V' benötigte Ethyl-1-azido-N-(methylsulfonyl)formamidat ist in der Literatur bekannt [Journal of Organic Chemistry, S. 3945 (1989)] und kann gemäß der zitierten Literatur hergestellt werden.
   Die Hydrolyse der Verbindung V' zu dem Aziridin der Formel I, in der Z für NH steht, erfolgt üblicherweise bei Temperaturen von 0°C bis 85°C, vorzugsweise 25°C bis 65°C, in einem inerten organischen Lösungsmittel in Gegenwart einer Base [vgl. Journal of Organic Chemistry, S. 3945 (1989)].
   Geeignete Lösungsmittel sind Ether wie Dioxan, Anisol und Tetrahydrofuran, Nitrile wie Acetonitril und Propionitril, Ketone wie Aceton, Methylethylketon und Diethylketon, Alkohole wie Methanol, Ethanol, n-Propanol, Isopropanol, n-Butanol und tert.-Butanol, sowie Wasser, besonders bevorzugt Methanol, Ethanol und Wasser. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetallcarbonate wie Lithiumcarbonat, Kaliumcarbonat und Calziumcarbonat, sowie Alkalimetallhydrogencarbonate wie Natriumhydrogencarbonat, in Betracht. Besonders bevorzugt werden Natriumhydroxid und Kaliumhydroxid.
   Die Basen werden im allgemeinen in katalytischen Mengen eingesetzt, sie können aber auch äquimolar, im Überschuß oder gegebenenfalls als Lösungsmittel verwendet werden.
   Verbindungen der Formel I, in denen Z für N-C₁-C₄-Alkyl steht, erhält man dadurch, daß man Verbindungen der Formel I, in denen Z für NH steht, mit Alkylierungsmitteln umsetzt. 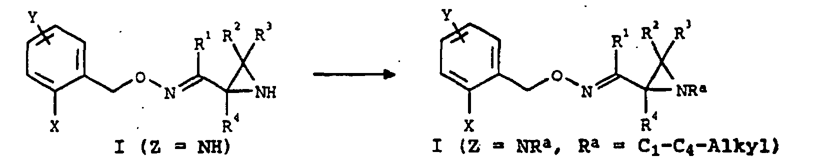
7. Die Alkylierung erfolgt üblicherweise bei Temperaturen von -30°C bis 120°C, vorzugsweise -10°C bis 100°C, in einem inerten organischen Lösungsmittel [vgl. Synth. Commun. S. 239 (1991)]
   Als Alkylierungsmittel kommen beispielsweise Alkylhalogenide, wie Methyljodid, Bromethan, oder Dialkylsufate, wie z. B. Dimethylsulfat, in Frage.
   Geeignete Lösungsmittel sind Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Tetrahydrofuran, Acetonitril, Aceton und Dimethylformamid. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkali-metall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat, Kaliumcarbonat und Calziumcarbonat, sowie Alkalimetallhydrogencarbonate wie Natriumhydrogencarbonat, Alkylmagnesiumhalogenide wie Methylmagnesiumchlorid, sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin, sowie bicyclische Amine in Betracht. Besonders bevorzugt werden NaHCO₃, K₂CO₃ und Triethylamin.
   Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, das Alkylierungsmittel in einem Überschuß bezogen auf I (Z = NH) einzusetzen. Der Zusatz von Tetrabutylammoniumhalogeniden, insbesondere Tetrabutylammoniumfluorid, kann ebenfalls für die Ausbeute vorteilhaft sein.
   Verbindungen der Formel I, in denen Z für Sauerstoff und X für C(=N-OCH₃)-CONHCH₃ steht, erhält man alternativ zu der im Kapitel 4A beschriebenen direkten Oxidation des Oximethers V, in dem man Verbindungen der Formel V, in denen X für C(=N-OCH₃)-COOCH₃ steht, unter allgemein üblichen Bedingungen [vgl. EP-A 398 692; EP-A 477 631] erst in das Carbonsäureamid Va und anschließend durch Oxidation in die Verbindung I überführt. 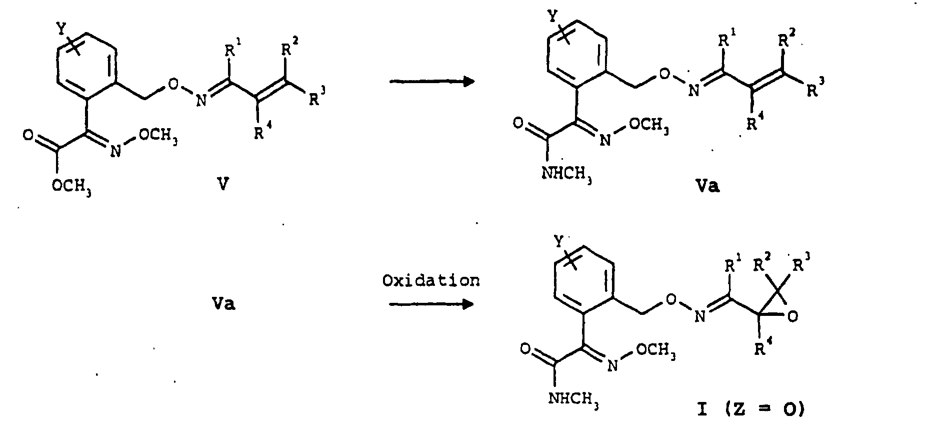
8. Die Oxidation von Va erfolgt unter den bei der Oxidation der Verbindung V im Kapitel 4A genannten Bedingungen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=C- und C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In der X entsprechenden Gruppe werden in Bezug auf die C=N- und C=C-Doppelbindung hinsichtlich ihrer Wirksamkeit die E-Isomere der Verbindungen I bevorzugt (Konfiguration bezogen auf die OCH₃ bzw. CH₃-Gruppe im Verhältnis zur Carbonylgruppe).

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**C**_{**1**}**-C**_{**4**}**-Alkyl:** gesättigte, geradkettige oder verzweigte Kohlenwasserstoffreste mit 1 bis 4 Kohlenstoffatomen, z.B. Methyl, Ethyl, Propyl, 1-Methylethyl, Butyl, 1-Methyl-propyl, 2-Methylpropyl und 1,1-Dimethylethyl;
**C**_{**1**}**-C**_{**4**}**-Halogenalkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt, vorzugsweise Fluor, Chlor und Brom, ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**C**_{**1**}**-C**_{**4**}**-Alkoxy:** geradkettige oder verzweigte Alkylgruppen mit 1 bis 4 Kohlenstoffatomen (wie vorstehend genannt), welche über ein Sauerstoffatom (-O-) an das Gerüst gebunden sind;
**C**_{**1**}**-C**_{**4**}**-Halogenalkoxy:** geradkettige oder verzweigte Halogenalkylgruppen mit 1 bis 4 Kohlenstoffatomen (wie vorstehend genannt), welche über ein Sauerstoffatom (-O-) an das Gerüst gebunden sind;
**C**_{**3**}**-C**_{**4**}**-Alkenyl:** ungesättigte, geradkettige oder verzweigte Kohlenwasserstoffreste mit 3 bis 4 Kohlenstoffatomen und einer Doppelbindung in einer beliebigen Position, z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl und 2-Methyl-2-propenyl;
**C**_{**3**}**-C**_{**4**}**-Alkinyl:** geradkettige oder verzweigte Kohlenwasserstoffgruppen mit 3 bis 4 Kohlenstoffatomen und einer Dreifachbindung in einer beliebigen Position, z.B. 1-Propinyl, 2-Propinyl, 1-Butinyl, 2-Butinyl, 3-Butinyl und 1-Methyl-2-propinyl;

Unter üblichen Gruppen sind in Bezug auf die Substitution des Phenylringes in der Position von R² insbesondere die folgenden Substituenten zu verstehen: Halogen, C₁-C₄-Alkyl und C₁-C₄-Halogenalkyl.

Im Hinblick auf die bestimmungsgemäße Verwendung der Verbindungen der Formel I sind die folgenden Bedeutungen der Substituenten, und zwar jeweils für sich allein oder in Kombination, besonders bevorzugt:

Verbindungen der Formel I, in denen X N(COOCH₃)-OCH₃ bedeutet, sind besonders bevorzugt.

Gleichermaßen bevorzugt sind Verbindungen I, in denen X für C(CONHCH₃)=N-OCH₃ steht.

Weiterhin bevorzugt sind Verbindungen I, in denen X C(COOCH₃)=CH-OCH₃, C (COOCH₃) =N-OCH₃ oder C(COOCH₃)=CH-CH₃ bedeutet.

Daneben sind Verbindungen I bevorzugt, in denen Y für Wasserstoff steht.

Für den Fall, daß Y nicht Wasserstoff bedeutet, sind Verbindungen I bevorzugt, worin Y für Halogen, C₁-C₂-Alkyl, C₁-C₂-Halogenalkyl oder C₁-C₂-Alkoxy, insbesondere für Fluor, Chlor, Methyl, Trifluormethyl oder Methoxy steht.

Darüber hinaus sind Verbindungen I für den Fall, daß Y nicht Wasserstoff bedeutet, bevorzugt, in denen Y in 3- oder in 6-Stellung steht.

Besonders bevorzugt sind Verbindungen I, in denen Z Sauerstoff bedeutet.

Gleichermaßen bevorzugt sind Verbindungen I, worin Z für NR^{a}, insbesondere NH oder NCH₃, steht.

Daneben sind Verbindungen I bevorzugt, in denen Z für C(R^{a})₂ steht.

Des weiteren sind Verbindungen I besonders bevorzugt, in denen R¹ für Halogen, insbesondere Fluor oder Chlor, steht.

Außerdem sind Verbindungen I bevorzugt, in denen R¹ für C₁-C₄-Alkyl, insbesondere Methyl, steht.

Gleichermaßen bevorzugt sind Verbindungen I, in denen R¹ für C₁-C₄-Halogenalkyl, insbesondere Trifluormethyl, steht.

Weiterhin bevorzugt sind Verbindungen I, in denen R² oder R³ Wasserstoff bedeutet.

Daneben sind Verbindungen I bevorzugt, in denen R² oder R³ für C₁-C₂-Alkyl stehen.

Außerdem sind Verbindungen I bevorzugt, in denen R² für Wasserstoff und R³ für C₁-C₄-Alkyl steht.

Insbesondere bevorzugt werden Verbindungen I, in denen R² durch übliche Gruppen substituiertes Phenyl bedeutet.

Für den Fall, daß Z Sauerstoff bedeutet, sind Verbindungen I bevorzugt, in denen R² Wasserstoff, und R³ C₁-C₄-Alkyl oder durch übliche Gruppen substituiertes Phenyl bedeuten.

Gleichermaßen bevorzugt sind Verbindungen I, worin R⁴ für Phenyl steht, das gegebenenfalls 1-3 Substituenten aus der folgenden Gruppe trägt: Halogen, C₁-C₄-Alkyl, C₁-C₄-Halogenalkyl, C₁-C₄-Alkoxy, C₁-C₄-Halogenalkoxy oder C(R^{b})=N-OR^{c} steht.

Weiterhin bevorzugt sind Verbindungen I, worin R⁴ für gegebenenfalls durch übliche Gruppen in 4-Stellung substituiertes Phenyl steht.

Die besonders bevorzugten Ausführungsformen der Zwischenprodukte in Bezug auf die Variablen entsprechen denen der Reste R¹, R², R³ und R⁴ der Formel I.

Insbesondere werden Verbindungen der Formel Ia.1 bevorzugt, in denen Y für Wasserstoff, 6-Methyl, 6-Chlor oder 3- oder 6-Fluor steht, R¹ Methyl, Ethyl, Chlor, Trifluormethyl oder Methoxy, R² Wasserstoff oder Methyl, R³ Methyl, Ethyl, n- oder iso-Propyl oder n- oder tert.-Butyl und R⁴ Methyl, Ethyl, n- oder iso-Propyl, n- oder tert.-Butyl oder ggf. durch Halogen, Methyl, Methoxy oder Alkoxyiminomethylen substituierte's Phenyl bedeuten.

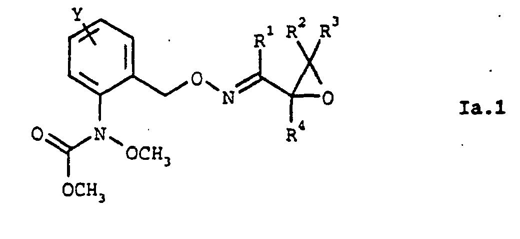

Außerdem werden Verbindungen der Formel Ia.2 bevorzugt, in denen Y für Wasserstoff, 6-Methyl, 6-Chlor oder 3- oder 6-Fluor steht, R¹ Methyl, Ethyl, Chlor, Trifluormethyl oder Methoxy, R² Wasserstoff oder Methyl, R³ Methyl, Ethyl, n- oder iso-Propyl oder n- oder tert.-Butyl und R⁴ Methyl, Ethyl, n- oder iso-Propyl, n- oder tert.-Butyl oder ggf. durch Halogen, Methyl, Methoxy oder Alkoxyiminomethylen substituiertes Phenyl bedeuten.

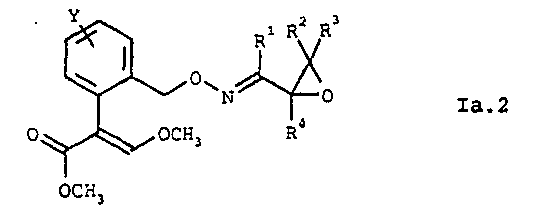

Gleichermaßen besonders bevorzugt sind Verbindungen der Formel Ia.3, in denen Y für Wasserstoff, 6-Methyl, 6-Chlor oder 3- oder 6-Fluor steht, R¹ Methyl, Ethyl, Chlor, Trifluormethyl oder Methoxy, R² Wasserstoff oder Methyl, R³ Methyl, Ethyl, n- oder iso-Propyl oder n- oder tert.-Butyl und R⁴ Methyl, Ethyl, n- oder iso-Propyl, n- oder tert.-Butyl oder ggf. durch Halogen, Methyl, Methoxy oder Alkoxyiminomethylen substituiertes Phenyl bedeuten.

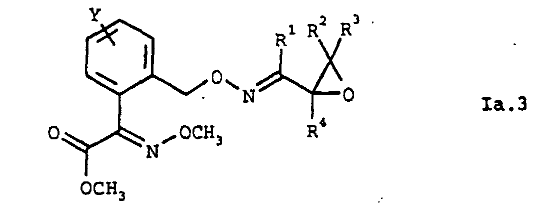

Daneben werden Verbindungen der Formel Ia.4 besonders bevorzugt, in denen Y für Wasserstoff, 6-Methyl, 6-Chlor oder 3- oder 6-Fluor steht, R¹ Methyl, Ethyl, Chlor, Trifluormethyl oder Methoxy, R² Wasserstoff oder Methyl, R³ Methyl, Ethyl, n- oder iso-Propyl oder n- oder tert.-Butyl und R⁴ Methyl, Ethyl, n- oder iso-Propyl, n- oder tert.-Butyl oder ggf. durch Halogen, Methyl, Methoxy oder Alkoxyiminomethylen substituiertes Phenyl bedeuten.

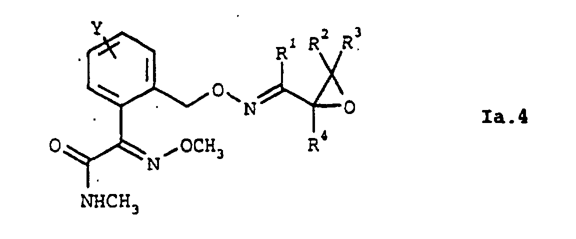

Insbesondere werden auch Verbindungen der Formel Ia.5 bevorzugt, in denen Y für Wasserstoff, 6-Methyl, 6-Chlor oder 3- oder 6-Fluor steht, R¹ Methyl, Ethyl, Chlor, Trifluormethyl oder Methoxy, R² Wasserstoff oder Methyl, R³ Methyl, Ethyl, n- oder iso-Propyl oder n- oder tert.-Butyl und R⁴ Methyl, Ethyl, n- oder iso-Propyl, n- oder tert.-Butyl oder ggf. durch Halogen, Methyl, Methoxy oder Alkoxyiminomethylen substituiertes Phenyl bedeuten.

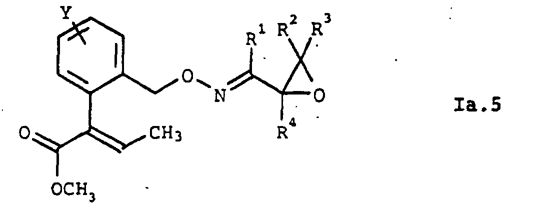

Außerdem werden Verbindungen IB besonders bevorzugt, in denen Y, R² und R^{a} für Wasserstoff stehen, R¹ Methyl bedeutet und X N(COOCH₃)-OCH₃, C(COOCH₃)=COCH₃, C(COOCH₃)=NOCH₃ oder C(COOCH₃)=C-CH₃, R³ Wasserstoff oder Methyl, R⁴ Methyl oder ggf. durch para-Halogen substituiertes Phenyl und R^{a}' Wasserstoff oder Methyl bedeuten.

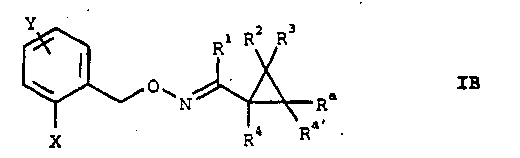

Gleichermaßen besonders bevorzugt sind Verbindungen IC, in denen Y für Wasserstoff steht, R¹ Methyl, X N(COOCH₃)-OCH₃, C(COOCH₃)=COCH₃, C(COOCH₃)=NOCH₃ oder C (COOCH₃)=C-CH₃, R² Wasserstoff, R³ Wasserstoff oder Methyl, R⁴ Methyl oder ggf. durch para-Halogen substituiertes Phenyl und R^{a} Wasserstoff, Methyl oder Ethyl bedeuten.

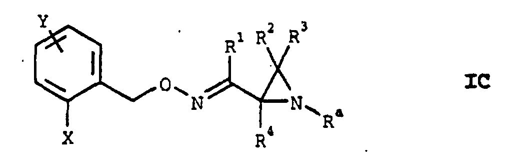

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der allgemeinen Formel Ia.1, in denen die Kombination der Substituenten Y, R¹, R², R³ und R⁴ für eine Verbindung einer Zeile der Tabelle A entspricht

### Tabelle 2

Verbindungen der allgemeinen Formel Ia.2, in denen die Kombination der Substituenten Y, R¹, R², R³ und R⁴ für eine Verbindung einer Zeile der Tabelle A entspricht

### Tabelle 3

Verbindungen der allgemeinen Formel Ia.3, in denen die Kombination der Substituenten Y, R¹, R², R³ und R⁴ für eine Verbindung einer Zeile der Tabelle A entspricht

### Tabelle 4

Verbindungen der allgemeinen Formel Ia.4, in denen die Kombination der Substituenten Y, R¹, R², R³ und R⁴ für eine Verbindung einer Zeile der Tabelle A entspricht

### Tabelle 5

Verbindungen der allgemeinen Formel Ia.5, in denen die Kombination der Substituenten Y, R¹, R², R³ und R⁴ für eine Verbindung einer Zeile der Tabelle A entspricht

### Tabelle 6

Verbindungen der allgemeinen Formel I.B, in denen Y, R² und R^{a} für Wasserstoff stehen, R¹ Methyl bedeutet und die Kombination der Reste X, R³, R⁴ und R^{a'} für eine Verbindung jeweils einer Zeile der Tabelle B entspricht

### Tabelle 7

Verbindungen der allgemeinen Formel I.C, in denen Y für Wasserstoff steht, R¹ Methyl bedeutet und die Kombination der Reste X, R², R³, R⁴ und R^{a} für eine Verbindung jeweils einer Zeile der Tabelle C entspricht

**Tabelle A:**

| **Nr.** | **Y** | **R**^{**1**} | **R**^{**2**} | **R**^{**3**} | **R**^{**4**} |
|---|---|---|---|---|---|
| A.1 | H | CH₃ | H | H | CH₃ |
| A.2 | H | CH₃ | H | H | Ethyl |
| A.3 | H | CH₃ | H | H | n-Propyl |
| A.4 | H | CH₃ | H | H | iso-Propyl |
| A.5 | H | CH₃ | H | H | tert. Butyl |
| A.6 | H | CH₃ | H | H | n-Butyl |
| A.7 | H | CH₃ | H | H | C₆H₅ |
| A.8 | H | CH₃ | H | H | 2-F-C₆H₄ |
| A.9 | H | CH₃ | H | H | 2-Cl-C₆H₄ |
| A.10 | H | CH₃ | H | H | 2-CH₃-C₆H₄ |
| A.11 | H | CH₃ | H | H | 2-CF₃-C₆H₄ |
| A.12 | H | CH₃ | H | H | 3-F-C₆H₄ |
| A.13 | H | CH₃ | H | H | 3-Cl-C₆H₄ |
| A.14 | H | CH₃ | H | H | 3-CH₃-C₆H₄ |
| A.15 | H | CH₃ | H | H | 3-CF₃-C₆H₄ |
| A.16 | H | CH₃ | H | H | 3-Br-C₆H₄ |
| A.17 | H | CH₃ | H | H | 4-F-C₆H₄ |
| A.18 | H | CH₃ | H | H | 4-Cl-C₆H₄ |
| A.19 | H | CH₃ | H | H | 4-CH₃-C₆H₄ |
| A.20 | H | CH₃ | H | H | 4-CF₃-C₆H₄ |
| A.21 | H | CH₃ | H | H | 4-OCH₃-C₆H₄ |
| A.22 | H | CH₃ | H | H | 4-OCF₃-C₆H₄ |
| A.23 | H | CH₃ | H | H | 4-Br-C₆H₄ |
| A.24 | H | CH₃ | H | H | 4-(CH=NOCH₃)-C₆H₄ |
| A.25 | H | CH₃ | H | H | 4-(CH=NOEt)-C₆H₄ |
| A.26 | H | CH₃ | H | H | 4-[C(CH₃)=NOCH₃]-C₆H₄ |
| A.27 | H | CH₃ | H | H | 4-[C(CH₃)=NOEt]-C₆H₄ |
| A.28 | H | CH₃ | H | H | 2,4-F₂-C₆H₃ |
| A.29 | H | CH₃ | H | H | 2,4-Cl₂-C₆H₃ |
| A.30 | H | CH₃ | H | H | 2,4-(CH₃)₂-C₆H₃ |
| A.31 | H | CH₃ | H | H | 3,5-F₂-C₆H₃ |
| A.32 | H | CH₃ | H | H | 3,5-Cl₂-C₆H₃ |
| A.33 | H | CH₃ | H | H | 3,5-(CH₃)₂-C₆H₃ |
| A.34 | H | CH₃ | H | H | 2-F-4-Cl-C₆H₃ |
| A.35 | H | CH₃ | H | H | 2-Cl-4-F-C₆H₃ |
| A.36 | H | CH₃ | H | H | 3,4-F₂-C₆H₃ |
| A.37 | H | CH₃ | H | H | 3,4-Cl₂-C₆H₃ |
| A.38 | H | CH₃ | H | H | 2,3-F₂-C₆H₃ |
| A.39 | H | CH₃ | H | CH₃ | CH₃ |
| A.40 | H | CH₃ | H | CH₃ | Ethyl |
| A.41 | H | CH₃ | H | CH₃ | n-Propyl |
| A.42 | H | CH₃ | H | CH₃ | iso-Propyl |
| A.43 | H | CH₃ | H | CH₃ | tert. Butyl |
| A.44 | H | CH₃ | H | CH₃ | n-Butyl |
| A.45 | H | CH₃ | H | CH₃ | C₆H₅ |
| A.46 | H | CH₃ | H | CH₃ | 2-F-C₆H₄ |
| A.47 | H | CH₃ | H | CH₃ | 2-Cl-C₆H₄ |
| A.48 | H | CH₃ | H | CH₃ | 2-CH₃-C₆H₄ |
| A.49 | H | CH₃ | H | CH₃ | 2-CF₃-C₆H₄ |
| A.50 | H | CH₃ | H | CH₃ | 3-F-C₆H₄ |
| A.51 | H | CH₃ | H | CH₃ | 3-Cl-C₆H₄ |
| A.52 | H | CH₃ | H | CH₃ | 3-CH₃-C₆H₄ |
| A.53 | H | CH₃ | H | CH₃ | 3-CF₃-C₆H₄ |
| A.54 | H | CH₃ | H | CH₃ | 3-Br-C₆H₄ |
| A.55 | H | CH₃ | H | CH₃ | 4-F-C₆H₄ |
| A.56 | H | CH₃ | H | CH₃ | 4-Cl-C₆H₄ |
| A.57 | H | CH₃ | H | CH₃ | 4-CH₃-C₆H₄ |
| A.58 | H | CH₃ | H | CH₃ | 4-CF₃-C₆H₄ |
| A.59 | H | CH₃ | H | CH₃ | 4-OCH₃-C₆H₄ |
| A.60 | H | CH₃ | H | CH₃ | 4-OCF₃-C₆H₄ |
| A.61 | H | CH₃ | H | CH₃ | 4-Br-C₆H₄ |
| A.62 | H | CH₃ | H | CH₃ | 4-(CH=NOCH₃)-C₆H₄ |
| A.63 | H | CH₃ | H | CH₃ | 4-(CH=NOEt)-C₆H₄ |
| A.64 | H | CH₃ | H | CH₃ | 4-[C(CH₃)=NOCH₃]-C₆H₄ |
| A.65 | H | CH₃ | H | CH₃ | 4-[C(CH₃)=NOEt]-C₆H₄ |
| A.66 | H | CH₃ | H | CH₃ | 2,4-F₂-C₆H₃ |
| A.67 | H | CH₃ | H | CH₃ | 2,4-Cl₂-C₆H₃ |
| A.68 | H | CH₃ | H | CH₃ | 2,4-(CH₃)₂-C₆H₃ |
| A.69 | H | CH₃ | H | CH₃ | 3,5-F₂-C₆H₃ |
| A.70 | H | CH₃ | H | CH₃ | 3,5-Cl₂-C₆H₃ |
| A.71. | H | CH₃ | H | CH₃ | 3,5-(CH₃)₂-C₆H₃ |
| A.72 | H | CH₃ | H | CH₃ | 2-F-4-Cl-C₆H₃ |
| A.73 | H | CH₃ | H | CH₃ | 2-Cl-4-F-C₆H₃ |
| A.74 | H | CH₃ | H | CH₃ | 3,4-F₂-C₆H₃ |
| A.75 | H | CH₃ | H | CH₃ | 3,4-Cl₂-C₆H₃ |
| A.76 | H | CH₃ | H | CH₃ | 2,3-F₂-C₆H₃ |
| A.77 | H | CH₃ | H | Ethyl | CH₃ |
| A.78 | H | CH₃ | H | Ethyl | Ethyl |
| A.79 | H | CH₃ | H | Ethyl | n-Propyl |
| A.80 | H | CH₃ | H | Ethyl | iso-Propyl |
| A.81 | H | CH₃ | H | Ethyl | tert. Butyl |
| A.82 | H | CH₃ | H | Ethyl | n-Butyl |
| A.83 | H | CH₃ | H | Ethyl | C₆H₅ |
| A.84 | H | CH₃ | H | Ethyl | 2-F-C₆H₄ |
| A.85 | H | CH₃ | H | Ethyl | 2-Cl-C₆H₄ |
| A.86 | H | CH₃ | H | Ethyl | 2-CH₃-C₆H₄ |
| A.87 | H | CH₃ | H | Ethyl | 2-CF₃-C₆H₄ |
| A.88 | H | CH₃ | H | Ethyl | 3-F-C₆H₄ |
| A.89 | H | CH₃ | H | Ethyl | 3-Cl-C₆H₄ |
| A.90 | H | CH₃ | H | Ethyl | 3-CH₃-C₆H₄ |
| A.91 | H | CH₃ | H | Ethyl | 3-CF₃-C₆H₄ |
| A.92 | H | CH₃ | H | Ethyl | 3-Br-C₆H₄ |
| A.93 | H | CH₃ | H | Ethyl | 4-F-C₆H₄ |
| A.94 | H | CH₃ | H | Ethyl | 4-Cl-C₆H₄ |
| A.95 | H | CH₃ | H | Ethyl | 4-CH₃-C₆H₄ |
| A.96 | H | CH₃ | H | Ethyl | 4-CF₃-C₆H₄ |
| A.97 | H | CH₃ | H | Ethyl | 4-OCH₃-C₆H₄ |
| A.98 | H | CH₃ | H | Ethyl | 4-OCF₃-C₆H₄ |
| A.99 | H | CH₃ | H | Ethyl | 4-Br-C₆H₄ |
| A.100 | H | CH₃ | H | Ethyl | 4-(CH=NOCH₃)-C₆H₄ |
| A.101 | H | CH₃ | H | Ethyl | 4-(CH=NOEt)-C₆H₄ |
| A.102 | H | CH₃ | H | Ethyl | 4-[C(CH₃)=NOCH₃]-C₆H₄ |
| A.103 | H | CH₃ | H | Ethyl | 4-[C(CH₃)=NOEt]-C₆H₄ |
| A.104 | H | CH₃ | H | Ethyl | 2,4-F₂-C₆H₃ |
| A.105 | H | CH₃ | H | Ethyl | 2,4-Cl₂-C₆H₃ |
| A.106 | H | CH₃ | H | Ethyl | 2,4-(CH₃)₂-C₆H₃ |
| A.107 | H | CH₃ | H | Ethyl | 3,5-F₂-C₆H₃ |
| A.108 | H | CH₃ | H | Ethyl | 3,5-Cl₂-C₆H₃ |
| A.109 | H | CH₃ | H | Ethyl | 3,5-(CH₃)₂-C₆H₃ |
| A.110 | H | CH₃ | H | Ethyl | 2-F-4-Cl-C₆H₃ |
| A.111 | H | CH₃ | H | Ethyl | 2-Cl-4-F-C₆H₃ |
| A.112 | H | CH₃ | H | Ethyl | 3,4-F₂-C₆H₃ |
| A.113 | H | CH₃ | H | Ethyl | 3,4-Cl₂-C₆H₃ |
| A.114 | H | CH₃ | H | Ethyl | 2,3-F₂-C₆H₃ |
| A.115 | H | CH₃ | H | n-Propyl | CH₃ |
| A.116 | H | CH₃ | H | n-Propyl | Ethyl |
| A.117 | H | CH₃ | H | n-Propyl | n-Propyl |
| A.118 | H | CH₃ | H | n-Propyl | iso-Propyl |
| A.119 | H | CH₃ | H | n-Propyl | tert. Butyl |
| A.120 | H | CH₃ | H | n-Propyl | n-Butyl |
| A.121 | H | CH₃ | H | n-Propyl | C₆H₅ |
| A.122 | H | CH₃ | H | n-Propyl | 2-F-C₆H₄ |
| A.123 | H | CH₃ | H | n-Propyl | 2-Cl-C₆H₄ |
| A.124 | H | CH₃ | H | n-Propyl | 2-CH₃-C₆H₄ |
| A.125 | H | CH₃ | H | n-Propyl | 2-CF₃-C₆H₄ |
| A.126 | H | CH₃ | H | n-Propyl | 3-F-C₆H₄ |
| A.127 | H | CH₃ | H | n-Propyl | 3-Cl-C₆H₄ |
| A.128 | H | CH₃ | H | n-Propyl | 3-CH₃-C₆H₄ |
| A.129 | H | CH₃ | H | n-Propyl | 3-CF₃-C₆H₄ |
| A.130 | H | CH₃ | H | n-Propyl | 3-Br-C₆H₄ |
| A.131 | H | CH₃ | H | n-Propyl | 4-F-C₆H₄ |
| A.132 | H | CH₃ | H | n-Propyl | 4-Cl-C₆H₄ |
| A.133 | H | CH₃ | H | n-Propyl | 4-CH₃-C₆H₄ |
| A.134 | H | CH₃ | H | n-Propyl | 4-CF₃-C₆H₄ |
| A.135 | H | CH₃ | H | n-Propyl | 4-OCH₃-C₆H₄ |
| A.136 | H | CH₃ | H | n-Propyl | 4-OCF₃-C₆H₄ |
| A.137 | H | CH₃ | H | n-Propyl | 4-Br-C₆H₄ |
| A.138 | H | CH₃ | H | n-Propyl | 4-(CH=NOCH₃)-C₆H₄ |
| A.139 | H | CH₃ | H | n-Propyl | 4-(CH=NOEt)-C₆H₄ |
| A.140 | H | CH₃ | H | n-Propyl | 4-[C(CH₃)=NOCH₃]-C₆H₄ |
| A.141 | H | CH₃ | H | n-Propyl | 4-[C(CH₃)=NOEt]-C₆H₄ |
| A.142 | H | CH₃ | H | n-Propyl | 2,4-F₂-C₆H₃ |
| A.143 | H | CH₃ | H | n-Propyl | 2,4-Cl₂-C₆H₃ |
| A.144 | H | CH₃ | H | n-Propyl | 2,4-(CH₃)₂-C₆H₃ |
| A.145 | H | CH₃ | H | n-Propyl | 3,5-F₂-C₆H₃ |
| A.146 | H | CH₃ | H | n-Propyl | 3,5-Cl₂-C₆H₃ |
| A.147 | H | CH₃ | H | n-Propyl | 3,5-(CH₃)₂-C₆H₃ |
| A.148 | H | CH₃ | H | n-Propyl | 2-F-4-Cl-C₆H₃ |
| A.149 | H | CH₃ | H | n-Propyl | 2-Cl-4-F-C₆H₃ |
| A.150 | H | CH₃ | H | n-Propyl | 3,4-F₂-C₆H₃ |
| A.151 | H | CH₃ | H | n-Propyl | 3,4-Cl₂-C₆H₃ |
| A.152 | H | CH₃ | H | n-Propyl | 2,3-F₂-C₆H₃ |
| A.153 | H | CH₃ | H | n-Propyl | CH₃ |
| A.154 | H | CH₃ | H | n-Propyl | Ethyl |
| A.155 | H | CH₃ | H | n-Propyl | n-Propyl |
| A.156 | H | CH₃ | H | n-Propyl | iso-Propyl |
| A.157 | H | CH₃ | H | n-Propyl | tert. Butyl |
| A.158 | H | CH₃ | H | n-Propyl | n-Butyl |
| A.159 | H | CH₃ | H | n-Propyl | C₆H₅ |
| A.160 | H | CH₃ | H | n-Propyl | 2-F-C₆H₄ |
| A.161 | H | CH₃ | H | n-Propyl | 2-Cl-C₆H₄ |
| A.162 | H | CH₃ | H | n-Propyl | 2-CH₃-C₆H₄ |
| A.163 | H | CH₃ | H | n-Propyl | 2-CF₃-C₆H₄ |
| A.164 | H | CH₃ | H | n-Propyl | 3-F-C₆H₄ |
| A.165 | H | CH₃ | H | n-Propyl | 3-Cl-C₆H₄ |
| A.166 | H | CH₃ | H | n-Propyl | 3-CH₃-C₆H₄ |
| A.167 | H | CH₃ | H | n-Propyl | 3-CF₃-C₆H₄ |
| A.168 | H | CH₃ | H | n-Propyl | 3-Br-C₆H₄ |
| A.169 | H | CH₃ | H | n-Propyl | 4-F-C₆H₄ |
| A.170 | H | CH₃ | H | n-Propyl | 4-Cl-C₆H₄ |
| A.171 | H | CH₃ | H | n-Propyl | 4-CH₃-C₆H₄ |
| A.172 | H | CH₃ | H | n-Propyl | 4-CF₃-C₆H₄ |
| A.173 | H | CH₃ | H | n-Propyl | 4-OCH₃-C₆H₄ |
| A.174 | H | CH₃ | H | n-Propyl | 4-OCF₃-C₆H₄ |
| A.175 | H | CH₃ | H | n-Propyl | 4-Br-C₆H₄ |
| A.176 | H | CH₃ | H | n-Propyl | 4-(CH=NOCH₃)-C₆H₄ |
| A.177 | H | CH₃ | H | n-Propyl | 4-(CH=NOEt)-C₆H₄ |
| A.178 | H | CH₃ | H | n-Propyl | 4-[C(CH₃)=NOCH₃]-C₆H₄ |
| A.179 | H | CH₃ | H | n-Propyl | 4-[C(CH₃)=NOEt]-C₆H₄ |
| A.180 | H | CH₃ | H | n-Propyl | 2,4-F₂-C₆H₃ |
| A.181 | H | CH₃ | H | n-Propyl | 2,4-Cl₂-C₆H₃ |
| A.182 | H | CH₃ | H | n-Propyl | 2,4-(CH₃)₂-C₆H₃ |
| A.183 | H | CH₃ | H | n-Propyl | 3,5-F₂-C₆H₃ |
| A.184 | H | CH₃ | H | n-Propyl | 3,5-Cl₂-C₆H₃ |
| A.185 | H | CH₃ | H | n-Propyl | 3,5-(CH₃)₂-C₆H₃ |
| A.186 | H | CH₃ | H | n-Propyl | 2-F-4-Cl-C₆H₃ |
| A.187 | H | CH₃ | H | n-Propyl | 2-Cl-4-F-C₆H₃ |
| A.188 | H | CH₃ | H | n-Propyl | 3,4-F₂-C₆H₃ |
| A.189 | H | CH₃ | H | n-Propyl | 3,4-Cl₂-C₆H₃ |
| A.190 | H | CH₃ | H | n-Propyl | 2,3-F₂-C₆H₃ |
| A.191 | H | CH₃ | H | n-Butyl | CH₃ |
| A.192 | H | CH₃ | H | n-Butyl | Ethyl |
| A.193 | H | CH₃ | H | n-Butyl | n-Propyl |
| A.194 | H | CH₃ | H | n-Butyl | iso-Propyl |
| A.195 | H | CH₃ | H | n-Butyl | tert. Butyl |
| A.196 | H | CH₃ | H | n-Butyl | n-Butyl |
| A.197 | H | CH₃ | H | n-Butyl | C₆H₅ |
| A.198 | H | CH₃ | H | n-Butyl | 2-F-C₆H₄ |
| A.199 | H | CH₃ | H | n-Butyl | 2-Cl-C₆H₄ |
| A.200 | H | CH₃ | H | n-Butyl | 2-CH₃-C₆H₄ |
| A.201 | H | CH₃ | H | n-Butyl | 2-CF₃-C₆H₄ |
| A.202 | H | CH₃ | H | n-Butyl | 3-F-C₆H₄ |
| A.203 | H | CH₃ | H | n-Butyl | 3-Cl-C₆H₄ |
| A.204 | H | CH₃ | H | n-Butyl | 3-CH₃-C₆H₄ |
| A.205 | H | CH₃ | H | n-Butyl | 3-CF₃-C₆H₄ |
| A.206 | H | CH₃ | H | n-Butyl | 3-Br-C₆H₄ |
| A.207 | H | CH₃ | H | n-Butyl | 4-F-C₆H₄ |
| A.208 | H | CH₃ | H | n-Butyl | 4-Cl-C₆H₄ |
| A.209 | H | CH₃ | H | n-Butyl | 4-CH₃-C₆H₄ |
| A.210 | H | CH₃ | H | n-Butyl | 4-CF₃-C₆H₄ |
| A.211 | H | CH₃ | H | n-Butyl | 4-OCH₃-C₆H₄ |
| A.212 | H | CH₃ | H | n-Butyl | 4-OCF₃-C₆H₄ |
| A.213 | H | CH₃ | H | n-Butyl | 4-Br-C₆H₄ |
| A.214 | H | CH₃ | H | n-Butyl | 4-(CH=NOCH₃)-C₆H₄ |
| A.215 | H | CH₃ | H | n-Butyl | 4-(CH=NOEt)-C₆H₄ |
| A.216 | H | CH₃ | H | n-Butyl | 4-[C(CH₃)=NOCH₃]-C₆H₄ |
| A.217 | H | CH₃ | H | n-Butyl | 4-[C(CH₃)=NOEt]-C₆H₄ |
| A.218 | H | CH₃ | H | n-Butyl | 2,4-F₂-C₆H₃ |
| A.219 | H | CH₃ | H | n-Butyl | 2,4-Cl₂-C₆H₃ |
| A.220 | H | CH₃ | H | n-Butyl | 2,4-(CH₃)₂-C₆H₃ |
| A.221 | H | CH₃ | H | n-Butyl | 3,5-F₂-C₆H₃ |
| A.222 | H | CH₃ | H | n-Butyl | 3,5-Cl₂-C₆H₃ |
| A.223 | H | CH₃ | H | n-Butyl | 3,5-(CH₃)₂-C₆H₃ |
| A.224 | H | CH₃ | H | n-Butyl | 2-F-4-Cl-C₆H₃ |
| A.225 | H | CH₃ | H | n-Butyl | 2-Cl-4-F-C₆H₃ |
| A.226 | H | CH₃ | H | n-Butyl | 3,4-F₂-C₆H₃ |
| A.227 | H | CH₃ | H | n-Butyl | 3,4-Cl₂-C₆H₃ |
| A.228 | H | CH₃ | H | n-Butyl | 2,3-F₂-C₆H₃ |
| A.229 | H | CH₃ | H | n-Butyl | CH₃ |
| A.230 | H | CH₃ | H | n-Butyl | Ethyl |
| A.231 | H | CH₃ | H | n-Butyl | n-Propyl |
| A.232 | H | CH₃ | H | n-Butyl ' | iso-Propyl |
| A.233 | H | CH₃ | H | n-Butyl | tert. Butyl |
| A.234 | H | CH₃ | H | n-Butyl | n-Butyl |
| A.235 | H | CH₃ | H | n-Butyl | C₆H₅ |
| A.236 | H | CH₃ | H | n-Butyl | 2-F-C₆H₄ |
| A.237 | H | CH₃ | H | n-Butyl | 2-Cl-C₆H₄ |
| A.238 | H | CH₃ | H | n-Butyl | 2-CH₃-C₆H₄ |
| A.239 | H | CH₃ | H | n-Butyl | 2-CF₃-C₆H₄ |
| A.240 | H | CH₃ | H | n-Butyl | 3-F-C₆H₄ |
| A.241 | H | CH₃ | H | n-Butyl | 3-Cl-C₆H₄ |
| A.242 | H | CH₃ | H | n-Butyl | 3-CH₃-C₆H₄ |
| A.243 | H | CH₃ | H | n-Butyl | 3-CF₃-C₆H₄ |
| A.244 | H | CH₃ | H | n-Butyl | 3-Br-C₆H₄ |
| A.245 | H | CH₃ | H | n-Butyl | 4-F-C₆H₄ |
| A.246 | H | CH₃ | H | n-Butyl | 4-Cl-C₆H₄ |
| A.247 | H | CH₃ | H | n-Butyl | 4-CH₃-C₆H₄ |
| A.248 | H | CH₃ | H | n-Butyl | 4-CF₃-C₆H₄ |
| A.249 | H | CH₃ | H | n-Butyl | 4-OCH₃-C₆H₄ |
| A.250 | H | CH₃ | H | n-Butyl | 4-OCF₃-C₆H₄ |
| A.251 | H | CH₃ | H | n-Butyl | 4-Br-C₆H₄ |
| A.252 | H | CH₃ | H | n-Butyl | 4-(CH=NOCH₃)-C₆H₄ |
| A.253 | H | CH₃ | H | n-Butyl | 4-(CH=NOEt)-C₆H₄ |
| A.254 | H | CH₃ | H | n-Butyl | 4-[C(CH₃)=NOCH₃]-C₆H₄ |
| A.255 | H | CH₃ | H | n-Butyl | 4-[C(CH₃)=NOEt]-C₆H₄ |
| A.256 | H | CH₃ | H | n-Butyl | 2,4-F₂-C₆H₃ |
| A.257 | H | CH₃ | H | n-Butyl | 2,4-Cl₂-C₆H₃ |
| A.258 | H | CH₃ | H | n-Butyl | 2,4-(CH₃)₂-C₆H₃ |
| A.259 | H | CH₃ | H | n-Butyl | 3,5-F₂-C₆H₃ |
| A.260 | H | CH₃ | H | n-Butyl | 3,5-Cl₂-C₆H₃ |
| A.261 | H | CH₃ | H | n-Butyl | 3,5-(CH₃)₂-C₆H₃ |
| A.262 | H | CH₃ | H | n-Butyl | 2-F-4-Cl-C₆H₃ |
| A.263 | H | CH₃ | H | n-Butyl | 2-Cl-4-F-C₆H₃ |
| A.264 | H | CH₃ | H | n-Butyl | 3,4-F₂-C₆H₃ |
| A.265 | H | CH₃ | H | n-Butyl | 3,4-Cl₂-C₆H₃ |
| A.266 | H | CH₃ | H | n-Butyl | 2,3-F₂-C₆H₃ |
| A.267 | H | CH₃ | H | iso-Propyl | CH₃ |
| A.268 | H | CH₃ | H | iso-Propyl | Ethyl |
| A.269 | H | CH₃ | H | iso-Propyl | n-Propyl |
| A.270 | H | CH₃ | H | iso-Propyl | iso-Propyl |
| A.271 | H | CH₃ | H | iso-Propyl | tert. Butyl |
| A.272 | H | CH₃ | H | iso-Propyl | n-Butyl |
| A.273 | H | CH₃ | H | iso-Propyl | C₆H₅ |
| A.274 | H | CH₃ | H | iso-Propyl | 2-F-C₆H₄ |
| A.275 | H | CH₃ | H | iso-Propyl | 2-Cl-C₆H₄ |
| A.276 | H | CH₃ | H | iso-Propyl | 2-CH₃-C₆H₄ |
| A.277 | H | CH₃ | H | iso-Propyl | 2-CF₃-C₆H₄ |
| A.278 | H | CH₃ | H | iso-Propyl | 3-F-C₆H₄ |
| A.279 | H | CH₃ | H | iso-Propyl | 3-Cl-C₆H₄ |
| A.280 | H | CH₃ | H | iso-Propyl | 3-CH₃-C₆H₄ |
| A.281 | H | CH3 | H | iso-Propyl | 3-CF₃-C₆H₄ |
| A.282 | H | CH3 | H | iso-Propyl | 3-Br-C₆H₄ |
| A.283 | H | CH3 | H | iso-Propyl | 4-F-C₆H₄ |
| A.284 | H | CH3 | H | iso-Propyl | 4-Cl-C₆H₄ |
| A.285 | H | CH3 | H | iso-Propyl | 4-CH₃-C₆H₄ |
| A.286 | H | CH3 | H | iso-Propyl | 4-CF₃-C₆H₄ |
| A.287 | H | CH3 | H | iso-Propyl | 4-OCH₃-C₆H₄ |
| A.288 | H | CH3 | H | iso-Propyl | 4-OCF₃-C₆H₄ |
| A.289 | H | CH3 | H | iso-Propyl | 4-Br-C₆H₄ |
| A.290 | H | CH3 | H | iso-Propyl | 4-(CH=NOCH₃)-C₆H₄ |
| A.291 | H | CH3 | H | iso-Propyl | 4-(CH=NOEt)-C₆H₄ |
| A.292 | H | CH3 | H | iso-Propyl | 4-[C(CH₃)=NOCH₃]-C₆H₄ |
| A.293 | H | CH3 | H | iso-Propyl | 4-[C(CH₃)=NOEt]-C₆H₄ |
| A.294 | H | CH3 | H | iso-Propyl | 2,4-F₂-C₆H₃ |
| A.295 | H | CH3 | H | iso-Propyl | 2,4-Cl₂-C₆H₃ |
| A.296 | H | CH3 | H | iso-Propyl | 2,4-(CH₃)₂-C₆H₃ |
| A.297 | H | CH₃ | H | iso-Propyl | 3,5-F₂-C₆H₃ |
| A.298 | H | CH₃ | H | iso-Propyl | 3,5-Cl₂-C₆H₃ |
| A.299 | H | CH₃ | H | iso-Propyl | 3,5-(CH₃)₂-C₆H₃ |
| A.300 | H | CH₃ | H | iso-Propyl | 2-F-4-Cl-C₆H₃ |
| A.301 | H | CH₃ | H | iso-Propyl | 2-Cl-4-F-C₆H₃ |
| A.302 | H | CH₃ | H | iso-Propyl | 3,4-F₂-C₆H₃ |
| A.303 | H | CH₃ | H | iso-Propyl | 3,4-Cl₂-C₆H₃ |
| A.304 | H | CH₃ | H | iso-Propyl | 2,3-F₂-C₆H₃ |
| A.305 | H | CH₃ | H | iso-Propyl | CH₃ |
| A.306 | H | CH₃ | H | iso-Propyl | Ethyl |
| A.307 | H | CH₃ | H | iso-Propyl | n-Propyl |
| A.308 | H | CH₃ | H | iso-Propyl | iso-Propyl |
| A.309 | H | CH₃ | H | iso-Propyl | tert. Butyl |
| A.310 | H | CH₃ | H | iso-Propyl | n-Butyl |
| A.311 | H | CH₃ | H | iso-Propyl | C₆H₅ |
| A.312 | H | CH₃ | H | iso-Propyl | 2-F-C₆H₄ |
| A.313 | H | CH₃ | H | iso-Propyl | 2-Cl-C₆H₄ |
| A.314 | H | CH₃ | H | iso-Propyl | 2-CH₃-C₆H₄ |
| A.315 | H | CH₃ | H | iso-Propyl | 2-CF₃-C₆H₄ |
| A.316 | H | CH₃ | H | iso-Propyl | 3-F-C₆H₄ |
| A.317 | H | CH₃ | H | iso-Propyl | 3-Cl-C₆H₄ |
| A.318 | H | CH₃ | H | iso-Propyl | 3-CH₃-C₆H₄ |
| A.319 | H | CH₃ | H | iso-Propyl | 3-CF₃-C₆H₄ |
| A.320 | H | CH₃ | H | iso-Propyl | 3-Br-C₆H₄ |
| A.321 | H | CH₃ | H | iso-Propyl | 4-F-C₆H₄ |
| A.322 | H | CH₃ | H | iso-Propyl | 4-Cl-C₆H₄ |
| A.323 | H | CH₃ | H | iso-Propyl | 4-CH₃-C₆H₄ |
| A.324 | H | CH₃ | H | iso-Propyl | 4-CF₃-C₆H₄ |
| A.325 | H | CH₃ | H | iso-Propyl | 4-OCH₃-C₆H₄ |
| A.326 | H | CH₃ | H | iso-Propyl | 4-OCF₃-C₆H₄ |
| A.327 | H | CH₃ | H | iso-Propyl | 4-Br-C₆H₄ |
| A.328 | H | CH₃ | H | iso-Propyl | 4-(CH=NOCH₃)-C₆H₄ |
| A.329 | H | CH₃ | H | iso-Propyl | 4-(CH=NOEt)-C₆H₄ |
| A.330 | H | CH₃ | H | iso-Propyl | 4-[C(CH₃)=NOCH₃]-C₆H₄ |
| A.331 | H | CH₃ | H | iso-Propyl | 4-[C(CH₃)=NOEt]-C₆H₄ |
| A.332 | H | CH₃ | H | iso-Propyl | 2,4-F₂-C₆H₃ |
| A.333 | H | CH₃ | H | iso-Propyl | 2,4-Cl₂-C₆H₃ |
| A.334 | H | CH₃ | H | iso-Propyl | 2,4-(CH₃)₂-C₆H₃ |
| A.335 | H | CH₃ | H | iso-Propyl | 3,5-F₂-C₆H₃ |
| A.336 | H | CH₃ | H | iso-Propyl | 3,5-Cl₂-C₆H₃ |
| A.337 | H | CH₃ | H | iso-Propyl | 3,5-(CH₃)₂-C₆H₃ |
| A.338 | H | CH₃ | H | iso-Propyl | 2-F-4-Cl-C₆H₃ |
| A.339 | H | CH₃ | H | iso-Propyl | 2-Cl-4-F-C₆H₃ |
| A.340 | H | CH₃ | H | iso-Propyl | 3,4-F₂-C₆H₃ |
| A.341 | H | CH₃ | H | iso-Propyl | 3,4-Cl₂-C₆H₃ |
| A.342 | H | CH₃ | H | iso-Propyl | 2,3-F₂-C₆H₃ |
| A.343 | H | CH₃ | H | tert. Butyl | CH₃ |
| A.344 | H | CH₃ | H | tert. Butyl | Ethyl |
| A.345 | H | CH₃ | H | tert. Butyl | n-Propyl |
| A.346 | H | CH₃ | H | tert. Butyl | iso-Propyl |
| A.347 | H | CH₃ | H | tert. Butyl | tert. Butyl |
| A.348 | H | CH₃ | H | tert. Butyl | n-Butyl |
| A.349 | H | CH₃ | H | tert. Butyl | C₆H₅ |
| A.350 | H | CH₃ | H | tert. Butyl | 2-F-C₆H₄ |
| A.351 | H | CH₃ | H | tert. Butyl | 2-Cl-C₆H₄ |
| A.352 | H | CH₃ | H | tert. Butyl | 2-CH₃-C₆H₄ |
| A.353 | H | CH₃ | H | tert. Butyl | 2-CF₃-C₆H₄ |
| A.354 | H | CH₃ | H | tert. Butyl | 3-F-C₆H₄ |
| A.355 | H | CH₃ | H | tert. Butyl | 3-Cl-C₆H₄ |
| A.356 | H | CH₃ | H | tert. Butyl | 3-CH₃-C₆H₄ |
| A.357 | H | CH₃ | H | tert. Butyl | 3-CF₃-C₆H₄ |
| A.358 | H | CH₃ | H | tert. Butyl | 3-Br-C₆H₄ |
| A.359 | H | CH₃ | H | tert. Butyl | 4-F-C₆H₄ |
| A.360 | H | CH₃ | H | tert. Butyl | 4-Cl-C₆H₄ |
| A.361 | H | CH₃ | H | tert. Butyl | 4-CH₃-C₆H₄ |
| A.362 | H | CH₃ | H | tert. Butyl | 4-CF₃-C₆H₄ |
| A.363 | H | CH₃ | H | tert. Butyl | 4-OCH₃-C₆H₄ |
| A.364 | H | CH₃ | H | tert. Butyl | 4-OCF₃-C₆H₄ |
| A.365 | H | CH₃ | H | tert. Butyl | 4-Br-C₆H₄ |
| A.366 | H | CH₃ | H | tert. Butyl | 4-(CH=NOCH₃)-C₆H₄ |
| A.367 | H | CH₃ | H | tert. Butyl | 4-(CH=NOEt)-C₆H₄ |
| A.368 | H | CH₃ | H | tert. Butyl | 4-[C(CH₃)=NOCH₃]-C₆H₄ |
| A.369 | H | CH₃ | H | tert. Butyl | 4-[C(CH₃)=NOEt]-C₆H₄ |
| A.370 | H | CH₃ | H | tert. Butyl | 2,4-F₂-C₆H₃ |
| A.371 | H | CH₃ | H | tert. Butyl | 2,4-Cl₂-C₆H₃ |
| A.372 | H | CH₃ | H | tert. Butyl | 2,4-(CH₃)₂-C₆H₃ |
| A.373 | H | CH₃ | H | tert. Butyl | 3,5-F₂-C₆H₃ |
| A.374 | H | CH₃ | H | tert. Butyl | 3,5-Cl₂-C₆H₃ |
| A.375 | H | CH₃ | H | tert. Butyl | 3,5-(CH₃)₂-C₆H₃ |
| A.376 | H | CH₃ | H | tert. Butyl | 2-F-4-Cl-C₆H₃ |
| A.377 | H | CH₃ | H | tert. Butyl | 2-Cl-4-F-C₆H₃ |
| A.378 | H | CH₃ | H | tert. Butyl | 3,4-F₂-C₆H₃ |
| A.379 | H | CH₃ | H | tert. Butyl | 3,4-Cl₂-C₆H₃ |
| A.380 | H | CH₃ | H | tert. Butyl | 2,3-F₂-C₆H₃ |
| A.381 | H | CH₃ | H | tert. Butyl | CH₃ |
| A.382 | H | CH₃ | H | tert. Butyl | Ethyl |
| A.383 | H | CH₃ | H | tert. Butyl | n-Propyl |
| A.384 | H | CH₃ | H | tert. Butyl | iso-Propyl |
| A.385 | H | CH₃ | H | tert. Butyl | tert. Butyl |
| A.386 | H | CH₃ | H | tert. Butyl | n-Butyl |
| A.387 | H | CH₃ | H | tert. Butyl | C₆H₅ |
| A.388 | H | CH₃ | H | tert. Butyl | 2-F-C₆H₄ |
| A.389 | H | CH₃ | H | tert. Butyl | 2-Cl-C₆H₄ |
| A.390 | H | CH₃ | H | tert. Butyl | 2-CH₃-C₆H₄ |
| A.391 | H | CH₃ | H | tert. Butyl | 2-CF₃-C₆H₄ |
| A.392 | H | CH₃ | H | tert. Butyl | 3-F-C₆H₄ |
| A.393 | H | CH₃ | H | tert. Butyl | 3-Cl-C₆H₄ |
| A.394 | H | CH₃ | H | tert. Butyl | 3-CH₃-C₆H₄ |
| A.395 | H | CH₃ | H | tert. Butyl | 3-CF₃-C₆H₄ |
| A.396 | H | CH₃ | H | tert. Butyl | 3-Br-C₆H₄ |
| A.397 | H | CH₃ | H | tert. Butyl | 4-F-C₆H₄ |
| A.398 | H | CH₃ | H | tert. Butyl | 4-Cl-C₆H₄ |
| A.399 | H | CH₃ | H | tert. Butyl | 4-CH₃-C₆H₄ |
| A.400 | H | CH₃ | H | tert. Butyl | 4-CF₃-C₆H₄ |
| A.401 | H | CH₃ | H | tert. Butyl | 4-OCH₃-C₆H₄ |
| A.402 | H | CH₃ | H | tert. Butyl | 4-OCF₃-C₆H₄ |
| A.403 | H | CH₃ | H | tert. Butyl | 4-Br-C₆H₄ |
| A.404 | H | CH₃ | H | tert. Butyl | 4-(CH=NOCH₃)-C₆H₄ |
| A.405 | H | CH₃ | H | tert. Butyl | 4-(CH=NOEt)-C₆H₄ |
| A.406 | H | CH₃ | H | tert. Butyl | 4-[C(CH₃)=NOCH₃]-C₆H₄ |
| A.407 | H | CH₃ | H | tert. Butyl | 4-[C(CH₃)=NOEt]-C₆H₄ |
| A.408 | H | CH₃ | H | tert. Butyl | 2,4-F₂-C₆H₃ |
| A.409 | H | CH₃ | H | tert. Butyl | 2,4-Cl₂-C₆H₃ |
| A.410 | H | CH₃ | H | tert. Butyl | 2,4-(CH₃)2-C₆H₃ |
| A.411 | H | CH₃ | H | tert. Butyl | 3,5-F₂-C₆H₃. |
| A.412 | H | CH₃ | H | tert. Butyl | 3,5-Cl₂-C₆H₃ |
| A.413 | H | CH₃ | H | tert. Butyl | 3,5-(CH₃)₂-C₆H₃ |
| A.414 | H | CH₃ | H | tert. Butyl | 2-F-4-Cl-C₆H₃ |
| A.415 | H | CH₃ | H | tert. Butyl | 2-Cl-4-F-C₆H₃ |
| A.416 | H | CH₃ | H | tert. Butyl | 3,4-F₂-C₆H₃ |
| A.417 | H | CH₃ | H | tert. Butyl | 3,4-Cl₂-C₆H₃ |
| A.418 | H | CH₃ | H | tert. Butyl | 2,3-F₂-C₆H₃ |
| A.419 | H | Cl | CH₃ | CH₃ | C₆H₅ |
| A.420 | H | Cl | CH₃ | CH₃ | 4-F-C₆H₄ |
| A.421 | H | Cl | CH₃ | CH₃ | 4-Cl-C₆H₄ |
| A.422 | H | Cl | CH₃ | CH₃ | CH₃ |
| A.423 | H | CF₃ | CH₃ | CH₃ | C₆H₅ |
| A.424 | H | CF₃ | CH₃ | CH₃ | 4-F-C₆H₄ |
| A.425 | H | CF₃ | CH₃ | CH₃ | 4-Cl-C₆H₄ |
| A.426 | H | CF₃ | CH₃ | CH₃ | CH₃ |
| A.427 | H | OCH₃ | CH₃ | CH₃ | C₆H₅ |
| A.428 | H | OCH₃ | CH₃ | CH₃ | 4-F-C₆H₄ |
| A.429 | H | OCH₃ | CH₃ | CH₃ | 4-Cl-C₆H₄ |
| A.430 | H | OCH₃ | CH₃ | CH₃ | CH₃ |
| A.431 | 6-CH₃ | CH₃ | H | CH₃ | CH3 |
| A.432 | 6-CH₃ | CH₃ | H | CH₃ | C₆H₅ |
| A.433 | 6-CH₃ | CH₃ | H | CH₃ | 4-F-C₆H₄ |
| A.434 | 6-CH₃ | CH₃ | H | CH₃ | 4-Cl-C₆H₄ |
| A.435 | 6-CH₃ | CH₃ | H | Ethyl | CH₃ |
| A.436 | 6-CH₃ | CH₃ | H | Ethyl | C₆H₅ |
| A.437 | 6-CH₃ | CH₃ | H | Ethyl | 4-F-C₆H₄ |
| A.438 | 6-CH₃ | CH₃ | H | Ethyl | 4-Cl-C₆H₄ |
| A.439 | 6-CH₃ | CH₃ | H | n-Propyl | CH₃ |
| A.440 | 6-CH₃ | CH₃ | H | n-Propyl | C₆H₅ |
| A.441 | 6-CH₃ | CH₃ | H | n-Propyl | 4-F-C₆H₄ |
| A.442 | 6-CH₃ | CH₃ | H | n-Propyl | 4-Cl-C₆H₄ |
| A.443 | 6-CH₃ | CH₃ | H | n-Butyl | CH₃ |
| A.444 | 6-CH₃ | CH₃ | H | n-Butyl | C6H5 |
| A.445 | 6-CH₃ | CH₃ | H | n-Butyl | 4-F-C₆H₄ |
| A.446 | 6-CH₃ | CH₃ | H | n-Butyl | 4-Cl-C₆H₄ |
| A.447 | 6-CH₃ | Ethyl | H | CH₃ | CH₃ |
| A.448 | 6-CH₃ | Ethyl | H | CH₃ | C₆H₅ |
| A.449 | 6-CH₃ | Ethyl | H | CH₃ | 4-F-C₆H₄ |
| A.450 | 6-CH₃ | Ethyl | H | CH₃ | 4-Cl-C₆H₄ |
| A.451 | 6-CH₃ | Ethyl | H | Ethyl | CH₃ |
| A.452 | 6-CH₃ | Ethyl | H | Ethyl | C₆H₅ |
| A.453 | 6-CH₃ | Ethyl | H | Ethyl | 4-F-C₆H₄ |
| A.454 | 6-CH₃ | Ethyl | H | Ethyl | 4-Cl-C₆H₄ |
| A.455 | 6-CH₃ | Ethyl | H | n-Propyl | CH₃ |
| A.456 | 6-CH₃ | Ethyl | H | n-Propyl | C₆H₅ |
| A.457 | 6-CH₃ | Ethyl | H | n-Propyl | 4-F-C₆H₄ |
| A.458 | 6-CH₃ | Ethyl | H | n-Propyl | 4-Cl-C₆H₄ |
| A.459 | 6-CH₃ | Ethyl | H | n-Butyl | CH₃ |
| A.460 | 6-CH₃ | Ethyl | H | n-Butyl | C₆H₅ |
| A.461 | 6-CH₃ | Ethyl | H | n-Butyl | 4-F-C₆H₄ |
| A.462 | 6-CH₃ | Ethyl | H | n-Butyl | 4-Cl-C₆H₄ |
| A.463 | 6-Cl | CH₃ | H | CH₃ | CH₃ |
| A.464 | 6-Cl | CH₃ | H | CH₃ | C₆H₅ |
| A.465 | 6-Cl | CH₃ | H | CH₃ | 4-F-C₆H₄ |
| A.466 | 6-Cl | CH₃ | H | CH₃ | 4-Cl-C₆H₄ |
| A.467 | 6-Cl | CH₃ | H | Ethyl | CH₃ |
| A.468 | 6-Cl | CH₃ | H | Ethyl | C₆H₅ |
| A.469 | 6-C1 | CH₃ | H | Ethyl | 4-F-C₆H₄ |
| A.470 | 6-C1 | CH₃ | H | Ethyl | 4-Cl-C₆H₄ |
| A.471 | 6-Cl | CH₃ | H | n-Propyl | CH₃ |
| A.472 | 6-Cl | CH₃ | H | n-Propyl | C₆H₅ |
| A.473 | 6-Cl | CH₃ | H | n-Propyl | 4-F-C₆H₄ |
| A.474 | 6-Cl | CH₃ | H | n-Propyl | 4-Cl-C₆H₄ |
| A.475 | 6-Cl | CH₃ | H | n-Butyl | CH₃ |
| A.476 | 6-Cl | CH₃ | H | n-Butyl | C₆H₅ |
| A.477 | 6-Cl | CH₃ | H | n-Butyl | 4-F-C₆H₄ |
| A.478 | 6-Cl | CH₃ | H | n-Butyl | 4-Cl-C₆H₄ |
| A.479 | 6-Cl | Ethyl | H | CH₃ | CH₃ |
| A.480 | 6-Cl | Ethyl | H | CH₃ | C₆H₅ |
| A.481 | 6-Cl | Ethyl | H | CH₃ | 4-F-C₆H₄ |
| A.482 | 6-Cl | Ethyl | H | CH₃ | 4-Cl-C₆H₄ |
| A.483 | 6-Cl | Ethyl | H | Ethyl | CH₃ |
| A.484 | 6-Cl | Ethyl | H | Ethyl | C₆H₅ |
| A.485 | 6-Cl | Ethyl | H | Ethyl | 4-F-C₆H₄ |
| A.486 | 6-Cl | Ethyl | H | Ethyl | 4-Cl-C₆H₄ |
| A.487 | 6-Cl | Ethyl | H | n-Propyl | CH₃ |
| A.488 | 6-Cl | Ethyl | H | n-Propyl | C₆H₅ |
| A.489 | 6-Cl | Ethyl | H | n-Propyl | 4-F-C₆H₄ |
| A.490 | 6-Cl | Ethyl | H | n-Propyl | 4-Cl-C₆H₄ |
| A.491 | 6-Cl | Ethyl | H | n-Butyl | CH₃ |
| A.492 | 6-Cl | Ethyl | H | n-Butyl | C₆H₅ |
| A.493 | 6-Cl | Ethyl | H | n-Butyl | 4-F-C₆H₄ |
| A.494 | 6-Cl | Ethyl | H | n-Butyl | 4-Cl-C₆H₄ |
| A.495 | 6-F | CH₃ | H | CH₃ | CH₃ |
| A.496 | 6-F | CH₃ | H | CH₃ | C₆H₅ |
| A.497 | 6-F | CH₃ | H | CH₃ | 4-F-C₆H₄ |
| A.498 | 6-F | CH₃ | H | CH₃ | 4-Cl-C₆H₄ |
| A.499 | 6-F | CH₃ | H | Ethyl | CH₃ |
| A.500 | 6-F | CH₃ | H | Ethyl | C₆H₅ |
| A.501 | 6-F | CH₃ | H | Ethyl | 4-F-C₆H₄ |
| A.502 | 6-F | CH₃ | H | Ethyl | 4-Cl-C₆H₄ |
| A.503 | 6-F | CH₃ | H | n-Propyl | CH₃ |
| A.504 | 6-F | CH₃ | H | n-Propyl | C₆H₅ |
| A.505 | 6-F | CH₃ | H | n-Propyl | 4-F-C₆H₄ |
| A.506 | 6-F | CH₃ | H | n-Propyl | 4-Cl-C₆H₄ |
| A.507 | 6-F | CH₃ | H | n-Butyl | CH₃ |
| A.508 | 6-F | CH₃ | H | n-Butyl | C₆H₅ |
| A.509 | 6-F | CH₃ | H | n-Butyl | 4-F-C₆H₄ |
| A.510 | 6-F | CH₃ | H | n-Butyl | 4-Cl-C₆H₄ |
| A.511 | 6-F | Ethyl | H | CH₃ | CH₃ |
| A.512 | 6-F | Ethyl | H | CH₃ | C₆H₅ |
| A.513 | 6-F | Ethyl | H | CH₃ | 4-F-C₆H₄ |
| A.514 | 6-F | Ethyl | H | CH₃ | 4-Cl-C₆H₄ |
| A.515 | 6-F | Ethyl | H | Ethyl | CH₃ |
| A.516 | 6-F | Ethyl | H | Ethyl | C₆H₅ |
| A.517 | 6-F | Ethyl | H | Ethyl | 4-F-C₆H₄ |
| A.518 | 6-F | Ethyl | H | Ethyl | 4-Cl-C₆H₄ |
| A.519 | 6-F | Ethyl | H | n-Propyl | CH₃ |
| A.520 | 6-F | Ethyl | H | n-Propyl | C₆H₅ |
| A.521 | 6-F | Ethyl | H | n-Propyl | 4-F-C₆H₄ |
| A.522 | 6-F | Ethyl | H | n-Propyl | 4-Cl-C₆H₄ |
| A.523 | 6-F | Ethyl | H | n-Butyl | CH₃ |
| A.524 | 6-F | Ethyl | H | n-Butyl | C₆H₅ |
| A.525 | 6-F | Ethyl | H | n-Butyl | 4-F-C₆H₄ |
| A.526 | 6-F | Ethyl | H | n-Butyl | 4-Cl-C₆H₄ |
| A.527 | 3-F | CH₃ | H | CH₃ | CH₃ |
| A.528 | 3-F | CH₃ | H | CH₃ | C₆H₅ |
| A.529 | 3-F | CH₃ | H | CH₃ | 4-F-C₆H₄ |
| A.530 | 3-F | CH₃ | H | CH₃ | 4-Cl-C₆H₄ |
| A.531 | 3-F | CH₃ | H | Ethyl | CH₃ |
| A.532 | 3-F | CH₃ | H | Ethyl | C₆H₅ |
| A.533 | 3-F | CH₃ | H | Ethyl | 4-F-C₆H₄ |
| A.534 | 3-F | CH₃ | H | Ethyl | 4-Cl-C₆H₄ |
| A.535 | 3-F | CH₃ | H | n-Propyl | CH₃ |
| A.536 | 3-F | CH₃ | H | n-Propyl | C₆H₅ |
| A.537 | 3-F | CH₃ | H | n-Propyl | 4-F-C₆H₄ |
| A.538 | 3-F | CH₃ | H | n-Propyl | 4-Cl-C₆H₄ |
| A.539 | 3-F | CH₃ | H | n-Butyl | CH₃ |
| A.540 | 3-F | CH₃ | H | n-Butyl | C₆H₅ |
| A.541 | 3-F | CH₃ | H | n-Butyl | 4-F-C₆H₄ |
| A.542 | 3-F | CH₃ | H | n-Butyl | 4-Cl-C₆H₄ |
| A.543 | 3-F | Ethyl | H | CH₃ | CH₃ |
| A.544 | 3-F | Ethyl | H | CH₃ | C₆H₅ |
| A.545 | 3-F | Ethyl | H | CH₃ | 4-F-C₆H₄ |
| A.546 | 3-F | Ethyl | H | CH₃ | 4-Cl-C₆H₄ |
| A.547 | 3-F | Ethyl | H | Ethyl | CH₃ |
| A.548 | 3-F | Ethyl | H | Ethyl | C₆H₅ |
| A.549 | 3-F | Ethyl | H | Ethyl | 4-F-C₆H₄ |
| A.550 | 3-F | Ethyl | H | Ethyl | 4-Cl-C₆H₄ |
| A.551 | 3-F | Ethyl | H | n-Propyl | CH₃ |
| A.552 | 3-F | Ethyl | H | n-Propyl | C₆H₅ |
| A.553 | 3-F | Ethyl | H | n-Propyl | 4-F-C₆H₄ |
| A.554 | 3-F | Ethyl | H | n-Propyl | 4-Cl-C₆H₄ |
| A.555 | 3-F | Ethyl | H | n-Butyl | CH₃ |
| A.556 | 3-F | Ethyl | H | n-Butyl | C₆H₅ |
| A.557 | 3-F | Ethyl | H | n-Butyl | 4-F-C₆H₄ |
| A.558 | 3-F | Ethyl | H | n-Butyl | 4-Cl-C₆H₄ |
| A.559 | H | Ethyl | H | H | CH₃ |
| A.560 | H | Ethyl | H | H | Ethyl |
| A.561 | H | Ethyl | H | H | n-Propyl |
| A.562 | H | Ethyl | H | H | iso-Propyl |
| A.563 | H | Ethyl | H | H | tert. Butyl |
| A.564 | H | Ethyl | H | H | n-Butyl |
| A.565 | H | Ethyl | H | H | C₆H₅ |
| A.566 | H | Ethyl | H | H | 2-F-C₆H₄ |
| A.567 | H | Ethyl | H | H | 2-Cl-C₆H₄ |
| A.568 | H | Ethyl | H | H | 2-CH₃-C₆H₄ |
| A.569 | H | Ethyl | H | H | 2-CF₃-C₆H₄ |
| A.570 | H | Ethyl | H | H | 3-F-C₆H₄ |
| A.571 | H | Ethyl | H | H | 3-Cl-C₆H₄ |
| A.572 | H | Ethyl | H | H | 3-CH₃-C₆H₄ |
| A.573 | H | Ethyl | H | H | 3-CF₃-C₆H₄ |
| A.574 | H | Ethyl | H | H | 3-Br-C₆H₄ |
| A.575 | H | Ethyl | H | H | 4-F-C₆H₄ |
| A.576 | H | Ethyl | H | H | 4-Cl-C₆H₄ |
| A.577 | H | Ethyl | H | H | 4-CH₃-C₆H₄ |
| A.578 | H | Ethyl | H | H | 4-CF₃-C₆H₄ |
| A.579 | H | Ethyl | H | H | 4-OCH₃-C₆H₄ |
| A.580 | H | Ethyl | H | H | 4-OCF₃-C₆H₄ |
| A.581 | H | Ethyl | H | H | 4-Br-C₆H₄ |
| A.582 | H | Ethyl | H | H | 4-(CH=NOCH₃)-C₆H₄ |
| A.583 | H | Ethyl | H | H | 4-(CH=NOEt)-C₆H₄ |
| A.584 | H | Ethyl | H | H | 4-[C(CH₃)=NOCH₃]-C₆H₄ |
| A.585 | H | Ethyl | H | H | 4-[C(CH₃)=NOEt]-C₆H₄ |
| A.586 | H | Ethyl | H | H | 2,4-F₂-C₆H₃ |
| A.587 | H | Ethyl | H | H | 2,4-Cl₂-C₆H₃ |
| A.588 | H | Ethyl | H | H | 2,4-(CH₃)₂-C₆H₃ |
| A.589 | H | Ethyl | H | H | 3,5-F₂-C₆H₃ |
| A.590 | H | Ethyl | H | H | 3,5-Cl₂-C₆H₃ |
| A.591 | H | Ethyl | H | H | 3,5-(CH₃)₂-C₆H₃ |
| A.592 | H | Ethyl | H | H | 2-F-4-Cl-C₆H₃ |
| A.593 | H | Ethyl | H | H | 2-Cl-4-F-C₆H₃ |
| A.594 | H | Ethyl | H | H | 3,4-F₂-C₆H₃ |
| A.595 | H | Ethyl | H | H | 3,4-Cl₂-C₆H₃ |
| A.596 | H | Ethyl | H | H | 2,3-F₂-C₆H₃ |
| A.597 | H | Ethyl | H | CH₃ | CH₃ |
| A.598 | H | Ethyl | H | CH₃ | Ethyl |
| A.599 | H | Ethyl | H | CH₃ | n-Propyl |
| A.600 | H | Ethyl | H | CH₃ | iso-Propyl |
| A.601 | H | Ethyl | H | CH₃ | tert. Butyl |
| A.602 | H | Ethyl | H | CH₃ | n-Butyl |
| A.603 | H | Ethyl | H | CH₃ | C₆H₅ |
| A.604 | H | Ethyl | H | CH₃ | 2-F-C₆H₄ |
| A.605 | H | Ethyl | H | CH₃ | 2-Cl-C₆H₄ |
| A.606 | H | Ethyl | H | CH₃ | 2-CH₃-C₆H₄ |
| A.607 | H | Ethyl | H | CH₃ | 2-CF₃-C₆H₄ |
| A.608 | H | Ethyl | H | CH₃ | 3-F-C₆H₄ |
| A.609 | H | Ethyl | H | CH₃ | 3-Cl-C₆H₄ |
| A.610 | H | Ethyl | H | CH₃ | 3-CH₃-C₆H₄ |
| A.611 | H | Ethyl | H | CH₃ | 3-CF₃-C₆H₄ |
| A.612 | H | Ethyl | H | CH₃ | 3-Br-C₆H₄ |
| A.613 | H | Ethyl | H | CH₃ | 4-F-C₆H₄ |
| A.614 | H | Ethyl | H | CH₃ | 4-Cl-C₆H₄ |
| A.615 | H | Ethyl | H | CH₃ | 4-CH₃-C₆H₄ |
| A.616 | H | Ethyl | H | CH₃ | 4-CF₃-C₆H₄ |
| A.617 | H | Ethyl | H | CH₃ | 4-OCH₃-C₆H₄ |
| A.618 | H | Ethyl | H | CH₃ | 4-OCF₃-C₆H₄ |
| A.619 | H | Ethyl | H | CH₃ | 4-Br-C₆H₄ |
| A.620 | H | Ethyl | H | CH₃ | 4-(CH=NOCH₃)-C₆H₄ |
| A.621 | H | Ethyl | H | CH₃ | 4-(CH=NOEt)-C₆H₄ |
| A.622 | H | Ethyl | H | CH₃ | 4-[C(CH₃)=NOCH₃]-C₆H₄ |
| A.623 | H | Ethyl | H | CH₃ | 4-[C(CH₃)=NOEt]-C₆H₄ |
| A.624 | H | Ethyl | H | CH₃ | 2,4-F₂-C₆H₃ |
| A.625 | H | Ethyl | H | CH₃ | 2,4-Cl₂-C₆H₃ |
| A.626 | H | Ethyl | H | CH₃ | 2,4-(CH₃)₂-C₆H₃ |
| A.627 | H | Ethyl | H | CH₃ | 3,5-F₂-C₆H₃ |
| A.628 | H | Ethyl | H | CH₃ | 3,5-Cl₂-C₆H₃ |
| A.629 | H | Ethyl | H | CH₃ | 3,5-(CH₃)₂-C₆H₃ |
| A.630 | H | Ethyl | H | CH₃ | 2-F-4-Cl-C₆H₃ |
| A.631 | H | Ethyl | H | CH₃ | 2-Cl-4-F-C₆H₃ |
| A.632 | H | Ethyl | H | CH₃ | 3,4-F₂-C₆H₃ |
| A.633 | H | Ethyl | H | CH₃ | 3,4-Cl₂-C₆H₃ |
| A.634 | H | Ethyl | H | CH₃ | 2,3-F₂-C₆H₃ |
| A.635 | H | Ethyl | H | Ethyl | CH₃ |
| A.636 | H | Ethyl | H | Ethyl | Ethyl |
| A.637 | H | Ethyl | H | Ethyl | n-Propyl |
| A.638 | H | Ethyl | H | Ethyl | iso-Propyl |
| A.639 | H | Ethyl | H | Ethyl | tert. Butyl |
| A.640 | H | Ethyl | H | Ethyl | n-Butyl |
| A.641 | H | Ethyl | H | Ethyl | C₆H₅ |
| A.642 | H | Ethyl | H | Ethyl | 2-F-C₆H₄ |
| A.643 | H | Ethyl | H | Ethyl | 2-Cl-C₆H₄ |
| A.644 | H | Ethyl | H | Ethyl | 2-CH₃-C₆H₄ |
| A.645 | H | Ethyl | H | Ethyl | 2-CF₃-C₆H₄ |
| A.646 | H | Ethyl | H | Ethyl | 3-F-C₆H₄ |
| A.647 | H | Ethyl | H | Ethyl | 3-Cl-C₆H₄ |
| A.648 | H | Ethyl | H | Ethyl | 3-CH₃-C₆H₄ |
| A.649 | H | Ethyl | H | Ethyl | 3-CF₃-C₆H₄ |
| A.650 | H | Ethyl | H | Ethyl | 3-Br-C₆H₄ |
| A.651 | H | Ethyl | H | Ethyl | 4-F-C₆H₄ |
| A.652 | H | Ethyl | H | Ethyl | 4-Cl-C₆H₄ |
| A.653 | H | Ethyl | H | Ethyl | 4-CH₃-C₆H₄ |
| A.654 | H | Ethyl | H | Ethyl | 4-CF₃-C₆H₄ |
| A.655 | H | Ethyl | H | Ethyl | 4-OCH₃-C₆H₄ |
| A.656 | H | Ethyl | H | Ethyl | 4-OCF₃-C₆H₄ |
| A.657 | H | .Ethyl | H | Ethyl | 4-Br-C₆H₄ |
| A.658 | H | Ethyl | H | Ethyl | 4-(CH=NOCH₃)-C₆H₄ |
| A.659 | H | Ethyl | H | Ethyl | 4-(CH=NOEt)-C₆H₄ |
| A.660 | H | Ethyl | H | Ethyl | 4-[C(CH₃)=NOCH₃]-C₆H₄ |
| A.661 | H | Ethyl | H | Ethyl | 4-[C(CH₃)=NOEt]-C₆H₄ |
| A.662 | H | Ethyl | H | Ethyl | 2,4-F₂-C₆H₃ |
| A.663 | H | Ethyl | H | Ethyl | 2,4-Cl₂-C₆H₃ |
| A.664 | H | Ethyl | H | Ethyl | 2,4-(CH₃)₂-C₆H₃ |
| A.665 | H | Ethyl | H | Ethyl | 3,5-F₂-C₆H₃ |
| A.666 | H | Ethyl | H | Ethyl | 3,5-Cl₂-C₆H₃ |
| A.667 | H | Ethyl | H | Ethyl | 3,5-(CH₃)₂-C₆H₃ |
| A.668 | H | Ethyl | H | Ethyl | 2-F-4-Cl-C₆H₃ |
| A.669 | H | Ethyl | H | Ethyl | 2-Cl-4-F-C₆H₃ |
| A.670 | H | Ethyl | H | Ethyl | 3,4-F₂-C₆H₃ |
| A.671 | H | Ethyl | H | Ethyl | 3,4-Cl₂-C₆H₃ |
| A.672 | H | Ethyl | H | Ethyl | 2,3-F₂-C₆H₃ |
| A.673 | H | Ethyl | H | n-Propyl | CH₃ |
| A.674 | H | Ethyl | H | n-Propyl | Ethyl |
| A.675 | H | Ethyl | H | n-Propyl | n-Propyl |
| A.676 | H | Ethyl | H | n-Propyl | iso-Propyl |
| A.677 | H | Ethyl | H | n-Propyl | tert. Butyl |
| A.678 | H | Ethyl | H | n-Propyl | n-Butyl |
| A.679 | H | Ethyl | H | n-Propyl | C₆H₅ |
| A.680 | H | Ethyl | H | n-Propyl | 2-F-C₆H₄ |
| A.681 | H | Ethyl | H | n-Propyl | 2-Cl-C₆H₄ |
| A.682 | H | Ethyl | H | n-Propyl | 2-CH₃-C₆H₄ |
| A.683 | H | Ethyl | H | n-Propyl | 2-CF₃-C₆H₄ |
| A.684 | H | Ethyl | H | n-Propyl | 3-F-C₆H₄ |
| A.685 | H | Ethyl | H | n-Propyl | 3-Cl-C₆H₄ |
| A.686 | H | Ethyl | H | n-Propyl | 3-CH₃-C₆H₄ |
| A.687 | H | Ethyl | H | n-Propyl | 3-CF₃-C₆H₄ |
| A.688 | H | Ethyl | H | n-Propyl | 3-Br-C₆H₄ |
| A.689 | H | Ethyl | H | n-Propyl | 4-F-C₆H₄ |
| A.690 | H | Ethyl | H | n-Propyl | 4-Cl-C₆H₄ |
| A.691 | H | Ethyl | H | n-Propyl | 4-CH₃-C₆H₄ |
| A.692 | H | Ethyl | H | n-Propyl | 4-CF₃-C₆H₄ |
| A.693 | H | Ethyl | H | n-Propyl | 4-OCH₃-C₆H₄ |
| A.694 | H | Ethyl | H | n-Propyl | 4-OCF₃-C₆H₄ |
| A.695 | H | Ethyl | H | n-Propyl | 4-Br-C₆H₄ |
| A.696 | H | Ethyl | H | n-Propyl | 4-(CH=NOCH₃)-C₆H₄ |
| A.697 | H | Ethyl | H | n-Propyl | 4-(CH=NOEt)-C₆H₄ |
| A.698 | H | Ethyl | H | n-Propyl | 4-[C(CH₃)=NOCH₃]-C₆H₄ |
| A.699 | H | Ethyl | H | n-Propyl | 4-[C(CH₃)=NOEt]-C₆H₄ |
| A.700 | H | Ethyl | H | n-Propyl | 2,4-F₂-C₆H₃ |
| A.701 | H | Ethyl | H | n-Propyl | 2,4-Cl₂-C₆H₃ |
| A.702 | H | Ethyl | H | n-Propyl | 2,4-(CH₃)₂-C₆H₃ |
| A.703 | H | Ethyl | H | n-Propyl | 3,5-F₂-C₆H₃ |
| A.704 | H | Ethyl | H | n-Propyl | 3,5-Cl₂-C₆H₃ |
| A.705 | H | Ethyl | H | n-Propyl | 3,5-(CH₃)₂-C₆H₃ |
| A.706 | H | Ethyl | H | n-Propyl | 2-F-4-Cl-C₆H₃ |
| A.707 | H | Ethyl | H | n-Propyl | 2-Cl-4-F-C₆H₃ |
| A.708 | H | Ethyl | H | n-Propyl | 3,4-F₂-C₆H₃ |
| A.709 | H | Ethyl | H | n-Propyl | 3,4-Cl₂-C₆H₃ |
| A.710 | H | Ethyl | H | n-Propyl | 2,3-F₂-C₆H₃ |
| A.711 | H | Ethyl | H | n-Propyl | CH₃ |
| A.712 | H | Ethyl | H | n-Propyl | Ethyl |
| A.713 | H | Ethyl | H | n-Propyl | n-Propyl |
| A.714 | H | Ethyl | H | n-Propyl | iso-Propyl |
| A.715 | H | Ethyl | H | n-Propyl | tert. Butyl |
| A.716 | H | Ethyl | H | n-Propyl | n-Butyl |
| A.717 | H | Ethyl | H | n-Propyl | C₆H₅ |
| A.718 | H | Ethyl | H | n-Propyl | 2-F-C₆H₄ |
| A.719 | H | Ethyl | H | n-Propyl | 2-Cl-C₆H₄ |
| A.720 | H | Ethyl | H | n-Propyl | 2-CH₃-C₆H₄ |
| A.721 | H | Ethyl | H | n-Propyl | 2-CF₃-C₆H₄ |
| A.722 | H | Ethyl | H | n-Propyl | 3-F-C₆H₄ |
| A.723 | H | Ethyl | H | n-Propyl | 3-Cl-C₆H₄ |
| A.724 | H | Ethyl | H | n-Propyl | 3-CH₃-C₆H₄ |
| A.725 | H | Ethyl | H | n-Propyl | 3-CF₃-C₆H₄ |
| A.726 | H | Ethyl | H | n-Propyl | 3-Br-C₆H₄ |
| A.727 | H | Ethyl | H | n-Propyl | 4-F-C₆H₄ |
| A.728 | H | Ethyl | H | n-Propyl | 4-Cl-C₆H₄ |
| A.729 | H | Ethyl | H | n-Propyl | 4-CH₃-C₆H₄ |
| A.730 | H | Ethyl | H | n-Propyl | 4-CF₃-C₆H₄ |
| A.731 | H | Ethyl | H | n-Propyl | 4-OCH₃-C₆H₄ |
| A.732 | H | Ethyl | H | n-Propyl | 4-OCF₃-C₆H₄ |
| A.733 | H | Ethyl | H | n-Propyl | 4-Br-C₆H₄ |
| A.734 | H | Ethyl | H | n-Propyl | 4-(CH=NOCH₃)-C₆H₄ |
| A.735 | H | Ethyl | H | n-Propyl | 4-(CH=NOEt)-C₆H₄ |
| A.736 | H | Ethyl | H | n-Propyl | 4-[C(CH₃)=NOCH₃]-C₆H₄ |
| A.737 | H | Ethyl | H | n-Propyl | 4-[C(CH₃)=NOEt]-C₆H₄ |
| A.738 | H | Ethyl | H | n-Propyl | 2,4-F₂-C₆H₃ |
| A.739 | H | Ethyl | H | n-Propyl | 2,4-Cl₂-C₆H₃ |
| A.740 | H | Ethyl | H | n-Propyl | 2,4-(CH₃)₂-C₆H₃ |
| A.741 | H | Ethyl | H | n-Propyl | 3,5-F₂-C₆H₃ |
| A.742 | H | Ethyl | H | n-Propyl | 3,5-C₁₂-C₆H₃ |
| A.743 | H | Ethyl | H | n-Propyl | 3,5-(CH₃)₂-C₆H₃ |
| A.744 | H | Ethyl | H | n-Propyl | 2-F-4-Cl-C₆H₃ |
| A.745 | H | Ethyl | H | n-Propyl | 2-Cl-4-F-C₆H₃ |
| A.746 | H | Ethyl | H | n-Propyl | 3,4-F₂-C₆H₃ |
| A.747 | H | Ethyl | H | n-Propyl | 3,4-Cl₂-C₆H₃ |
| A.748 | H | Ethyl | H | n-Propyl | 2,3-F₂-C₆H₃ |
| A.749 | H | Ethyl | H | n-Butyl | CH₃ |
| A.750 | H | Ethyl | H | n-Butyl | Ethyl |
| A.751 | H | Ethyl | H | n-Butyl | n-Propyl |
| A.752 | H | Ethyl | H | n-Butyl | iso-Propyl |
| A.753 | H | Ethyl | H | n-Butyl | tert. Butyl |
| A.754 | H | Ethyl | H | n-Butyl | n-Butyl |
| A.755 | H | Ethyl | H | n-Butyl | C₆H₅ |
| A.756 | H | Ethyl | H | n-Butyl | 2-F-C₆H₄ |
| A.757 | H | Ethyl | H | n-Butyl | 2-Cl-C₆H₄ |
| A.758 | H | Ethyl | H | n-Butyl | 2-CH₃-C₆H₄ |
| A.759 | H | Ethyl | H | n-Butyl | 2-CF₃-C₆R₄ |
| A.760 | H | Ethyl | H | n-Butyl | 3-F-C₆H₄ |
| A.761 | H | Ethyl | H | n-Butyl | 3-Cl-C₆H₄ |
| A.762 | H | Ethyl | H | n-Butyl | 3-CH₃-C₆H₄ |
| A.763 | H | Ethyl | H | n-Butyl | 3-CF₃-C₆H₄ |
| A.764 | H | Ethyl | H | n-Butyl | 3-Br-C₆H₄ |
| A.765 | H | Ethyl | H | n-Butyl | 4-F-C₆H₄ |
| A.766 | H | Ethyl | H | n-Butyl | 4-Cl-C₆H₄ |
| A.767 | H | Ethyl | H | n-Butyl | 4-CH₃-C₆H₄ |
| A.768 | H | Ethyl | H | n-Butyl | 4-CF₃-C₆H₄ |
| A.769 | H | Ethyl | H | n-Butyl | 4-OCH₃-C₆H₄ |
| A.770 | H | Ethyl | H | n-Butyl | 4-OCF₃-C₆H₄ |
| A.771 | H | Ethyl | H | n-Butyl | 4-Br-C₆H₄ |
| A.772 | H | Ethyl | H | n-Butyl | 4-(CH=NOCH₃)-C₆H₄ |
| A.773 | H | Ethyl | H | n-Butyl | 4-(CH=NOEt)-C₆H₄ |
| A.774 | H | Ethyl | H | n-Butyl | 4-[C(CH₃)=NOCH₃]-C₆H₄ |
| A.775 | H | Ethyl | H | n-Butyl | 4-[C(CH₃)=NOEt]-C₆H₄ |
| A.776 | H | Ethyl | H | n-Butyl | 2,4-F₂-C₆H₃ |
| A.777 | H | Ethyl | H | n-Butyl | 2,4-Cl₂-C₆H₃ |
| A.778 | H | Ethyl | H | n-Butyl | 2,4-(CH₃)₂-C₆H₃ |
| A.779 | H | Ethyl | H | n-Butyl | 3,5-F₂-C₆H₃ |
| A.780 | H | Ethyl | H | n-Butyl | 3,5-Cl₂-C₆H₃ |
| A.781 | H | Ethyl | H | n-Butyl | 3,5-(CH₃)₂-C₆H₃ |
| A.782 | H | Ethyl | H | n-Butyl | 2-F-4-Cl-C₆H₃ |
| A.783 | H | Ethyl | H | n-Butyl | 2-Cl-4-F-C₆H₃ |
| A.784 | H | Ethyl | H | n-Butyl | 3,4-F₂-C₆H₃ |
| A.785 | H | Ethyl | H | n-Butyl | 3,4-Cl₂-C₆H₃ |
| A.786 | H | Ethyl | H | n-Butyl | 2,3-F₂-C₆H₃ |
| A.787 | H | Ethyl | H | n-Butyl | CH₃ |
| A.788 | H | Ethyl | H | n-Butyl | Ethyl |
| A.789 | H | Ethyl | H | n-Butyl | n-Propyl |
| A.790 | H | Ethyl | H | n-Butyl | iso-Propyl |
| A.791 | H | Ethyl | H | n-Butyl | tert. Butyl |
| A.792 | H | Ethyl | H | n-Butyl | n-Butyl |
| A.793 | H | Ethyl | H | n-Butyl | C₆H₅ |
| A.794 | H | Ethyl | H | n-Butyl | 2-F-C₆H₄ |
| A.795 | H | Ethyl | H | n-Butyl | 2-Cl-C₆H₄ |
| A.796 | H | Ethyl | H | n-Butyl | 2-CH₃-C₆H₄ |
| A.797 | H | Ethyl | H | n-Butyl | 2-CF₃-C₆H₄ |
| A.798 | H | Ethyl | H | n-Butyl | 3-F-C₆H₄ |
| A.799 | H | Ethyl | H | n-Butyl | 3-Cl-C₆H₄ |
| A.800 | H | Ethyl | H | n-Butyl | 3-CH₃-C₆H₄ |
| A.801 | H | Ethyl | H | n-Butyl | 3-CF₃-C₆H₄ |
| A.802 | H | Ethyl | H | n-Butyl | 3-Br-C₆H₄ |
| A.803 | H | Ethyl | H | n-Butyl | 4-F-C₆H₄ |
| A.804 | H | Ethyl | H | n-Butyl | 4-Cl-C₆H₄ |
| A.805 | H | Ethyl | H | n-Butyl | 4-CH₃-C₆H₄ |
| A.806 | H | Ethyl | H | n-Butyl | 4-CF₃-C₆H₄ |
| A.807 | H | Ethyl | H | n-Butyl | 4-OCH₃-C₆H₄ |
| A.808 | H | Ethyl | H | n-Butyl | 4-OCF₃-C₆H₄ |
| A.809 | H | Ethyl | H | n-Butyl | 4-Br-C₆H₄ |
| A.810 | H | Ethyl | H | n-Butyl | 4-(CH=NOCH₃)-C₆H₄ |
| A.811 | H | Ethyl | H | n-Butyl | 4-(CH=NOEt)-C₆H₄ |
| A.812 | H | Ethyl | H | n-Butyl | 4-[C(CH₃)=NOCH₃]-C₆H₄ |
| A.813 | H | Ethyl | H | n-Butyl | 4-[C(CH₃)=NOEt}-C₆H₄ |
| A.814 | H | Ethyl | H | n-Butyl | 2,4-F₂-C₆H₃ |
| A.815 | H | Ethyl | H | n-Butyl | 2,4-Cl₂-C₆H₃ |
| A.816 | H | Ethyl | H | n-Butyl | 2,4-(CH₃)₂-C₆H₃ |
| A.817 | H | Ethyl | H | n-Butyl | 3,5-F₂-C₆H₃ |
| A.818 | H | Ethyl | H | n-Butyl | 3,5-Cl₂-C₆H₃ |
| A.819 | H | Ethyl | H | n-Butyl | 3,5-(CH₃)₂-C₆H₃ |
| A.820 | H | Ethyl | H | n-Butyl | 2-F-4-Cl-C₆H₃ |
| A.821 | H | Ethyl | H | n-Butyl | 2-Cl-4-F-C₆H₃ |
| A.822 | H | Ethyl | H | n-Butyl | 3,4-F₂-C₆H₃ |
| A.823 | H | Ethyl | H | n-Butyl | 3,4-Cl₂-C₆H₃ |
| A.824 | H | Ethyl | H | n-Butyl | 2,3-F₂-C₆H₃ |
| A.825 | H | Ethyl | H | iso-Propyl | CH₃ |
| A.826 | H | Ethyl | H | iso-Propyl | Ethyl |
| A.827 | H | Ethyl | H | iso-Propyl | n-Propyl |
| A.828 | H | Ethyl | H | iso-Propyl | iso-Propyl |
| A.829 | H | Ethyl | H | iso-Propyl | tert. Butyl |
| A.830 | H | Ethyl | H | iso-Propyl | n-Butyl |
| A.831 | H | Ethyl | H | iso-Propyl | C₆H₅ |
| A.832 | H | Ethyl | H | iso-Propyl | 2-F-C₆H₄ |
| A.833 | H | Ethyl | H | iso-Propyl | 2-Cl-C₆H₄ |
| A.834 | H | Ethyl | H | iso-Propyl | 2-CH₃-C₆H₄ |
| A.835 | H | Ethyl | H | iso-Propyl | 2-CF3-C₆H₄ |
| A.836 | H | Ethyl | H | iso-Propyl | 3-F-C₆H₄ |
| A.837 | H | Ethyl | H | iso-Propyl | 3-Cl-C₆H₄ |
| A.838 | H | Ethyl | H | iso-Propyl | 3-CH₃-C₆H₄ |
| A.839 | H | Ethyl | H | iso-Propyl | 3-CF₃-C₆H₄ |
| A.840 | H | Ethyl | H | iso-Propyl | 3-Br-C₆H₄ |
| A.841 | H | Ethyl | H | iso-Propyl | 4-F-C₆H₄ |
| A.842 | H | Ethyl | H | iso-Propyl | 4-Cl-C₆H₄ |
| A.843 | H | Ethyl | H | iso-Propyl | 4-CH₃-C₆H₄ |
| A.844 | H | Ethyl | H | iso-Propyl | 4-CF₃-C₆H₄ |
| A.845 | H | Ethyl | H | iso-Propyl | 4-OCH₃-C₆H₄ |
| A.846 | H | Ethyl | H | iso-Propyl | 4-OCF₃-C₆H₄ |
| A.847 | H | Ethyl | H | iso-Propyl | 4-Br-C₆H₄ |
| A.848 | H | Ethyl | H | iso-Propyl | 4-(CH=NOCH₃)-C₆R₄ |
| A.849 | H | Ethyl | H | iso-Propyl | 4-(CH=NOEt)-C₆H₄ |
| A.850 | H | Ethyl | H | iso-Propyl | 4-[C(CH₃)=NOCH₃]-C₆H₄ |
| A.851 | H | Ethyl | H | iso-Propyl | 4-[C(CH₃)=NOEt]-C₆H₄ |
| A.852 | H | Ethyl | H | iso-Propyl | 2,4-F₂-C₆H₃ |
| A.853 | H | Ethyl | H | iso-Propyl | 2,4-Cl₂-C₆H₃ |
| A.854 | H | Ethyl | H | iso-Propyl | 2,4-(CH₃)₂-C₆H₃ |
| A.855 | H | Ethyl | H | iso-Propyl | 3,5-F₂-C₆H₃ |
| A.856 | H | Ethyl | H | iso-Propyl | 3,5-Cl₂-C₆H₃ |
| A.857 | H | Ethyl | H | iso-Propyl | 3,5-(CH₃)₂-C₆H₃ |
| A.858 | H | Ethyl | H | iso-Propyl | 2-F-4-Cl-C₆H₃ |
| A.859 | H | Ethyl | H | iso-Propyl | 2-Cl-4-F-C₆H₃ |
| A.860 | H | Ethyl | H | iso-Propyl | 3,4-F₂-C₆H₃ |
| A.861 | H | Ethyl | H | iso-Propyl | 3,4-Cl₂-C₆H₃ |
| A.862 | H | Ethyl | H | iso-Propyl | 2,3-F₂-C₆H₃ |
| A.863 | H | Ethyl | H | iso-Propyl | CH₃ |
| A.864 | H | Ethyl | H | iso-Propyl | Ethyl |
| A.865 | H | Ethyl | H | iso-Propyl | n-Propyl |
| A.866 | H | Ethyl H | | iso-Propyl | iso-Propyl |
| A.867 | H | Ethyl | H | iso-Propyl | tert. Butyl |
| A.868 | H | Ethyl | H | iso-Propyl | n-Butyl |
| A.869 | H | Ethyl | H | iso-Propyl | C₆H₅ |
| A.870 | H | Ethyl | H | iso-Propyl | 2-F-C₆H₄ |
| A.871 | H | Ethyl | H | iso-Propyl | 2-Cl-C₆H₄ |
| A.872 | H | Ethyl | H | iso-Propyl | 2-CH₃-C₆H₄ |
| A.873 | H | Ethyl | H | iso-Propyl | 2-CF₃-C₆H₄ |
| A.874 | H | Ethyl | H | iso-Propyl | 3-F-C₆H₄ |
| A.875 | H | Ethyl | H | iso-Propyl | 3-Cl-C₆H₄ |
| A.876 | H | Ethyl | H | iso-Propyl | 3-CH₃-C₆H₄ |
| A.877 | H | Ethyl | H | iso-Propyl | 3-CF₃-C₆H₄ |
| A.878 | H | Ethyl | H | iso-Propyl | 3-Br-C₆H₄ |
| A.879 | H | Ethyl | H | iso-Propyl | 4-F-C₆H₄ |
| A.880 | H | Ethyl | H | iso-Propyl | 4-Cl-C₆H₄ |
| A.881 | H | Ethyl | H | iso-Propyl | 4-CH₃-C₆H₄ |
| A.882 | H | Ethyl | H | iso-Propyl | 4-CF₃-C₆H₄ |
| A.883 | H | Ethyl | H | iso-Propyl | 4-OCH₃-C₆H₄ |
| A.884 | H | Ethyl | H | iso-Propyl | 4-OCF₃-C₆H₄ |
| A.885 | H | Ethyl | H | iso-Propyl | 4-Br-C₆H₄ |
| A.886 | H | Ethyl | H | iso-Propyl | 4-(CH=NOCH₃)-C₆H₄ |
| A.887 | H | Ethyl | H | iso-Propyl | 4-(CH=NOEt)-C₆H₄ |
| A.888 | H | Ethyl | H | iso-Propyl | 4-[C(CH₃)=NOCH₃]-C₆H₄ |
| A.889 | H | Ethyl | H | iso-Propyl | 4-[C(CH₃)=NOEt]-C₆H₄ |
| A.890 | H | Ethyl | H | iso-Propyl | 2,4-F₂-C₆H₃ |
| A.891 | H | Ethyl | H | iso-Propyl | 2,4-Cl₂-C₆H₃ |
| A.892 | H | Ethyl | H | iso-Propyl | 2,4-(CH₃)₂-C₆H₃ |
| A.893 | H | Ethyl | H | iso-Propyl | 3,5-F₂-C₆H₃ |
| A.894 | H | Ethyl | H | iso-Propyl | 3,5-Cl₂-C₆H₃ |
| A.895 | H | Ethyl | H | iso-Propyl | 3,5-(CH₃)₂-C₆H₃ |
| A.896 | H | Ethyl | H | iso-Propyl | 2-F-4-Cl-C₆H₃ |
| A.897 | H | Ethyl | H | iso-Propyl | 2-Cl-4-F-C₆H₃ |
| A.898 | H | Ethyl | H | iso-Propyl | 3,4-F₂-C₆H₃ |
| A.899 | H | Ethyl | H | iso-Propyl | 3,4-Cl₂-C₆H₃ |
| A.900 | H | Ethyl | H | iso-Propyl | 2,3-F₂-C₆H₃ |
| A.901 | H | Ethyl | H | tert. Butyl | CH₃ |
| A.902 | H | Ethyl | H | tert. Butyl | Ethyl |
| A.903 | H | Ethyl | H | tert. Butyl | n-Propyl |
| A.904 | H | Ethyl | H | tert. Butyl | iso-Propyl |
| A.905 | H | Ethyl | H | tert. Butyl | tert. Butyl |
| A.906 | H | Ethyl | H | tert. Butyl | n-Butyl |
| A.907 | H | Ethyl | H | tert. Butyl | C₆H₅ |
| A.908 | H | Ethyl | H | tert. Butyl | 2-F-C₆H₄ |
| A.909 | H | Ethyl | H | tert. Butyl | 2-Cl-C₆H₄ |
| A.910 | H | Ethyl | H | tert. Butyl | 2-CH₃-C₆H₄ |
| A.911 | H | Ethyl | H | tert. Butyl | 2-CF₃-C₆H₄ |
| A.912 | H | Ethyl | H | tert. Butyl | 3-F-C₆H₄ |
| A.913 | H | Ethyl | H | tert. Butyl | 3-Cl-C₆H₄ |
| A.914 | H | Ethyl | H | tert. Butyl | 3-CH₃-C₆H₄ |
| A.915 | H | Ethyl | H | tert. Butyl | 3-CF₃-C₆H₄ |
| A.916 | H | Ethyl | H | tert. Butyl | 3-Br-C₆H₄ |
| A.917 | H | Ethyl | H | tert. Butyl | 4-F-C₆H₄ |
| A.918 | H | Ethyl | H | tert. Butyl | 4-Cl-C₆H₄ |
| A.919 | H | Ethyl | H | tert. Butyl | 4-CH₃-C₆H₄ |
| A.920 | H | Ethyl | H | tert. Butyl | 4-CF₃-C₆H₄ |
| A.921 | H | Ethyl | H | tert. Butyl | 4-OCH₃-C₆H₄ |
| A.922 | H | Ethyl | H | tert. Butyl | 4-OCF₃-C₆H₄ |
| A.923 | H | Ethyl | H | tert. Butyl | 4-Br-C₆H₄ |
| A.924 | H | Ethyl | H | tert. Butyl | 4-(CH=NOCH₃)-C₆H₄ |
| A.925 | H | Ethyl | H | tert. Butyl | 4-(CH=NOEt)-C₆H₄ |
| A.926 | H | Ethyl | H | tert. Butyl | 4-[C(CH₃)=NOCH₃]-C₆H₄ |
| A.927 | H | Ethyl | H | tert. Butyl | 4-[C(CH₃)=NOEt]-C₆H₄ |
| A.928 | H | Ethyl | H | tert. Butyl | 2,4-F₂-C₆H₃ |
| A.929 | H | Ethyl | H | tert. Butyl | 2,4-Cl₂-C₆H₃ |
| A.930 | H | Ethyl | H | tert. Butyl | 2,4-(CH₃)₂-C₆H₃ |
| A.931 | H | Ethyl | H | tert. Butyl | 3,5-F₂-C₆H₃ |
| A.932 | H | Ethyl | H | tert. Butyl | 3,5-Cl₂-C₆H₃ |
| A.933 | H | Ethyl | H | tert. Butyl | 3,5-(CH₃)₂-C₆H₃ |
| A.934 | H | Ethyl | H | tert. Butyl | 2-F-4-Cl-C₆H₃ |
| A.935 | H | Ethyl | H | tert. Butyl | 2-Cl-4-F-C₆H₃ |
| A.936 | H | Ethyl | H | tert. Butyl | 3,4-F₂-C₆H₃ |
| A.937 | H | Ethyl | H | tert. Butyl | 3,4-Cl₂-C₆H₃ |
| A.938 | H | Ethyl | H | tert. Butyl | 2,3-F₂-C₆H₃ |
| A.939 | H | Ethyl | H | tert. Butyl | CH₃ |
| A.940 | H | Ethyl | H | tert. Butyl | Ethyl |
| A.941 | H | Ethyl | H | tert. Butyl | n-Propyl |
| A.942 | H | Ethyl | H | tert. Butyl | iso-Propyl |
| A.943 | H | Ethyl | H | tert. Butyl | tert. Butyl |
| A.944 | H | Ethyl | H | tert. Butyl | n-Butyl |
| A.945 | H | Ethyl | H | tert. Butyl | C₆H₅ |
| A.946 | H | Ethyl | H | tert. Butyl | 2-F-C₆H₄ |
| A.947 | H | Ethyl | H | tert. Butyl | 2-Cl-C₆H₄ |
| A.948 | H | Ethyl | H | tert. Butyl | 2-CH₃-C₆H₄ |
| A.949 | H | Ethyl | H | tert. Butyl | 2-CF₃-C₆H₄ |
| A.950 | H | Ethyl | H | tert. Butyl | 3-F-C₆H₄ |
| A.951 | H | Ethyl | H | tert. Butyl | 3-Cl-C₆H₄ |
| A.952 | H | Ethyl | H | tert. Butyl | 3-CH₃-C₆H₄ |
| A.953 | H | Ethyl | H | tert. Butyl | 3-CF₃-C₆H₄ |
| A.954 | H | Ethyl | H | tert. Butyl | 3-Br-C₆H₄ |
| A.955 | H | Ethyl | H | tert. Butyl | 4-F-C₆H₄ |
| A.956 | H | Ethyl | H | tert. Butyl | 4-Cl-C₆H₄ |
| A.957 | H | Ethyl | H | tert. Butyl | 4-CH₃-C₆H₄ |
| A.958 | H | Ethyl | H | tert. Butyl | 4-CF₃-C₆H₄ |
| A.959 | H | Ethyl | H | tert. Butyl | 4-OCH₃-C₆H₄ |
| A.960 | H | Ethyl | H | tert. Butyl | 4-OCF₃-C₆H₄ |
| A.961 | H | Ethyl | H | tert. Butyl | 4-Br-C₆H₄ |
| A.962 | H | Ethyl | H | tert. Butyl | 4-(CH=NOCH₃)-C₆H₄ |
| A.963 | H | Ethyl | H | tert. Butyl | 4-(CH=NOEt)-C₆H₄ |
| A.964 | H | Ethyl | H | tert. Butyl | 4-[C(CH₃)=NOCH₃]-C₆H₄ |
| A.965 | H | Ethyl | H | tert. Butyl | 4-[C(CH₃)=NOEt]-C₆H₄ |
| A.966 | H | Ethyl | H | tert. Butyl | 2,4-F₂-C₆H₃ |
| A.967 | H | Ethyl | H | tert. Butyl | 2,4-Cl₂-C₆H₃ |
| A.968 | H | Ethyl | H | tert. Butyl | 2,4-(CH₃)₂-C₆H₃ |
| A.969 | H | Ethyl | H | tert. Butyl | 3,5-F₂-C₆H₃ |
| A.970 | H | Ethyl | H | tert. Butyl | 3,5-Cl₂-C₆H₃ |
| A.971 | H | Ethyl | H | tert. Butyl | 3,5-(CH₃)₂-C₆H₃ |
| A.972 | H | Ethyl | H | tert. Butyl | 2-F-4-Cl-C₆H₃ |
| A.973 | H | Ethyl | H | tert. Butyl | 2-Cl-4-F-C₆H₃ |
| A.974 | H | Ethyl | H | tert. Butyl | 3,4-F₂-C₆H₃ |
| A.975 | H | Ethyl | H | tert. Butyl | 3,4-Cl₂-C₆H₃ |
| A.976 | H | Ethyl | H | tert. Butyl | 2,3-F₂-C₆H₃ |
| A.977 | H | Cl | H | H | CH₃ |
| A.978 | H | Cl | H | H | Ethyl |
| A.979 | H | Cl | H | H | n-Propyl. |
| A.980 | H | Cl | H | H | iso-Propyl |
| A.981 | H | Cl | H | H | tert. Butyl |
| A.982 | H | Cl | H | H | n-Butyl |
| A.983 | H | Cl | H | H | C₆H₅ |
| A.984 | H | Cl | H | H | 2-F-C₆H₄ |
| A.985 | H | Cl | H | H | 2-Cl-C₆H₄ |
| A.986 | H | Cl | H | H | 2-CH₃-C₆H₄ |
| A.987 | H | Cl | H | H | 2-CF₃-C₆H₄ |
| A.988 | H | Cl | H | H | 3-F-C₆H₄ |
| A.989 | H | Cl | H | H | 3-Cl-C₆H₄ |
| A.990 | H | Cl | H | H | 3-CH₃-C₆H₄ |
| A.991 | H | Cl | H | H | 3-CF₃-C₆H₄ |
| A.992 | H | Cl | H | H | 3-Br-C₆H₄ |
| A.993 | H | Cl | H | H | 4-F-C₆H₄ |
| A.994 | H | Cl | H | H | 4-Cl-C₆H₄ |
| A.995 | H | Cl | H | H | 4-CH₃-C₆H₄ |
| A.996 | H | Cl | H | H | 4-CF₃-C₆H₄ |
| A.997 | H | Cl | H | H | 4-OCH₃-C₆H₄ |
| A.998 | H | Cl | H | H | 4-OCF₃-C₆H₄ |
| A.999 | H | Cl | H | H | 4-Br-C₆H₄ |
| A.1000 | H | Cl | H | H | 4-(CH=NOCH₃)-C₆H₄ |
| A.1001 | H | Cl | H | H | 4-(CH=NOEt)-C₆H₄ |
| A.1002 | H | Cl | H | H | 4-[C(CH₃)=NOCH₃]-C₆H₄ |
| A.1003 | H | Cl | H | H | 4-[C(CH₃)=NOEt]-C₆H₄ |
| A.1004 | H | Cl | H | H | 2,4-F₂-C₆H₃ |
| A.1005 | H | Cl | H | H | 2,4-Cl₂-C₆H₃ |
| A.1006 | H | Cl | H | H | 2,4-(CH₃)₂-C₆H₃ |
| A.1007 | H | Cl | H | H | 3,5-F₂-C₆H₃ |
| A.1008 | H | Cl | H | H | 3,5-Cl₂-C₆H₃ |
| A.1009 | H | Cl | H | H | 3,5-(CH₃)₂-C₆H₃ |
| A.1010 | H | Cl | H | H | 2-F-4-Cl-C₆H₃ |
| A.1011 | H | Cl | H | H | 2-Cl-4-F-C₆H₃ |
| A.1012 | H | Cl | H | H | 3,4-F₂-C₆H₃ |
| A.1013 | H | Cl | H | H | 3,4-Cl₂-C₆H₃ |
| A.1014 | H | Cl | H | H | 2,3-F₂-C₆H₃ |
| A.1015 | H | Cl | H | CH₃ | CH₃ |
| A.1016 | H | Cl | H | CH₃ | Ethyl |
| A.1017 | H | Cl | H | CH₃ | n-Propyl |
| A.1018 | H | Cl | H | CH₃ | iso-Propyl |
| A.1019 | H | Cl | H | CH₃ | tert. Butyl |
| A.1020 | H | Cl | H | CH₃ | n-Butyl |
| A.1021 | H | Cl | H | CH₃ | C₆H₅ |
| A.1022 | H | Cl | H | CH₃ | 2-F-C₆H₄ |
| A.1023 | H | Cl | H | CH₃ | 2-Cl-C₆H₄ |
| A.1024 | H | Cl | H | CH₃ | 2-CH₃-C₆H₄ |
| A.1025 | H | Cl | H | CH₃ | 2-CF₃-C₆H₄ |
| A.1026 | H | Cl | H | CH₃ | 3-F-C₆H₄ |
| A.1027 | H | Cl | H | CH₃ | 3-Cl-C₆H₄ |
| A.1028 | H | Cl | H | CH₃ | 3-CH₃-C₆H₄ |
| A.1029 | H | Cl | H | CH₃ | 3-CF₃-C₆H₄ |
| A.1030 | H | Cl | H | CH₃ | 3-Br-C₆H₄ |
| A.1031 | H | Cl | H | CH₃ | 4-F-C₆H₄ |
| A.1032 | H | Cl | H | CH₃ | 4-Cl-C₆H₄ |
| A.1033 | H | Cl | H | CH₃ | 4-CH₃-C₆H₄ |
| A.1034 | H | Cl | H | CH₃ | 4-CF₃-C₆H₄ |
| A.1035 | H | Cl | H | CH₃ | 4-OCH₃-C₆H₄ |
| A.1036 | H | Cl | H | CH₃ | 4-OCF₃-C₆H₄ |
| A.1037 | H | Cl | H | CH₃ | 4-Br-C₆H₄ |
| A.1038 | H | Cl | H | CH₃ | 4-CH=NOCH₃-C₆H₄ |
| A.1039 | H | Cl | H | CH₃ | 4-CH=NOEt-C₆H₄ |
| A.1040 | H | Cl | H | CH₃ | 4-[C(CH₃)=NOCH₃] -C₆H₄ |
| A.1041 | H | Cl | H | CH₃ | 4-[C(CH₃)=NOEt]-C₆H₄ |
| A.1042 | H | Cl | H | CH₃ | 2,4-F₂-C₆H₃ |
| A.1043 | H | Cl | H | CH₃ | 2,4-Cl₂-C₆H₃ |
| A.1044 | H | Cl | H | CH₃ | 2,4-(CH₃)₂-C₆H₃ |
| A.1045 | H | Cl | H | CH₃ | 3,5-F₂-C₆H₃ |
| A.1046 | H | Cl | H | CH₃ | 3,5-Cl₂-C₆H₃ |
| A.1047 | H | Cl | H | CH₃ | 3,5-(CH₃)₂-C₆H₃ |
| A.1048 | H | Cl | H | CH₃ | 2-F-4-Cl-C₆H₃ |
| A.1049 | H | Cl | H | CH₃ | 2-Cl-4-F-C₆H₃ |
| A.1050 | H | Cl | H | CH₃ | 3,4-F₂-C₆H₃ |
| A.1051 | H | Cl | H | CH₃ | 3,4-Cl₂-C₆H₃ |
| A.1052 | H | Cl | H | CH₃ | 2,3-F₂-C₆H₃ |
| A.1053 | H | Cl | H | Ethyl | CH₃ |
| A.1054 | H | Cl | H | Ethyl | Ethyl |
| A.1055 | H | Cl | H | Ethyl | n-Propyl |
| A.1056 | H | Cl | H | Ethyl | iso-Propyl |
| A.1057 | H | Cl | H | Ethyl | tert. Butyl |
| A.1058 | H | Cl | H | Ethyl | n-Butyl |
| A.1059 | H | Cl | H | Ethyl | C₆H₅ |
| A.1060 | H | Cl | H | Ethyl | 2-F-C₆H₄ |
| A.1061 | H | Cl | H | Ethyl | 2-Cl-C₆H₄ |
| A.1062 | H | Cl | H | Ethyl | 2-CH₃-C₆H₄ |
| A.1063 | H | Cl | H | Ethyl | 2-CF₃-C₆H₄ |
| A.1064 | H | Cl | H | Ethyl | 3-F-C₆H₄ |
| A.1065 | H | Cl | H | Ethyl | 3-Cl-C₆H₄ |
| A.1066 | H | Cl | H | Ethyl | 3-CH₃-C₆H₄ |
| A.1067 | H | Cl | H | Ethyl | 3-CF₃-C₆H₄ |
| A.1068 | H | Cl | H | Ethyl | 3-Br-C₆H₄ |
| A.1069 | H | Cl | H | Ethyl | 4-F-C₆H₄ |
| A.1070 | H | Cl | H | Ethyl | 4-Cl-C₆H₄ |
| A.1071 | H | Cl | H | Ethyl | 4-CH₃-C₆H₄ |
| A.1072 | H | Cl | H | Ethyl | 4-CF₃-C₆H₄ |
| A.1073 | H | Cl | H | Ethyl | 4-OCH₃-C₆H₄ |
| A.1074 | H | Cl | H | Ethyl | 4-OCF₃-C₆H₄ |
| A.1075 | H | Cl | H | Ethyl | 4-Br-C₆H₄ |
| A.1076 | H | Cl | H | Ethyl | 4-CH=NOCH₃-C₆H₄ |
| A.1077 | H | Cl | H | Ethyl | 4-CH=NOEt-C₆H₄ |
| A.1078 | H | Cl | H | Ethyl | 4-[C(CH₃)=NOCH₃]-C₆H₄ |
| A.1079 | H | Cl | H | Ethyl | 4-[C(CH₃)=NOEt]-C₆H₄ |
| A.1080 | H | Cl | H | Ethyl | 2,4-F₂-C₆H₃ |
| A.1081 | H | Cl | H | Ethyl | 2,4-Cl₂-C₆H₃ |
| A.1082 | H | Cl | H | Ethyl | 2,4-(CH₃)₂-C₆H₃ |
| A.1083 | H | Cl | H | Ethyl | 3,5-F₂-C₆H₃ |
| A.1084 | H | Cl | H | Ethyl | 3,5-Cl₂-C₆H₃ |
| A.1085 | H | Cl | H | Ethyl | 3,5-(CH₃)₂-C₆H₃ |
| A.1086 | H | Cl | H | Ethyl | 2-F-4-Cl-C₆H₃ |
| A.1087 | H | Cl | H | Ethyl | 2-Cl-4-F-C₆H₃ |
| A.1088 | H | Cl | H | Ethyl | 3,4-F₂-C₆H₃ |
| A.1089 | H | Cl | H | Ethyl | 3,4-Cl₂-C₆H₃ |
| A.1090 | H | Cl | H | Ethyl | 2,3-F₂-C₆H₃ |
| A.1091 | H | Cl | H | n-Propyl | CH₃ |
| A.1092 | H | Cl | H | n-Propyl | Ethyl |
| A.1093 | H | Cl | H | n-Propyl | n-Propyl |
| A.1094 | H | Cl | H | n-Propyl | iso-Propyl |
| A.1095 | H | Cl | H | n-Propyl | tert. Butyl |
| A.1096 | H | Cl | H | n-Propyl | n-Butyl |
| A.1097 | H | Cl | H | n-Propyl | C₆H₅ |
| A.1098 | H | Cl | H | n-Propyl | 2-F-C₆H₄ |
| A.1099 | H | Cl | H | n-Propyl | 2-Cl-C₆H₄ |
| A.1100 | H | Cl | H | n-Propyl | 2-CH₃-C₆H₄ |
| A.1101 | H | Cl | H | n-Propyl | 2-CF₃-C₆H₄ |
| A.1102 | H | Cl | H | n-Propyl | 3-F-C₆H₄ |
| A.1103 | H | Cl | H | n-Propyl | 3-Cl-C₆H₄ |
| A.1104 | H | Cl | H | n-Propyl | 3-CH₃-C₆H₄ |
| A.1105 | H | Cl | H | n-Propyl | 3-CF₃-C₆H₄ |
| A.1106 | H | Cl | H | n-Propyl | 3-Br-C₆H₄ |
| A.1107 | H | Cl | H | n-Propyl | 4-F-C₆H₄ |
| A.1108 | H | Cl | H | n-Propyl | 4-Cl-C₆H₄ |
| A.1109 | H | Cl | H | n-Propyl | 4-CH₃-C₆H₄ |
| A.1110 | H | Cl | H | n-Propyl | 4-CF₃-C₆H₄ |
| A.1111 | H | Cl | H | n-Propyl | 4-OCH₃-C₆H₄ |
| A.1112 | H | Cl | H | n-Propyl | 4-OCF₃-C₆H₄ |
| A.1113 | H | Cl | H | n-Propyl | 4-Br-C₆H₄ |
| A.1114 | H | Cl | H | n-Propyl | 4-CH=NOCH₃-C₆H₄ |
| A.1115 | H | Cl | H | n-Propyl | 4-CH=NOEt-C₆H₄ |
| A.1116 | H | Cl | H | n-Propyl | 4-[C(CH₃)=NOCH₃]-C₆H₄ |
| A.1117 | H | Cl | H | n-Propyl | 4-[C(CH₃)=NOEt]-C₆H₄ |
| A.1118 | H | Cl | H | n-Propyl | 2,4-F₂-C₆H₃ |
| A.1119 | H | Cl | H | n-Propyl | 2,4-Cl₂-C₆H₃ |
| A.1120 | H | Cl | H | n-Propyl | 2,4-(CH₃)₂-C₆H₃ |
| A.1121 | H | Cl | H | n-Propyl | 3,5-F₂-C₆H₃ |
| A.1122 | H | Cl | H | n-Propyl | 3,5-Cl₂-C₆H₃ |
| A.1123 | H | Cl | H | n-Propyl | 3,5-(CH₃)₂-C₆H₃ |
| A.1124 | H | Cl | H | n-Propyl | 2-F-4-Cl-C₆H₃ |
| A.1125 | H | Cl | H | n-Propyl | 2-Cl-4-F-C₆H₃ |
| A.1126 | H | Cl | H | n-Propyl | 3,4-F₂-C₆H₃ |
| A.1127 | H | Cl | H | n-Propyl | 3,4-Cl₂-C₆H₃ |
| A.1128 | H | Cl | H | n-Propyl | 2,3-F₂-C₆H₃ |
| A.1129 | H | Cl | H | n-Propyl | CH₃ |
| A.1130 | H | Cl | H | n-Propyl | Ethyl |
| A.1131 | H | Cl | H | n-Propyl | n-Propyl |
| A.1132 | H | Cl | H | n-Propyl | iso-Propyl |
| A.1133 | H | Cl | H | n-Propyl | tert. Butyl |
| A.1134 | H | Cl | H | n-Propyl | n-Butyl |
| A.1135 | H | Cl | H | n-Propyl | C₆H₅ |
| A.1136 | H | Cl | H | n-Propyl | 2-F-C₆H₄ |
| A.1137 | H | Cl | H | n-Propyl | 2-Cl-C₆H₄ |
| A.1138 | H | Cl | H | n-Propyl | 2-CH₃-C₆H₄ |
| A.1139 | H | Cl | H | n-Propyl | 2-CF₃-C₆H₄ |
| A.1140 | H | Cl | H | n-Propyl | 3-F-C₆H₄ |
| A.1141 | H | Cl | H | n-Propyl | 3-Cl-C₆H₄ |
| A.1142 | H | Cl | H | n-Propyl | 3-CH₃-C₆H₄ |
| A.1143 | H | Cl | H | n-Propyl | 3-CF₃-C₆H₄ |
| A.1144 | H | Cl | H | n-Propyl | 3-Br-C₆H₄ |
| A.1145 | H | Cl | H | n-Propyl | 4-F-C₆H₄ |
| A.1146 | H | Cl | H | n-Propyl | 4-Cl-C₆H₄ |
| A.1147 | H | Cl | H | n-Propyl | 4-CH₃-C₆H₄ |
| A.1148 | H | Cl | H | n-Propyl | 4-CF₃-C₆H₄ |
| A.1149 | H | Cl | H | n-Propyl | 4-OCH₃-C₆H₄ |
| A.1150 | H | Cl | H | n-Propyl | 4-OCF₃-C₆H₄ |
| A.1151 | H | Cl | H | n-Propyl | 4-Br-C₆H₄ |
| A.1152 | H | Cl | H | n-Propyl | 4-CH=NOCH₃-C₆H₄ |
| A.1153 | H | Cl | H | n-Propyl | 4-CH=NOEt-C₆H₄ |
| A.1154 | H | Cl | H | n-Propyl | 4-[C(CH₃)=NOCH₃]-C₆H₄ |
| A.1155 | H | Cl | H | n-Propyl | 4-[C(CH₃)=NOEt]-C₆H₄ |
| A.1156 | H | Cl | H | n-Propyl | 2,4-F₂-C₆H₃ |
| A.1157 | H | Cl | H | n-Propyl | 2,4-Cl₂-C₆H₃ |
| A.1158 | H | Cl | H | n-Propyl | 2,4-(CH₃)₂-C₆H₃ |
| A.1159 | H | Cl | H | n-Propyl | 3,5-F₂-C₆H₃ |
| A.1160 | H | Cl | H | n-Propyl | 3,5-Cl₂-C₆H₃ |
| A.1161 | H | Cl | H | n-Propyl | 3,5-(CH₃)₂-C₆H₃ |
| A.1162 | H | Cl | H | n-Propyl | 2-F-4-Cl-C₆H₃ |
| A.1163 | H | Cl | H | n-Propyl | 2-Cl-4-F-C₆H₃ |
| A.1164 | H | Cl | H | n-Propyl | 3,4-F₂-C₆H₃ |
| A.1165 | H | Cl | H | n-Propyl | 3,4-Cl₂-C₆H₃ |
| A.1166 | H | Cl | H | n-Propyl | 2,3-F₂-C₆H₃ |
| A.1167 | H | Cl | H | n-Butyl | CH₃ |
| A.1168 | H | Cl | H | n-Butyl | Ethyl |
| A.1169 | H | Cl | H | n-Butyl | n-Propyl |
| A.1170 | H | Cl | H | n-Butyl | iso-Propyl |
| A.1171 | H | Cl | H | n-Butyl | tert. Butyl |
| A.1172 | H | Cl | H | n-Butyl | n-Butyl |
| A.1173 | H | Cl | H | n-Butyl | C₆H₅ |
| A.1174 | H | Cl | H | n-Butyl | 2-F-C₆H₄ |
| A.1175 | H | Cl | H | n-Butyl | 2-Cl-C₆H₄ |
| A.1176 | H | Cl | H | n-Butyl | 2-CH₃-C₆H₄ |
| A.1177 | H | Cl | H | n-Butyl | 2-CF₃-C₆H₄ |
| A.1178 | H | Cl | H | n-Butyl | 3-F-C₆H₄ |
| A.1179 | H | Cl | H | n-Butyl | 3-Cl-C₆H₄ |
| A.1180 | H | Cl | H | n-Butyl | 3-CH₃-C₆H₄ |
| A.1181 | H | Cl | H | n-Butyl | 3-CF₃-C₆H₄ |
| A.1182 | H | Cl | H | n-Butyl | 3-Br-C₆H₄ |
| A.1183 | H | Cl | H | n-Butyl | 4-F-C₆H₄ |
| A.1184 | H | Cl | H | n-Butyl | 4-Cl-C₆H₄ |
| A.1185 | H | Cl | H | n-Butyl | 4-CH₃-C₆H₄ |
| A.1186 | H | Cl | H | n-Butyl | 4-CF₃-C₆H₄ |
| A.1187 | H | Cl | H | n-Bufcyl | 4-OCH₃-C₆H₄ |
| A.1188 | H | Cl | H | n-Butyl | 4-OCF₃-C₆H₄ |
| A.1189 | H | Cl | H | n-Butyl | 4-Br-C₆H₄ |
| A.1190 | H | Cl | H | n-Butyl | 4-CH=NOCH₃-C₆H₄ |
| A.1191 | H | Cl | H | n-Butyl | 4-CH=NOEt-C₆H₄ |
| A.1192 | H | Cl | H | n-Butyl | 4-[C(CH₃)=NOCH₃]-C₆H₄ |
| A.1193 | H | Cl | H | n-Butyl | 4-[C(CH₃)=NoEt]-C₆H₄ |
| A.1194 | H | Cl | H | n-Butyl | 2,4-F₂-C₆H₃ |
| A.1195 | H | Cl | H | n-Butyl | 2,4-Cl₂-C₆H₃ |
| A.1196 | H | Cl | H | n-Butyl | 2,4-(CH₃)₂-C₆H₃ |
| A.1197 | H | Cl | H | n-Butyl | 3,5-F₂-C₆H₃ |
| A.1198 | H | Cl | H | n-Butyl | 3,5-Cl₂-C₆H₃ |
| A.1199 | H | Cl | H | n-Butyl | 3,5-(CH₃)₂-C₆H₃ |
| A.1200 | H | Cl | H | n-Butyl | 2-F-4-Cl-C₆H₃ |
| A.1201 | H | Cl | H | n-Butyl | 2-Cl-4-F-C₆H₃ |
| A.1202 | H | Cl | H | n-Butyl | 3,4-F₂-C₆H₃ |
| A.1203 | H | Cl | H | n-Butyl | 3,4-Cl₂-C₆H₃ |
| A.1204 | H | Cl | H | n-Butyl | 2,3-F₂-C₆H₃ |
| A.1205 | H | Cl | H | n-Butyl | CH₃ |
| A.1206 | H | Cl | H | n-Butyl | Ethyl |
| A.1207 | H | Cl | H | n-Butyl | n-Propyl |
| A.1208 | H | Cl | H | n-Butyl | iso-Propyl |
| A.1209 | H | Cl | H | n-Butyl | tert. Butyl |
| A.1210 | H | Cl | H | n-Butyl | n-Butyl |
| A.1211 | H | Cl | H | n-Butyl | C₆H₅ |
| A.1212 | H | Cl | H | n-Butyl | 2-F-C₆H₄ |
| A.1213 | H | Cl | H | n-Butyl | 2-Cl-C₆H₄ |
| A.1214 | H | Cl | H | n-Butyl | 2-CH₃-C₆H₄ |
| A.1215 | H | Cl | H | n-Butyl | 2-CF₃-C₆H₄ |
| A.1216 | H | Cl | H | n-Butyl | 3-F-C₆H₄ |
| A.1217 | H | Cl | H | n-Butyl | 3-Cl-C₆H₄ |
| A.1218 | H | Cl | H | n-Butyl | 3-CH₃-C₆H₄ |
| A.1219 | H | Cl | H | n-Butyl | 3-CF₃-C₆H₄ |
| A.1220 | H | Cl | H | n-Butyl | 3-Br-C₆H₄ |
| A.1221 | H | Cl | H | n-Butyl | 4-F-C₆H₄ |
| A.1222 | H | Cl | H | n-Butyl | 4-Cl-C₆H₄ |
| A.1223 | H | Cl | H | n-Butyl | 4-CH₃-C₆H₄ |
| A.1224 | H | Cl | H | n-Butyl | 4-CF₃-C₆H₄ |
| A.1225 | H | Cl | H | n-Butyl | 4-OCH₃-C₆H₄ |
| A.1226 | H | Cl | H | n-Butyl | 4-OCF₃-C₆H₄ |
| A.1227 | H | Cl | H | n-Butyl | 4-Br-C₆H₄ |
| A.1228 | H | Cl | H | n-Butyl | 4-CH=NOCH₃-C₆H₄ |
| A.1229 | H | Cl | H | n-Butyl | 4-CH=NOEt-C₆H₄ |
| A.1230 | H | Cl | H | n-Butyl | 4-[C(CH₃)=NOCH₃]-C₆H₄ |
| A.1231 | H | Cl | H | n-Butyl | 4-[C(CH₃)=NOEt]-C₆H₄ |
| A.1232 | H | Cl | H | n-Butyl | 2,4-F₂-C₆H₃ |
| A.1233 | H | Cl | H | n-Butyl | 2,4-Cl₂-C₆H₃ |
| A.1234 | H | Cl | H | n-Butyl | 2,4-(CH₃)₂-C₆H₃ |
| A.1235 | H | Cl | H | n-Butyl | 3,5-F₂-C₆H₃ |
| A.1236 | H | Cl | H | n-Butyl | 3,5-Cl₂-C₆H₃ |
| A.1237 | H | Cl | H | n-Butyl | 3,5-(CH₃)₂-C₆H₃ |
| A.1238 | H | Cl | H | n-Butyl | 2-F-4-Cl-C₆H₃ |
| A.1239 | H | Cl | H | n-Butyl | 2-Cl-4-F-C₆H₃ |
| A.1240 | H | Cl | H | n-Butyl | 3,4-F₂-C₆H₃ |
| A.1241 | H | Cl | H | n-Butyl | 3,4-Cl₂-C₆H₃ |
| A.1242 | H | Cl | H | n-Butyl | 2,3-F₂-C₆H₃ |
| A.1243 | H | Cl | H | iso-Propyl | CH₃ |
| A.1244 | H | Cl | H | iso-Propyl | Ethyl |
| A.1245 | H | Cl | H | iso-Propyl | n-Propyl |
| A.1246 | H | Cl | H | iso-Propyl | iso-Propyl |
| A.1247 | H | Cl | H | iso-Propyl | tert. Butyl |
| A.1248 | H | Cl | H | iso-Propyl | n-Butyl |
| A.1249 | H | Cl | H | iso-Propyl | C₆H₅ |
| A.1250 | H | Cl | H | iso-Propyl | 2-F-C₆H₄ |
| A.1251 | H | Cl | H | iso-Propyl | 2-Cl-C₆H₄ |
| A.1252 | H | Cl | H | iso-Propyl | 2-CH₃-C₆H₄ |
| A.1253 | H | Cl | H | iso-Propyl | 2-CF₃-C₆H₄ |
| A.1254 | H | Cl | H | iso-Propyl | 3-F-C₆H₄ |
| A.1255 | H | Cl | H | iso-Propyl | 3-Cl-C₆H₄ |
| A.1256 | H | Cl | H | iso-Propyl | 3-CH₃-C₆H₄ |
| A.1257 | H | Cl | H | iso-Propyl | 3-CF₃-C₆H₄ |
| A.1258 | H | Cl | H | iso-Propyl | 3-Br-C₆H₄ |
| A.1259 | H | Cl | H | iso-Propyl | 4-F-C₆H₄ |
| A.1260 | H | Cl | H | iso-Propyl | 4-Cl-C₆H₄ |
| A.1261 | H | Cl | H | iso-Propyl | 4-CH₃-C₆H₄ |
| A.1262 | H | Cl | H | iso-Propyl | 4-CF₃-C₆H₄ |
| A.1263 | H | Cl | H | iso-Propyl | 4-OCH₃-C₆H₄ |
| A.1264 | H | Cl | H | iso-Propyl | 4-OCF₃-C₆H₄ |
| A.1265 | H | Cl | H | iso-Propyl | 4-Br-C₆H₄ |
| A.1266 | H | Cl | H | iso-Propyl | 4-CH=NOCH₃-C₆H₄ |
| A.1267 | H | Cl | H | iso-Propyl | 4-CH=NOEt-C₆H₄ |
| A.1268 | H | Cl | H | iso-Propyl | 4-[C(CH₃)=NOCH₃]-C₆H₄ |
| A.1269 | H | Cl | H | iso-Propyl | 4-[C(CH₃)=NOEt]-C₆H₄ |
| A.1270 | H | Cl | H | iso-Propyl | 2,4-F₂-C₆H₃ |
| A.1271 | H | Cl | H | iso-Propyl | 2,4-Cl₂-C₆H₃ |
| A.1272 | H | Cl | H | iso-Propyl | 2,4-(CH₃)₂-C₆H₃ |
| A.1273 | H | Cl | H | iso-Propyl | 3,5-F₂-C₆H₃ |
| A.1274 | H | Cl | H | iso-Propyl | 3,5-Cl₂-C₆H₃ |
| A.1275 | H | Cl | H | iso-Propyl | 3,5-(CH₃)₂-C₆H₃ |
| A.1276 | H | Cl | H | iso-Propyl | 2-F-4-Cl-C₆H₃ |
| A.1277 | H | Cl | H | iso-Propyl | 2-Cl-4-F-C₆H₃ |
| A.1278 | H | Cl | H | iso-Propyl | 3,4-F₂-C₆H₃ |
| A.1279 | H | Cl | H | iso-Propyl | 3,4-Cl₂-C₆H₃ |
| A.1280 | H | Cl | H | iso-Propyl | 2,3-F₂-C₆H₃ |
| A.1281 | H | Cl | H | iso-Propyl | CH₃ |
| A.1282 | H | Cl | H | iso-Propyl | Ethyl |
| A.1283 | H | Cl | H | iso-Propyl | n-Propyl |
| A.1284 | H | Cl | H | iso-Propyl | iso-Propyl |
| A.1285 | H | Cl | H | iso-Propyl | tert. Butyl |
| A.1286 | H | Cl | H | iso-Propyl | n-Butyl |
| A.1287 | H | Cl | H | iso-Propyl | C₆H₅ |
| A.1288 | H | Cl | H | iso-Propyl | 2-F-C₆H₄ |
| A.1289 | H | Cl | H | iso-Propyl | 2-Cl-C₆H₄ |
| A.1290 | H | Cl | H | iso-Propyl | 2-CH₃-C₆H₄ |
| A.1291 | H | Cl | H | iso-Propyl | 2-CF₃-C₆H₄ |
| A.1292 | H | Cl | H | iso-Propyl | 3-F-C₆H₄ |
| A.1293 | H | Cl | H | iso-Propyl | 3-Cl-C₆H₄ |
| A.1294 | H | Cl | H | iso-Propyl | 3-CH₃-C₆H₄ |
| A.1295 | H | Cl | H | iso-Propyl | 3-CF₃-C₆H₄ |
| A.1296 | H | Cl | H | iso-Propyl | 3-Br-C₆H₄ |
| A.1297 | H | Cl | H | iso-Propyl | 4-F-C₆H₄ |
| A.1298 | H | Cl | H | iso-Propyl | 4-Cl-C₆H₄ |
| A.1299 | H | Cl | H | iso-Propyl | 4-CH₃-C₆H₄ |
| A.1300 | H | Cl | H | iso-Propyl | 4-CF₃-C₆H₄ |
| A.1301 | H | Cl | H | iso-Propyl | 4-OCH₃-C₆H₄ |
| A.1302 | H | Cl | H | iso-Propyl | 4-OCF₃-C₆H₄ |
| A.1303 | H | Cl | H | iso-Propyl | 4-Br-C₆H₄ |
| A.1304 | H | Cl | H | iso-Propyl | 4-CH=NOCH₃-C₆H₄ |
| A.1305 | H | Cl | H | iso-Propyl | 4-CH=NOEt-C₆H₄ |
| A.1306 | H | Cl | H | iso-Propyl | 4-[C(CH₃)=NOCH₃]-C₆H₄ |
| A.1307 | H | Cl | H | iso-Propyl | 4-[C(CH₃)=NOEt]-C₆H₄ |
| A.1308 | H | Cl | H | iso-Propyl | 2,4-F₂-C₆H₃ |
| A.1309 | H | Cl | H | iso-Propyl | 2,4-Cl₂-C₆H₃ |
| A.1310 | H | Cl | H | iso-Propyl | 2,4-(CH₃)₂-C₆H₃ |
| A.1311 | H | Cl | H | iso-Propyl | 3,5-F₂-C₆H3 |
| A.1312 | H | Cl | H | iso-Propyl | 3,5-Cl₂-C₆H₃ |
| A.1313 | H | Cl | *H* | iso-Propyl | 3,5-(CH₃)₂-C₆H₃ |
| A.1314 | H | Cl | H | iso-Propyl | 2-F-4-Cl-C₆H₃ |
| A.1315 | H | Cl | H | iso-Propyl | 2-Cl-4-F-C₆H₃ |
| A.1316 | H | Cl | H | iso-Propyl | 3,4-F₂-C₆H₃ |
| A.1317 | H | Cl | H | iso-Propyl | 3,4-Cl₂-C₆H₃ |
| A.1318 | H | Cl | H | iso-Propyl | 2,3-F₂-C₆H₃ |
| A.1319 | H | Cl | H | tert. Butyl | CH₃ |
| A.1320 | H | Cl | H | tert. Butyl | Ethyl |
| A.1321 | H | Cl | H | tert. Butyl | n-Propyl |
| A.1322 | H | Cl | H | tert. Butyl | iso-Propyl |
| A.1323 | H | Cl | H | tert. Butyl | tert. Butyl |
| A.1324 | H | Cl | H | tert. Butyl | n-Butyl |
| A.1325 | H | Cl | H | tert. Butyl | C₆H₅ |
| A.1326 | H | Cl | H | tert. Butyl | 2-F-C₆H₄ |
| A.1327 | H | Cl | H | tert. Butyl | 2-Cl-C₆H₄ |
| A.1328 | H | Cl | H | tert. Butyl. | 2-CH₃-C₆H₄ |
| A.1329 | H | Cl | H | tert. Butyl | 2-CF₃-C₆H₄ |
| A.1330 | H | Cl | H | tert. Butyl | 3-F-C₆H₄ |
| A.1331 | H | Cl | H | tert. Butyl | 3-Cl-C₆H₄ |
| A.1332 | H | Cl | H | tert. Butyl | 3-CH₃-C₆H₄ |
| A.1333 | H | Cl | H | tert. Butyl | 3-CF₃-C₆H₄ |
| A.1334 | H | Cl | H | tert. Butyl | 3-Br-C₆H₄ |
| A.1335 | H | Cl | H | tert. Butyl | 4-F-C₆H₄ |
| A.1336 | H | Cl | H | tert. Butyl | 4-Cl-C₆H₄ |
| A.1337 | H | Cl | H | tert. Butyl | 4-CH₃-C₆H₄ |
| A.1338 | H | Cl | H | tert. Butyl | 4-CF₃-C₆H₄ |
| A.1339 | H | Cl | H | tert. Butyl | 4-OCH₃-C₆H₄ |
| A.1340 | H | Cl | H | tert. Butyl | 4-OCF₃-C₆H₄ |
| A.1341 | H | Cl | H | tert. Butyl | 4-Br-C₆H₄ |
| A.1342 | H | Cl | H | tert. Butyl | 4-CH=NOCH₃-C₆H₄ |
| A.1343 | H | Cl | H | tert. Butyl | 4-CH=NOEt-C₆H₄ |
| A.1344 | H | Cl | H | tert. Butyl | 4-[C(CH₃)=NOCH₃]-C₆H₄ |
| A.1345 | H | Cl | H | tert. Butyl | 4-[C(CH₃₎=NOEt]-C₆H₄ |
| A.1346 | H | Cl | H | tert. Butyl | 2,4-F₂-C₆H₃ |
| A.1347 | H | Cl | H | tert. Butyl | 2,4-Cl₂-C₆H₃ |
| A.1348 | H | Cl | H | tert. Butyl | 2,4-(CH₃)₂-C₆H₃ |
| A.1349 | H | Cl | H | tert. Butyl | 3,5-F₂-C₆H₃ |
| A.1350 | H | Cl | H | tert. Butyl | 3,5-Cl₂-C₆H₃ |
| A.1351 | H | Cl | H | tert. Butyl | 3,5-(CH₃)₂-C₆H₃ |
| A.1352 | H | Cl | H | tert. Butyl | 2-F-4-Cl-C₆H₃ |
| A.1353 | H | Cl | H | tert. Butyl | 2-Cl-4-F-C₆H₃ |
| A.1354 | H | Cl | H | tert. Butyl | 3,4-F₂-C₆H₃ |
| A.1355 | H | Cl | H | tert. Butyl | 3,4-Cl₂-C₆H₃ |
| A.1356 | H | Cl | H | tert. Butyl | 2,3-F₂-C₆H₃ |
| A.1357 | H | Cl | H | tert. Butyl | CH₃ |
| A.1358 | H | Cl | H | tert. Butyl | Ethyl |
| A.1359 | H | Cl | H | tert. Butyl | n-Propyl |
| A.1360 | H | Cl | H | tert. Butyl | iso-Propyl |
| A.1361 | H | Cl | H | tert. Butyl | tert. Butyl |
| A.1362 | H | Cl | H | tert. Butyl | n-Butyl |
| A.1363 | H | Cl | H | tert. Butyl | C₆H₅ |
| A.1364 | H | Cl | H | tert. Butyl | 2-F-C₆H₄ |
| A.1365 | H | Cl | H | tert. Butyl | 2-Cl-C₆H₄ |
| A.1366 | H | Cl | H | tert. Butyl | 2-CH₃-C₆H₄ |
| A.1367 | H | Cl | H | tert. Butyl | 2-CF₃-C₆H₄ |
| A.1368 | H | Cl | H | tert. Butyl | 3-F-C₆H₄ |
| A.1369 | H | Cl | H | tert. Butyl | 3-Cl-C₆H₄ |
| A.1370 | H | Cl | H | tert. Butyl | 3-CH₃-C₆H₄ |
| A.1371 | H | Cl | H | tert. Butyl | 3-CF₃-C₆H₄ |
| A.1372 | H | Cl | H | tert. Butyl | 3-Br-C₆H₄ |
| A.1373 | H | Cl | H | tert. Butyl | 4-F-C₆H₄ |
| A.1374 | H | Cl | H | tert. Butyl | 4-Cl-C₆H₄ |
| A.1375 | H | Cl | H | tert. Butyl | 4-CH₃-C₆H₄ |
| A.1376 | H | Cl | H | tert. Butyl | 4-CF₃-C₆H₄ |
| A.1377 | H | Cl | H | tert. Butyl | 4-OCH₃-C₆H₄ |
| A.1378 | H | Cl | H | tert. Butyl | 4-OCF₃-C₆H₄ |
| A.1379 | H | Cl | H | tert. Butyl | 4-Br-C₆H₄ |
| A.1380 | H | Cl | H | tert. Butyl | 4-CH=NOCH₃-C₆H₄ |
| A.1381 | H | Cl | H | tert. Butyl | 4-CH=NOEt-C₆R₄ |
| A.1382 | H | Cl | H | tert. Butyl | 4-[C(CH₃)=NOCH₃]-C₆H₄ |
| A.1383 | H | Cl | H | tert. Butyl | 4-[C(CH₃)=NOEt]-C₆H₄ |
| A.1384 | H | Cl | H | tert. Butyl | 2,4-F₂-C₆H₃ |
| A.1385 | H | Cl | H | tert. Butyl | 2,4-Cl₂-C₆H₃ |
| A.1386 | H | Cl | H | tert. Butyl | 2,4-(CH₃)₂-C₆H₃ |
| A.1387 | H | Cl | H | tert. Butyl | 3,5-F₂-C₆H₃ |
| A.1388 | H | Cl | H | tert. Butyl | 3,5-Cl₂-C₆H₃ |
| A.1389 | H | Cl | H | tert. Butyl | 3,5-(CH₃)₂-C₆H₃ |
| A.1390 | H | Cl | H | tert. Butyl | 2-F-4-Cl-C₆H₃ |
| A.1391 | H | Cl | H | tert. Butyl | 2-Cl-4-F-C₆H₃ |
| A.1392 | H | Cl | H | tert. Butyl | 3,4-F₂-C₆H₃ |
| A.1393 | H | Cl | H | tert. Butyl | 3,4-Cl₂-C₆H₃ |
| A.1394 | H | Cl | H | tert. Butyl | 2,3-F₂-C₆H₃ |
| A.1395 | H | OCH₃ | H | H | CH₃ |
| A.1396 | H | OCH₃ | H | H | Ethyl |
| A.1397 | H | OCH₃ | H | H | n-Propyl |
| A.1398 | H | OCH₃ | H | H | iso-Propyl |
| A.1399 | H | OCH₃ | H | H | tert. Butyl |
| A.1400 | H | OCH₃ | H | H | n-Butyl |
| A.1401 | H | OCH₃ | H | H | C₆H₅ |
| A.1402 | H | OCH₃ | H | H | 2-F-C₆H₄ |
| A.1403 | H | OCH₃ | H | H | 2-Cl-C₆H₄ |
| A.1404 | H | OCH₃ | H | H | 2-CH₃-C₆H₄ |
| A.1405 | H | OCH3 | H | H | 2-CF₃-C₆H₄ |
| A.1406 | H | OCH₃ | H | H | 3-F-C₆H₄ |
| A.1407 | H | OCH₃ | H | H | 3-Cl-C₆H₄ |
| A.1408 | H | OCH₃ | H | H | 3-CH₃-C₆H₄ |
| A.1409 | H | OCH₃ | H | H | 3-CF₃-C₆H₄ |
| A.1410 | H | OCH₃ | H | H | 3-Br-C₆H₄ |
| A.1411 | H | OCH₃ | H | H | 4-F-C₆H₄ |
| A.1412 | H | OCH₃ | H | H | 4-Cl-C₆H₄ |
| A.1413 | H | OCH₃ | H | H | 4-CH₃-C₆H₄ |
| A.1414 | H | OCH₃ | H | H | 4-CF₃-C₆H₄ |
| A.1415 | H | OCH₃ | H | H | 4-OCH₃-C₆H₄ |
| A.1416 | H | OCH₃ | H | H | 4-OCF₃-C₆H₄ |
| A.1417 | H | OCH₃ | H | H | 4-Br-C₆H₄ |
| A.1418 | H | OCH₃ | H | H | 4-(CH=NOCH₃)-C₆H₄ |
| A.1419 | H | OCH₃ | H | H | 4-(CH=NOEt)-C₆H₄ |
| A.1420 | H | OCH₃ | H | H | 4-[C(CH₃)=NOCH₃]-C₆H₄ |
| A.1421 | H | OCH₃ | H | H | 4-[C(CH₃)=NOEt]-C₆H₄ |
| A.1422 | H | OCH₃ | H | H | 2,4-F₂-C₆H₃ |
| A.1423 | H | OCH₃ | H | H | 2,4-Cl₂-C₆H₃ |
| A.1424 | H | OCH₃ | H | H | 2,4-(CH₃)₂-C₆H₃ |
| A.1425 | H | OCH₃ | H | H | 3,5-F₂-C₆H₃ |
| A.1426 | H | OCH₃ | H | H | 3,5-Cl₂-C₆H₃ |
| A.1427 | H | OCH₃ | H | H | 3,5-(CH₃)₂-C₆H₃ |
| A.1428 | H | OCH₃ | H | H | 2-F-4-Cl-C₆H₃ |
| A.1429 | H | OCH₃ | H | H | 2-Cl-4-F-C₆H₃ |
| A.1430 | H | OCH₃ | H | H | 3,4-F₂-C₆H₃ |
| A.1431 | H | OCH₃ | H | H | 3,4-Cl₂-C₆H₃ |
| A.1432 | H | OCH₃ | H | H | 2,3-F₂-C₆H₃ |
| A.1433 | H | OCH₃ | H | CH₃ | CH₃ |
| A.1434 | H | OCH₃ | H | CH₃ | Ethyl |
| A.1435 | H | OCH₃ | H | CH₃ | n-Propyl |
| A.1436 | H | OCH₃ | H | CH₃ | iso-Propyl |
| A.1437 | H | OCH₃ | H | CH₃ | tert. Butyl |
| A.1438 | H | OCH₃ | H | CH₃ | n-Butyl |
| A.1439 | H | OCH₃ | H | CH₃ | C₆H₅ |
| A.1440 | H | OCH₃ | H | CH₃ | 2-F-C₆H₄ |
| A.1441 | H | OCH₃ | H | CH₃ | 2-Cl-C₆H₄ |
| A.1442 | H | OCH₃ | H | CH₃ | 2-CH₃-C₆H₄ |
| A.1443 | H | OCH₃ | H | CH₃ | 2-CF₃-C₆H₄ |
| A.1444 | H | OCH₃ | H | CH₃ | 3-F-C₆H₄ |
| A.1445 | H | OCH₃ | H | CH₃ | 3-Cl-C₆H₄ |
| A.1446 | H | OCH₃ | H | CH₃ | 3-CH₃-C₆H₄ |
| A.1447 | H | OCH₃ | H | CH₃ | 3-CF₃-C₆H₄ |
| A.1448 | H | OCH₃ | H | CH₃ | 3-Br-C₆H₄ |
| A.1449 | H | OCH₃ | H | CH₃ | 4-F-C₆H₄ |
| A.1450 | H | OCH₃ | H | CH₃ | 4-Cl-C₆H₄ |
| A.1451 | H | OCH₃ | H | CH₃ | 4-CH₃-C₆H₄ |
| A.1452 | H | OCH₃ | H | CH₃ | 4-CF₃-C₆H₄ |
| A.1453 | H | OCH₃ | H | CH₃ | 4-OCH₃-C₆H₄ |
| A.1454 | H | OCH₃ | H | CH₃ | 4-OCF₃-C₆H₄ |
| A.1455 | H | OCH₃ | H | CH₃ | 4-Br-C₆H₄ |
| A.1456 | H | OCH₃ | H | CH₃ | 4-(CH=NOCH₃)-C₆H₄ |
| A.1457 | H | OCH₃ | H | CH₃ | 4-(CH=NOEt)-C₆H₄ |
| A.1458 | H | OCH₃ | H | CH₃ | 4-[C(CH₃)=NOCH₃]-C₆H₄ |
| A.1459 | H | OCH₃ | H | CH₃ | 4-[C(CH₃)=NOEt]-C₆H₄ |
| A.1460 | H | OCH₃ | H | CH₃ | 2,4-F₂-C₆H₃ |
| A.1461 | H | OCH₃ | H | CH₃ | 2,4-Cl₂-C₆H₃ |
| A.1462 | H | OCH₃ | H | CH₃ | 2,4-(CH₃)₂-C₆H₃ |
| A.1463 | H | OCH₃ | H | CH₃ | 3,5-F₂-C₆H₃ |
| A.1464 | H | OCH₃ | H | CH₃ | 3,5-Cl₂-C₆H₃ |
| A.1465 | H | OCH₃ | H | CH₃ | 3,5-(CH₃)₂-C₆H₃ |
| A.1466 | H | OCH₃ | H | CH₃ | 2-F-4-Cl-C₆H₃ |
| A.1467 | H | OCH₃ | H | CH₃ | 2-Cl-4-F-C₆H₃ |
| A.1468 | H | OCH₃ | H | CH₃ | 3,4-F₂-C₆H₃ |
| A.1469 | H | OCH₃ | H | CH₃ | 3,4-Cl₂-C₆H₃ |
| A.1470 | H | OCH₃ | H | CH₃ | 2,3-F₂-C₆H₃ |
| A.1471 | H | OCH₃ | H | Ethyl | CH₃ |
| A.1472 | H | OCH₃ | H | Ethyl | Ethyl |
| A.1473 | H | OCH₃ | H | Ethyl | n-Propyl |
| A.1474 | H | OCH₃ | H | Ethyl | iso-Propyl |
| A.1475 | H | OCH₃ | H | Ethyl | tert. Butyl |
| A.1476 | H | OCH₃ | H | Ethyl | n-Butyl |
| A.1477 | H | OCH₃ | H | Ethyl | C₆H₅ |
| A.1478 | H | OCH₃ | H | Ethyl | 2-F-C₆H₄ |
| A.1479 | H | OCH₃ | H | Ethyl | 2-Cl-C₆H₄ |
| A.1480 | H | OCH₃ | H | Ethyl | 2-CH₃-C₆H₄ |
| A.1481 | H | OCH₃ | H | Ethyl | 2-CF₃-C₆H₄ |
| A.1482 | H | OCH₃ | H | Ethyl | 3-F-C₆H₄ |
| A.1483 | H | OCH₃ | H | Ethyl | 3-Cl-C₆H₄ |
| A.1484 | H | OCH₃ | H | Ethyl | 3-CH₃-C₆H₄ |
| A.1485 | H | OCH₃ | H | Ethyl | 3-CF₃-C₆H₄ |
| A.1486 | H | OCH₃ | H | Ethyl | 3-Br-C₆H₄ |
| A.1487 | H | OCH₃ | H | Ethyl | 4-F-C₆H₄ |
| A.1488 | H | OCH₃ | H | Ethyl | 4-Cl-C₆H₄ |
| A.1489 | H | OCH₃ | H | Ethyl | 4-CH₃-C₆H₄ |
| A.1490 | H | OCH₃ | H | Ethyl | 4-CF₃-C₆H₄ |
| A.1491 | H | OCH₃ | H | Ethyl | 4-OCH₃-C₆H₄ |
| A.1492 | H | OCH₃ | H | Ethyl | 4-OCF₃-C₆R₄ |
| A.1493 | H | OCH₃ | H | Ethyl | 4-Br-C₆H₄ |
| A.1494 | H | OCH₃ | H | Ethyl | 4-(CH=NOCH₃)-C₆H₄ |
| A.1495 | H | OCH₃ | H | Ethyl | 4-(CH=NOEt)-C₆H₄ |
| A.1496 | H | OCH₃ | H | Ethyl | 4-[C(CH₃)=NOCH₃]-C₆H₄ |
| A.1497 | H | OCH₃ | H | Ethyl | 4-[C(CH₃)=NOEt]-C₆H₄ |
| A.1498 | H | OCH₃ | H | Ethyl | 2,4-F₂-C₆H₃ |
| A.1499 | H | OCH₃ | H | Ethyl | 2,4-Cl₂-C₆H₃ |
| A.1500 | H | OCH₃ | H | Ethyl | 2,4-(CH₃)₂-C₆H₃ |
| A.1501 | H | OCH₃ | H | Ethyl | 3,5-F₂-C₆H₃ |
| A.1502 | H | OCH₃ | H | Ethyl | 3,5-Cl₂-C₆H₃ |
| A.1503 | H | OCH₃ | H | Ethyl | 3,5-(CH₃)₂-C₆H₃ |
| A.1504 | H | OCH₃ | H | Ethyl | 2-F-4-Cl-C₆H₃ |
| A.1505 | H | OCH₃ | H | Ethyl | 2-Cl-4-F-C₆H₃ |
| A.1506 | H | OCH₃ | H | Ethyl | 3,4-F₂-C₆H₃ |
| A.1507 | H | OCH₃ | H | Ethyl | 3,4-Cl₂-C₆H₃ |
| A.1508 | H | OCH₃ | H | Ethyl | 2,3-F₂-C₆H₃ |
| A.1509 | H | OCH₃ | H | n-Propyl | CH₃ |
| A.1510 | H | OCH₃ | H | n-Propyl | Ethyl |
| A.1511 | H | OCH₃ | H | n-Propyl | n-Propyl |
| A.1512 | H | OCH₃ | H | n-Propyl | iso-Propyl |
| A.1513 | H | OCH₃ | H | n-Propyl | tert. Butyl |
| A.1514 | H | OCH₃ | H | n-Propyl | n-Butyl |
| A.1515 | H | OCH₃ | H | n-Propyl | C₆H₅ |
| A.1516 | H | OCH₃ | H | n-Propyl | 2-F-C₆H₄ |
| A.1517 | H | OCH₃ | H | n-Propyl | 2-Cl-C₆H₄ |
| A.1518 | H | OCH₃ | H | n-Propyl | 2-CH₃-C₆H₄ |
| A.1519 | H | OCH₃ | H | n-Propyl | 2-CF₃-C₆H₄ |
| A.1520 | H | OCH₃ | H | n-Propyl | 3-F-C₆H₄ |
| A.1521 | H | OCH₃ | H | n-Propyl | 3-Cl-C₆H₄ |
| A.1522 | H | OCH₃ | H | n-Propyl | 3-CH₃-C₆H₄ |
| A.1523 | H | OCH₃ | H | n-Propyl | 3-CF₃-C₆H₄ |
| A.1524 | H | OCH₃ | H | n-Propyl | 3-Br-C₆H₄ |
| A.1525 | H | OCH₃ | H | n-Propyl | 4-F-C₆H₄ |
| A.1526 | H | OCH₃ | H | n-Propyl | 4-Cl-C₆H₄ |
| A.1527 | H | OCH₃ | H | n-Propyl | 4-CH₃-C₆H₄ |
| A.1528 | H | OCH₃ | H | n-Propyl | 4-CF₃-C₆H₄ |
| A.1529 | H | OCH₃ | H | n-Propyl | 4-OCH₃-C₆H₄ |
| A.1530 | H | OCH₃ | H | n-Propyl | 4-OCF₃-C₆H₄ |
| A.1531 | H | OCH₃ | H | n-Propyl | 4-Br-C₆H₄ |
| A.1532 | H | OCH₃ | H | n-Propyl | 4-(CH=NOCH₃)-C₆H₄ |
| A.1533 | H | OCH₃ | H | n-Propyl | 4-(CH=NOEt)-C₆H₄ |
| A.1534 | H | OCH₃ | H | n-Propyl | 4-[C(CH₃)=NOCH₃]-C₆H₄ |
| A.1535 | H | OCH₃ | H | n-Propyl | 4-[C(CH₃)=NOEt]-C₆H₄ |
| A.1536 | H | OCH₃ | H | n-Propyl | 2,4-F₂-C₆H₃ |
| A.1537 | H | OCH₃ | H | n-Propyl | 2,4-Cl₂-C₆H₃ |
| A.1538 | H | OCH₃ | H | n-Propyl | 2,4-(CH₃)₂-C₆H₃ |
| A.1539 | H | OCH₃ | H | n-Propyl | 3,5-F₂-C₆H₃ |
| A.1540 | H | OCH₃ | H | n-Propyl | 3,5-Cl₂-C₆H₃ |
| A.1541 | H | OCH₃ | H | n-Propyl | 3,5-(CH₃)₂-C₆H₃ |
| A.1542 | H | OCH₃ | H | n-Propyl | 2-F-4-Cl-C₆H₃ |
| A.1543 | H | OCH₃ | H | n-Propyl | 2-Cl-4-F-C₆H₃ |
| A.1544 | H | OCH₃ | H | n-Propyl | 3,4-F₂-C₆H₃ |
| A.1545 | H | OCH₃ | H | n-Propyl | 3,4-Cl₂-C₆H₃ |
| A.1546 | H | OCH₃ | H | n-Propyl | 2,3-F₂-C₆H₃ |
| A.1547 | H | OCH₃ | H | n-Propyl | CH₃ |
| A.1548 | H | OCH₃ | H | n-Propyl | Ethyl |
| A.1549 | H | OCH₃ | H | n-Propyl | n-Propyl |
| A.1550 | H | OCH₃ | H | n-Propyl | iso-Propyl |
| A.1551 | H | OCH₃ | H | n-Propyl | tert. Butyl |
| A.1552 | H | OCH₃ | H | n-Propyl | n-Butyl |
| A.1553 | H | OCH₃ | H | n-Propyl | C₆H₅ |
| A.1554 | H | OCH₃ | H | n-Propyl | 2-F-C₆H₄ |
| A.1555 | H | OCH₃ | H | n-Propyl | 2-Cl-C₆H₄ |
| A.1556 | H | OCH₃ | H | n-Propyl | 2-CH₃-C₆H₄ |
| A.1557 | H | OCH₃ | H | n-Propyl | 2-CF₃-C₆H₄ |
| A.1558 | H | OCH₃ | H | n-Propyl | 3-F-C₆H₄ |
| A.1559 | H | OCH₃ | H | n-Propyl | 3-Cl-C₆H₄ |
| A.1560 | H | OCH₃ | H | n-Propyl | 3-CH₃-C₆H₄ |
| A.1561 | H | OCH₃ | H | n-Propyl | 3-CF₃-C₆H₄ |
| A.1562 | H | OCH₃ | H | n-Propyl | 3-Br-C₆H₄ |
| A.1563 | H | OCH₃ | H | n-Propyl | 4-F-C₆H₄ |
| A.1564 | H | OCH₃ | H | n-Propyl | 4-Cl-C₆H₄ |
| A.1565 | H | OCH₃ | H | n-Propyl | 4-CH₃-C₆H₄ |
| A.1566 | H | OCH₃ | H | n-Propyl | 4-CF₃-C₆H₄ |
| A.1567 | H | OCH₃ | H | n-Propyl | 4-OCH₃-C₆H₄ |
| A.1568 | H | OCH₃ | H | n-Propyl | 4-OCF₃-C₆H₄ |
| A.1569 | H | OCH₃ | H | n-Propyl | 4-Br-C₆H₄ |
| A.1570 | H | OCH₃ | H | n-Propyl | 4-(CH=NOCH₃)-C₆H₄ |
| A.1571 | H | OCH₃ | H | n-Propyl | 4-(CH=NOEt)-C₆H₄ |
| A.1572 | H | OCH₃ | H | n-Propyl | 4-[C(CH₃)=NOCH₃]-C₆H₄ |
| A.1573 | H | OCH₃ | H | n-Propyl | 4-[C(CH₃)=NOEt]-C₆H₄ |
| A.1574 | H | OCH₃ | H | n-Propyl | 2,4-F₂-C₆H₃ |
| A.1575 | H | OCH₃ | H | n-Propyl | 2,4-Cl₂-C₆H₃ |
| A.1576 | H | OCH₃ | H | n-Propyl | 2,4-(CH₃)₂-C₆H₃ |
| A.1577 | H | OCH₃ | H | n-Propyl | 3,5-F₂-C₆H₃ |
| A.1578 | H | OCH₃ | H | n-Propyl | 3,5-Cl₂-C₆H₃ |
| A.1579 | H | OCH₃ | H | n-Propyl | 3,5-(CH₃)₂-C₆H₃ |
| A.1580 | H | OCH₃ | H | n-Propyl | 2-F-4-Cl-C₆H₃ |
| A.1581 | H | OCH₃ | H | n-Propyl | 2-Cl-4-F-C₆H₃ |
| A.1582 | H | OCH₃ | H | n-Propyl | 3,4-F₂-C₆H₃ |
| A.1583 | H | OCH₃ | H | n-Propyl | 3,4-Cl₂-C₆H₃ |
| A.1584 | H | OCH₃ | H | n-Propyl | 2,3-F₂-C₆H₃ |
| A.1585 | H | OCH₃ | H | n-Butyl | CH₃ |
| A.1586 | H | OCH₃ | H | n-Butyl | Ethyl |
| A.1587 | H | OCH₃ | H | n-Butyl | n-Propyl |
| A.1588 | H | OCH₃ | H | n-Butyl | iso-Propyl |
| A.1589 | H | OCH₃ | H | n-Butyl | tert. Butyl |
| A.1590 | H | OCH₃ | H | n-Butyl | n-Butyl |
| A.1591 | H | OCH₃ | H | n-Butyl | C₆H₅ |
| A.1592 | H | OCH₃ | H | n-Butyl | 2-F-C₆H₄ |
| A.1593 | H | OCH₃ | H | n-Butyl | 2-Cl-C₆H₄ |
| A.1594 | H | OCH₃ | H | n-Butyl | 2-CH₃-C₆H₄ |
| A.1595 | H | OCH₃ | H | n-Butyl | 2-CF₃-C₆H₄ |
| A.1596 | H | OCH3 | H | n-Butyl | 3-F-C₆H₄ |
| A.1597 | H | OCH₃ | H | n-Butyl | 3-Cl-C₆H₄ |
| A.1598 | H | OCH₃ | H | n-Butyl | 3-CH₃-C₆H₄ |
| A.1599 | H | OCH₃ | H | n-Butyl | 3-CF₃-C₆H₄ |
| A.1600 | H | OCH₃ | H | n-Butyl | 3-Br-C₆H₄ |
| A.1601 | H | OCH₃ | H | n-Butyl | 4-F-C₆H₄ |
| A.1602 | H | OCH₃ | H | n-Butyl | 4-Cl-C₆H₄ |
| A.1603 | H | OCH₃ | H | n-Butyl | 4-CH₃-C₆H₄ |
| A.1604 | H | OCH₃ | H | n-Butyl | 4-CF₃-C₆H₄ |
| A.1605 | H | OCH₃ | H | n-Butyl | 4-OCH₃-C₆H₄ |
| A.1606 | H | OCH₃ | H | n-Butyl | 4-OCF₃-C₆H₄ |
| A.1607 | H | OCH₃ | H | n-Butyl | 4-Br-C₆H₄ |
| A.1608 | H | OCH₃ | H | n-Butyl | 4-(CH=NOCH₃)-C₆H₄ |
| A.1609 | H | OCH₃ | H | n-Butyl | 4-(CH=NOEt)-C₆H₄ |
| A.1610 | H | OCH₃ | H | n-Butyl | 4-[C(CH₃)=NOCH₃]-C₆H₄ |
| A.1611 | H | OCH₃ | H | n-Butyl | 4-[C(CH₃)=NOEt]-C₆H₄ |
| A.1612 | H | OCH₃ | H | n-Butyl | 2,4-F₂-C₆H₃ |
| A.1613 | H | OCH₃ | H | n-Butyl | 2,4-Cl₂-C₆H₃ |
| A.1614 | H | OCH₃ | H | n-Butyl | 2,4-(CH₃)₂-C₆H₃ |
| A.1615 | H | OCH₃ | H | n-Butyl | 3,5-F₂-C₆H₃ |
| A.1616 | H | OCH₃ | H | n-Butyl | 3,5-Cl₂-C₆H₃ |
| A.1617 | H | OCH₃ | H | n-Butyl | 3,5-(CH₃)₂-C₆H₃ |
| A.1618 | H | OCH₃ | H | n-Butyl | 2-F-4-Cl-C₆H₃ |
| A.1619 | H | OCH₃ | H | n-Butyl | 2-Cl-4-F-C₆H₃ |
| A.1620 | H | OCH₃ | H | n-Butyl | 3,4-F₂-C₆H₃ |
| A.1621 | H | OCH₃ | H | n-Butyl | 3,4-Cl₂-C₆H₃ |
| A.1622 | H | OCH₃ | H | n-Butyl | 2,3-F₂-C₆H₃ |
| A.1623 | H | OCH₃ | H | n-Butyl | CH₃ |
| A.1624 | H | OCH₃ | H | n-Butyl | Ethyl |
| A.1625 | H | OCH₃ | H | n-Butyl | n-Propyl |
| A.1626 | H | OCH₃ | H | n-Butyl | iso-Propyl |
| A.1627 | H | OCH₃ | H | n-Butyl | tert. Butyl |
| A.1628 | H | OCH₃ | H | n-Butyl | n-Butyl |
| A.1629 | H | OCH₃ | H | n-Butyl | C₆H₅ |
| A.1630 | H | OCH₃ | H | n-Butyl | 2-F-C₆H₄ |
| A.1631 | H | OCH₃ | H | n-Butyl | 2-Cl-C₆H₄ |
| A.1632 | H | OCH₃ | H | n-Butyl | 2-CH₃-C₆H₄ |
| A.1633 | H | OCH₃ | H | n-Butyl | 2-CF₃-C₆H₄ |
| A.1634 | H | OCH₃ | H | n-Butyl | 3-F-C₆H₄ |
| A.1635 | H | OCH₃ | H | n-Butyl | 3-Cl-C₆H₄ |
| A.1636 | H | OCH₃ | H | n-Butyl | 3-CH₃-C₆H₄ |
| A.1637 | H | OCH₃ | H | n-Butyl | 3-CF₃-C₆H₄ |
| A.1638 | H | OCH₃ | H | n-Butyl | 3-Br-C₆H₄ |
| A.1639 | H | OCH₃ | H | n-Butyl | 4-F-C₆H₄ |
| A.1640 | H | OCH₃ | H | n-Butyl | 4-Cl-C₆H₄ |
| A.1641 | H | OCH₃ | H | n-Butyl | 4-CH₃-C₆H₄ |
| A.1642 | H | OCH₃ | H | n-Butyl | 4-CF₃-C₆H₄ |
| A.1643 | H | OCH₃ | H | n-Butyl | 4-OCH₃-C₆H₄ |
| A.1644 | H | OCH₃ | H | n-Butyl | 4-OCF₃-C₆H₄ |
| A.1645 | H | OCH₃ | H | n-Butyl | 4-Br-C₆H₄ |
| A.1646 | H | OCH₃ | H | n-Butyl | 4-(CH=NOCH₃)-C₆H₄ |
| A.1647 | H. | OCH₃ | H | n-Butyl | 4-(CH=NOEt)-C₆H₄ |
| A.1648 | H | OCH₃ | H | n-Butyl | 4-[C(CH₃)=NOCH₃]-C₆H₄ |
| A.1649 | H | OCH₃ | H | n-Butyl | 4-[C(CH₃)=NOEt]-C₆H₄ |
| A.1650 | H | OCH₃ | H | n-Butyl | 2,4-F₂-C₆H₃ |
| A.1651 | H | OCH₃ | H | n-Butyl | 2,4-Cl₂-C₆H₃ |
| A.1652 | H | OCH₃ | H | n-Butyl | 2,4-(CH₃)₂-C₆H₃ |
| A.1653 | H | OCH₃ | H | n-Butyl | 3,5-F₂-C₆H₃ |
| A.1654 | H | OCH₃ | H | n-Butyl | 3,5-Cl₂-C₆H₃ |
| A.1655 | H | OCH₃ | H | n-Butyl | 3,5-(CH₃)₂-C₆H₃ |
| A.1656 | H | OCH₃ | H | n-Butyl | 2-F-4-Cl-C₆H₃ |
| A.1657 | H | OCH₃ | H | n-Butyl | 2-Cl-4-F-C₆H₃ |
| A.1658 | H | OCH₃ | H | n-Butyl | 3,4-F₂-C₆H₃ |
| A.1659 | H | OCH₃ | H | n-Butyl | 3,4-Cl₂-C₆H₃ |
| A.1660 | H | OCH₃ | H | n-Butyl | 2,3-F₂-C₆H₃ |
| A.1661 | H | OCH₃ | H | iso-Propyl | CH₃ |
| A.1662 | H | OCH₃ | H | iso-Propyl | Ethyl |
| A.1663 | H | OCH₃ | H | iso-Propyl | n-Propyl |
| A.1664 | H | OCH₃ | H | iso-Propyl | iso-Propyl |
| A.1665 | H | OCH₃ | H | iso-Propyl | tert. Butyl |
| A.1666 | H | OCH₃ | H | iso-Propyl | n-Butyl |
| A.1667 | H | OCH₃ | H | iso-Propyl | C₆H₅ |
| A.1668 | H | OCH₃ | H | iso-Propyl | 2-F-C₆H₄ |
| A.1669 | H | OCH₃ | H | iso-Propyl | 2-Cl-C₆H₄ |
| A.1670 | H | OCH₃ | H | iso-Propyl | 2-CH₃-C₆H₄ |
| A.1671 | H | OCH₃ | H | iso-Propyl | 2-CF₃-C₆H₄ |
| A.1672 | H | OCH₃ | H | iso-Propyl | 3-F-C₆H₄ |
| A.1673 | H | OCH₃ | H | iso-Propyl | 3-Cl-C₆H₄ |
| A.1674 | H | OCH₃ | H | iso-Propyl | 3-CH₃-C₆H₄ |
| A.1675 | H | OCH₃ | H | iso-Propyl | 3-CF₃-C₆H₄ |
| A.1676 | H | OCH₃ | H | iso-Propyl | 3-Br-C₆H₄ |
| A.1677 | H | OCH₃ | H | iso-Propyl | 4-F-C₆H₄ |
| A.1678 | H | OCH₃ | H | iso-Propyl | 4-Cl-C₆H₄ |
| A.1679 | H | OCH₃ | H | iso-Propyl | 4-CH₃-C₆H₄ |
| A.1680 | H | OCH₃ | H | iso-Propyl | 4-CF₃-C₆H₄ |
| A.1681 | H | OCH₃ | H | iso-Propyl | 4-OCH₃-C₆H₄ |
| A.1682 | H | OCH₃ | H | iso-Propyl | 4-OCF₃-C₆H₄ |
| A.1683 | H | OCH₃ | H | iso-Propyl | 4-Br-C₆H₄ |
| A.1684 | H | OCH₃ | H | iso-Propyl | 4-(CH=NOCH₃)-C₆H₄ |
| A.1685 | H | OCH₃ | H | iso-Propyl | 4-(CH=NOEt)-C₆H₄ |
| A.1686 | H | OCH₃ | H | iso-Propyl | 4-[C(CH₃)=NOCH₃]-C₆H₄ |
| A.1687 | H | OCH₃ | H | iso-Propyl | 4-[C(CH₃)=NOEt]-C₆H₄ |
| A.1688 | H | OCH₃ | H | iso-Propyl | 2,4-F₂-C₆H₃ |
| A.1689 | H | OCH₃ | H | iso-Propyl | 2,4-Cl₂-C₆H₃ |
| A.1690 | H | OCH₃ | H | iso-Propyl | 2,4-(CH₃)₂-C₆H₃ |
| A.1691 | H | OCH₃ | H | iso-Propyl | 3,5-F₂-C₆H₃ |
| A.1692 | H | OCH₃ | H | iso-Propyl | 3,5-Cl₂-C₆H₃ |
| A.1693 | H | OCH₃ | H | iso-Propyl | 3,5-(CH₃)₂-C₆H₃ |
| A.1694 | H | OCH₃ | H | iso-Propyl | 2-F-4-Cl-C₆H₃ |
| A.1695 | H | OCH₃ | H | iso-Propyl | 2-Cl-4-F-C₆H₃ |
| A.1696 | H | OCH₃ | H | iso-Propyl | 3,4-F₂-C₆H₃ |
| A.1697 | H | OCH₃ | H | iso-Propyl | 3,4-Cl₂-C₆H₃ |
| A.1698 | H | OCH₃ | H | iso-Propyl | 2,3-F₂-C₆H₃ |
| A.1699 | H | OCH₃ | H | iso-Propyl | CH₃ |
| A.1700 | H | OCH₃ | H | iso-Propyl | Ethyl |
| A.1701 | H | OCH₃ | H | iso-Propyl | n-Propyl |
| A.1702 | H | OCH₃ | H | iso-Propyl | iso-Propyl |
| A.1703 | H | OCH₃ | H | iso-Propyl | tert. Butyl |
| A.1704 | H | OCH₃ | H | iso-Propyl | n-Butyl |
| A.1705 | H | OCH₃ | H | iso-Propyl | C₆H₅ |
| A.1706 | H | OCH₃ | H | iso-Propyl | 2-F-C₆H₄ |
| A.1707 | H | OCH₃ | H | iso-Propyl | 2-Cl-C₆H₄ |
| A.1708 | H | OCH₃ | H | iso-Propyl | 2-CH₃-C₆H₄ |
| A.1709 | H | OCH₃ | H | iso-Propyl | 2-CF₃-C₆H₄ |
| A.1710 | H | OCH₃ | H | iso-Propyl | 3-F-C₆H₄ |
| A.1711 | H | OCH₃ | H | iso-Propyl | 3-Cl-C₆H₄ |
| A.1712 | H | OCH₃ | H | iso-Propyl | 3-CH₃-C₆H₄ |
| A.1713 | H | OCH₃ | H | iso-Propyl | 3-CF₃-C₆H₄ |
| A.1714 | H | OCH₃ | H | iso-Propyl | 3-Br-C₆H₄ |
| A.1715 | H | OCH₃ | H | iso-Propyl | 4-F-C₆H₄ |
| A.1716 | H | OCH₃ | H | iso-Propyl | 4-Cl-C₆H₄ |
| A.1717 | H | OCH₃ | H | iso-Propyl | 4-CH₃-C₆H₄ |
| A.1718 | H | OCH₃ | H | iso-Propyl | 4-CF₃-C₆H₄ |
| A.1719 | H | OCH₃ | H | iso-Propyl | 4-OCH₃-C₆H₄ |
| A.1720 | H | OCH₃ | H | iso-Propyl | 4-OCF₃-C₆H₄ |
| A.1721 | H | OCH₃ | H | iso-Propyl | 4-Br-C₆H₄ |
| A.1722 | H | OCH₃ | H | iso-Propyl | 4-(CH=NOCH₃)-C₆H₄ |
| A.1723 | H | OCH₃ | H | iso-Propyl | 4-(CH=NOEt)-C₆H₄ |
| A.1724 | H | OCH₃ | H | iso-Propyl | 4-[C(CH₃)=NOCH₃]-C₆H₄ |
| A.1725 | H | OCH₃ | H | iso-Propyl | 4-[C(CH₃)=NOEt]-C₆H₄ |
| A.1726 | H | OCH₃ | H | iso-Propyl | 2,4-F₂-C₆H₃ |
| A.1727 | H | OCH₃ | H | iso-Propyl | 2,4-Cl₂-C₆H₃ |
| A.1728 | H | OCH₃ | H | iso-Propyl | 2,4-(CH₃)₂-C₆H₃ |
| A.1729 | H | OCH₃ | H | iso-Propyl | 3,5-F₂-C₆H₃ |
| A.1730 | H | OCH₃ | H | iso-Propyl | 3,5-Cl₂-C₆H₃ |
| A.1731 | H | OCH₃ | H | iso-Propyl | 3,5-(CH₃)₂-C₆H₃ |
| A.1732 | H | OCH₃ | H | iso-Propyl | 2-F-4-Cl-C₆H₃ |
| A.1733 | H | OCH₃ | H | iso-Propyl | 2-Cl-4-F-C₆H₃ |
| A.1734 | H | OCH₃ | H | iso-Propyl | 3,4-F₂-C₆H₃ |
| A.1735 | H | OCH₃ | H | iso-Propyl | 3,4-Cl₂-C₆H₃ |
| A.1736 | H | OCH₃ | H | iso-Propyl | 2,3-F₂-C₆H₃ |
| A.1737 | H | OCH₃ | H | tert. Butyl | CH₃ |
| A.1738 | H | OCH₃ | H | tert. Butyl | Ethyl |
| A.1739 | H | OCH₃ | H | tert. Butyl | n-Propyl |
| A.1740 | H | OCH₃ | H | tert. Butyl | iso-Propyl |
| A.1741 | H | OCH₃ | H | tert. Butyl | tert. Butyl |
| A.1742 | H | OCH₃ | H | tert. Butyl | n-Butyl |
| A.1743 | H | OCH₃ | H | tert. Butyl | C₆H₅ |
| A.1744 | H | OCH₃ | H | tert. Butyl | 2-F-C₆H₄ |
| A.1745 | H | OCH₃ | H | tert. Butyl | 2-Cl-C₆H₄ |
| A.1746 | H | OCH₃ | H | tert. Butyl | 2-CH₃-C₆H₄ |
| A.1747 | H | OCH₃ | H | tert. Butyl | 2-CF₃-C₆H₄ |
| A.1748 | H | OCH₃ | H | tert. Butyl | 3-F-C₆H₄ |
| A.1749 | H | OCH₃ | H | tert. Butyl | 3-Cl-C₆H₄ |
| A.1750 | H | OCH₃ | H | tert. Butyl | 3-CH₃-C₆H₄ |
| A.1751 | H | OCH₃ | H | tert. Butyl | 3-CF₃-C₆H₄ |
| A.1752 | H | OCH₃ | H | tert. Butyl | 3-Br-C₆H₄ |
| A.1753 | H | OCH₃ | H | tert. Butyl | 4-F-C₆H₄ |
| A.1754 | H | OCH₃ | H | tert. Butyl | 4-Cl-C₆H₄ |
| A.1755 | H | OCH₃ | H | tert. Butyl | 4-CH₃-C₆H₄ |
| A.1756 | H | OCH₃ | H | tert. Butyl | 4-CF₃-C₆H₄ |
| A.1757 | H | OCH₃ | H | tert. Butyl | 4-OCH₃-C₆H₄ |
| A.1758 | H | OCH₃ | H | tert. Butyl | 4-OCF₃-C₆H₄ |
| A.1759 | H | OCH₃ | H | tert. Butyl | 4-Br-C₆H₄ |
| A.1760 | H | OCH₃ | H | tert. Butyl | 4-(CH=NOCH₃)-C₆H₄ |
| A.1761 | H | OCH₃ | H | tert. Butyl | 4-(CH=NOEt)-C₆H₄ |
| A.1762 | H | OCH₃ | H | tert. Butyl | 4-[C(CH₃)=NOCH₃]-C₆H₄ |
| A.1763 | H | OCH₃ | H | tert. Butyl | 4-[C(CH₃)=NOEt]-C₆H₄ |
| A.1764 | H | OCH₃ | H | tert. Butyl | 2,4-F₂-C₆H₃ |
| A.1765 | H | OCH₃ | H | tert. Butyl | 2,4-Cl₂-C₆H₃ |
| A.1766 | H | OCH₃ | H | tert. Butyl | 2,4-(CH₃)₂-C₆H₃ |
| A.1767 | H | OCH₃ | H | tert. Butyl | 3,5-F₂-C₆H₃ |
| A.1768 | H | OCH₃ | H | tert. Butyl | 3,5-Cl₂-C₆H₃ |
| A.1769 | H | OCH₃ | H | tert. Butyl | 3,5-(CH₃)₂-C₆H₃ |
| A.1770 | H | OCH₃ | H | tert. Butyl | 2-F-4-Cl-C₆H₃ |
| A.1771 | H | OCH₃ | H | tert. Butyl | 2-Cl-4-F-C₆H₃ |
| A.1772 | H | OCH₃ | H | tert. Butyl | 3,4-F₂-C₆H₃ |
| A.1773 | H | OCH₃ | H | tert. Butyl | 3,4-Cl₂-C₆H₃ |
| A.1774 | H | OCH₃ | H | tert. Butyl | 2,3-F₂-C₆H₃ |
| A.1775 | H | OCH₃ | H | tert. Butyl | CH₃ |
| A.1776 | H | OCH₃ | H | tert. Butyl | Ethyl |
| A.1777 | H | OCH₃ | H | tert. Butyl | n-Propyl |
| A.1778 | H | OCH₃ | H | tert. Butyl | iso-Propyl |
| A.1779 | H | OCH₃ | H | tert. Butyl | tert. Butyl |
| A.1780 | H | OCH₃ | H | tert. Butyl | n-Butyl |
| A.1781 | H | OCH₃ | H | tert. Butyl | C₆H₅ |
| A.1782 | H | OCH₃ | H | tert. Butyl | 2-F-C₆H₄ |
| A.1783 | H | OCH₃ | H | tert. Butyl | 2-Cl-C₆H₄ |
| A.1784 | H | OCH₃ | H | tert. Butyl | 2-CH₃-C₆H₄ |
| A.1785 | H | OCH₃ | H | tert. Butyl | 2-CF₃-C₆H₄ |
| A.1786 | H | OCH₃ | H | tert. Butyl | 3-F-C₆H₄ |
| A.1787 | H | OCH₃ | H | tert. Butyl | 3-Cl-C₆H₄ |
| A.1788 | H | OCH₃ | H | tert. Butyl | 3-CH₃-C₆H₄ |
| A.1789 | H | OCH₃ | H | tert. Butyl | 3-CF₃-C₆H₄ |
| A.1790 | H | OCH₃ | H | tert. Butyl | 3-Br-C₆H₄ |
| A.1791 | H | OCH₃ | H | tert. Butyl | 4-F-C₆H₄ |
| A.1792 | H | OCH₃ | H | tert. Butyl | 4-Cl-C₆H₄ |
| A.1793 | H | OCH₃ | H | tert. Butyl | 4-CH₃-C₆H₄ |
| A.1794 | H | OCH₃ | H | tert. Butyl | 4-CF₃-C₆H₄ |
| A.1795 | H | OCH₃ | H | tert. Butyl | 4-OCH₃-C₆H₄ |
| A.1796 | H | OCH₃ | H | tert. Butyl | 4-OCF₃-C₆H₄ |
| A.1797 | H | OCH₃ | H | tert. Butyl | 4-Br-C₆H₄ |
| A.1798 | H | OCH₃ | H | tert. Butyl | 4-(CH=NOCH₃)-C₆H₄ |
| A.1799 | H | OCH₃ | H | tert. Butyl | 4-(CH=NOEt)-C₆H₄ |
| A.1800 | H | OCH₃ | H | tert. Butyl | 4-[C(CH₃)=NOCH₃]-C₆H₄ |
| A.1801 | H | OCH₃ | H | tert. Butyl | 4-[C(CH₃)=NOEt]-C₆H₄ |
| A.1802 | H | OCH₃ | H | tert. Butyl | 2,4-F₂-C₆H₃ |
| A.1803 | H | OCH₃ | H | tert. Butyl | 2,4-Cl₂-C₆H₃ |
| A.1804 | H | OCH₃ | H | tert. Butyl | 2,4-(CH₃)₂-C₆H₃ |
| A.1805 | H | OCH₃ | H | tert. Butyl | 3,5-F₂-C₆H₃ |
| A.1806 | H | OCH₃ | H | tert. Butyl | 3,5-Cl₂₋C₆H₃ |
| A.1807 | H | OCH₃ | H | tert. Butyl | 3,5-(CH₃)2-C₆H₃ |
| A.1808 | H | OCH₃ | H | tert. Butyl | 2-F-4-Cl-C₆H₃ |
| A.1809 | H | OCH₃ | H | tert. Butyl | 2-Cl-4-F-C₆H₃ |
| A.1810 | H | OCH₃ | H | tert. Butyl | 3,4-F₂-C₆H₃ |
| A.1811 | H | OCH₃ | H | tert. Butyl | 3,4-Cl₂-C₆H₃ |
| A.1812 | H | OCH₃ | H | tert. Butyl | 2,3-F₂-C₆H₃ |
| A.1813 | H | CF₃ | H | H | C₆H₅ |
| A.1814 | H | CF₃ | H | CH₃ | C₆H₅ |
| A.1815 | H | CF₃ | H | Ethyl | C₆H₅ |
| A.1816 | H | CF₃ | H | n-Propyl | C₆H₅ |
| A.1817 | H | CF₃ | H | n-Butyl | C₆H₅ |
| A.1818 | H | CF₃ | H | iso-Propyl | C₆H₅ |
| A.1819 | H | CF₃ | H | tert. Butyl | C₆H₅ |
| A.1820 | H | CF₃ | H | H | 4-F-C₆H₄ |
| A.1821 | H | CF₃ | H | CH₃ | 4-F-C₆H₄ |
| A.1822 | H | CF₃ | H | Ethyl | 4-F-C₆H₄ |
| A.1823 | H | CF₃ | H | n-Propyl | 4-F-C₆H₄ |
| A.1824 | H | CF₃ | H | n-Butyl | 4-F-C₆H₄ |
| A.1825 | H | CF₃ | H | iso-Propyl | 4-F-C₆H₄ |
| A.1826 | H | CF₃ | H | tert. Butyl | 4-F-C₆H₄ |
| A.1827 | H | CH₃ | CH₃ | CH₃ | CH₃ |
| A.1828 | H | CH₃ | CH₃ | CH₃ | Ethyl |
| A.1829 | H | CH₃ | CH₃ | CH₃ | n-Propyl |
| A.1830 | H | CH₃ | CH₃ | CH₃ | iso-Propyl |
| A.1831 | H | CH₃ | CH₃ | CH₃ | tert. Butyl |
| A.1832 | H | CH₃ | CH₃ | CH₃ | n-Butyl |
| A.1833 | H | CH₃ | CH₃ | CH₃ | C₆H₅ |
| A.1834 | H | CH₃ | CH₃ | CH₃ | 2-F-C₆H₄ |
| A.1835 | H | CH₃ | CH₃ | CH₃ | 2-Cl-C₆H₄ |
| A.1836 | H | CH₃ | CH₃ | CH₃ | 2-CH₃-C₆H₄ |
| A.1837 | H | CH₃ | CH₃ | CH₃ | 2-CF₃-C₆H₄ |
| A.1838 | H | CH₃ | CH₃ | CH₃ | 3-F-C₆H₄ |
| A.1839 | H | CH₃ | CH₃ | CH₃ | 3-Cl-C₆H₄ |
| A.1840 | H | CH₃ | CH₃ | CH₃ | 3-CH₃-C₆H₄ |
| A.1841 | H | CH₃ | CH₃ | CH₃ | 3-CF₃-C₆H₄ |
| A.1842 | H | CH₃ | CH₃ | CH₃ | 3-Br-C₆H₄ |
| A.1843 | H | CH₃ | CH₃ | CH₃ | 4-F-C₆H₄ |
| A.1844 | H | CH₃ | CH₃ | CH₃ | 4-Cl-C₆H₄ |
| A.1845 | H | CH₃ | CH₃ | CH₃ | 4-CH₃-C₆H₄ |
| A.1846 | H | CH₃ | CH₃ | CH₃ | 4-CF₃-C₆H₄ |
| A.1847 | H | CH₃ | CH₃ | CH₃ | 4-OCH₃-C₆H₄ |
| A.1848 | H | CH₃ | CH₃ | CH₃ | 4-OCF₃-C₆H₄ |
| A.1849 | H | CH₃ | CH₃ | CH₃ | 4-Br-C₆H₄ |
| A.1850 | H | CH₃ | CH₃ | CH₃ | 4-(CH=NOCH₃)-C₆H₄ |
| A.1851 | H | CH₃ | CH₃ | CH₃ | 4-(CH=NOEt)-C₆H₄ |
| A.1852 | H | CH₃ | CH₃ | CH₃ | 4-[C(CH₃)=NOCH₃]-C₆H₄ |
| A.1853 | H | CH₃ | CH₃ | CH₃ | 4-[C(CH₃)=NOEt]-C₆H₄ |
| A.1854 | H | CH₃ | CH₃ | CH₃ | 2,4-F₂-C₆H₃ |
| A.1855 | H | CH₃ | CH₃ | CH₃ | 2,4-Cl₂-C₆H₃ |
| A.1856 | H | CH₃ | CH₃ | CH₃ | 2,4-(CH₃)₂-C₆H₃ |
| A.1857 | H | CH₃ | CH₃ | CH₃ | 3,5-F₂-C₆H₃ |
| A.1858 | H | CH₃ | CH₃ | CH₃ | 3,5-Cl₂-C₆H₃ |
| A.1859 | H | CH₃ | CH₃ | CH₃ | 3,5-(CH₃)₂-C₆H₃ |
| A.1860 | H | CH₃ | CH₃ | CH₃ | 2-F-4-Cl-C₆H₃ |
| A.1861 | H | CH₃ | CH₃ | CH₃ | 2-Cl-4-F-C₆H₃ |
| A.1862 | H | CH₃ | CH₃ | CH₃ | 3,4-F₂-C₆H₃ |
| A.1863 | H | CH₃ | CH₃ | CH₃ | 3,4-Cl₂-C₆H₃ |
| A.1864 | H | CH₃ | CH₃ | CH₃ | 2,3-F₂-C₆H₃ |
| A.1865 | H | CH₃ | CH₃ | Ethyl | CH₃ |
| A.1866 | H | CH₃ | CH₃ | Ethyl | Ethyl |
| A.1867 | H | CH₃ | CH₃ | Ethyl | n-Propyl |
| A.1868 | H | CH₃ | CH₃ | Ethyl | iso-Propyl |
| A.1869 | H | CH₃ | CH₃ | Ethyl | tert. Butyl |
| A.1870 | H | CH₃ | CH₃ | Ethyl | n-Butyl |
| A.1871 | H | CH₃ | CH₃ | Ethyl | C₆H₅ |
| A.1872 | H | CH₃ | CH₃ | Ethyl | 2-F-C₆H₄ |
| A.1873 | H | CH₃ | CH₃ | Ethyl | 2-Cl-C₆H₄ |
| A.1874 | H | CH₃ | CH₃ | Ethyl | 2-CH₃-C₆H₄ |
| A.1875 | H | CH₃ | CH₃ | Ethyl | 2-CF₃-C₆H₄ |
| A.1876 | H | CH₃ | CH₃ | Ethyl | 3-F-C₆H₄ |
| A.1877 | H | CH₃ | CH₃ | Ethyl | 3-Cl-C₆H₄ |
| A.1878 | H | CH₃ | CH₃ | Ethyl | 3-CH₃-C₆H₄ |
| A.1879 | H | CH₃ | CH₃ | Ethyl | 3-CF₃-C₆H₄ |
| A.1880 | H | CH3 | CH₃ | Ethyl | 3-Br-C₆H₄ |
| A.1881 | H | CH₃ | CH₃ | Ethyl | 4-F-C₆H₄ |
| A.1882 | H | CH₃ | CH₃ | Ethyl | 4-Cl-C₆H₄ |
| A.1883 | H | CH₃ | CH₃ | Ethyl | 4-CH₃-C₆H₄ |
| A.1884 | H | CH₃ | CH₃ | Ethyl | 4-CF₃-C₆H₄ |
| A.1885 | H | CH₃ | CH₃ | Ethyl | 4-OCH₃-C₆H₄ |
| A.1886 | H | CH₃ | CH₃ | Ethyl | 4-OCF₃-C₆H₄ |
| A.1887 | H | CH₃ | CH₃ | Ethyl | 4-Br-C₆H₄ |
| A.1888 | H | CH₃ | CH₃ | Ethyl | 4-(CH=NOCH₃)-C₆H₄ |
| A.1889 | H | CH₃ | CH₃ | Ethyl | 4-(CH=NOEt)-C₆H₄ |
| A.1890 | H | CH₃ | CH₃ | Ethyl | 4-[C(CH₃)=NOCH₃]-C₆H₄ |
| A.1891 | H | CH₃ | CH₃ | Ethyl | 4-[C(CH₃)=NOEt]-C₆H₄ |
| A.1892 | H | CH₃ | CH₃ | Ethyl | 2,4-F₂-C₆H₃ |
| A.1893 | H | CH₃ | CH₃ | Ethyl | 2,4-Cl₂-C₆H₃ |
| A.1894 | H | CH₃ | CH₃ | Ethyl | 2,4-(CH₃)₂-C₆H₃ |
| A.1895 | H | CH₃ | CH₃ | Ethyl | 3,5-F₂-C₆H₃ |
| A.1896 | H | CH₃ | CH₃ | Ethyl | 3,5-Cl₂-C₆H₃ |
| A.1897 | H | CH₃ | CH₃ | Ethyl | 3,5-(CH₃)₂-C₆H₃ |
| A.1898 | H | CH₃ | CH₃ | Ethyl | 2-F-4-Cl-C₆H₃ |
| A.1899 | H | CH₃ | CH₃ | Ethyl | 2-Cl-4-F-C₆H₃ |
| A.1900 | H | CH₃ | CH₃ | Ethyl | 3,4-F₂-C₆H₃ |
| A.1901 | H | CH₃ | CH₃ | Ethyl | 3,4-Cl₂-C₆H₃ |
| A.1902 | H | CH₃ | CH₃ | Ethyl | 2,3-F₂-C₆H₃ |
| Et = Ethyl | | | | | |

**Tabelle B:**

| Nr. | **X** | **R**^{**3**} | **R**^{**4**} | **R**^{**a**} |
|---|---|---|---|---|
| B.1 | N(COOCH₃)-OCH₃ | H | CH₃ | H |
| B.2 | C(COOCH₃)=COCH₃ | H | CH₃ | H |
| B.3 | C(COOCH₃)=NOCH₃ | H | CH₃ | H |
| B.4 | C(COOCH₃)=C-CH₃ | H | CH₃ | H |
| B.5 | C(CONHCH₃)=NOCH₃ | H | CH₃ | H |
| B.6 | N(COOCH₃)-OCH₃ | H | C₆H₅ | H |
| B.7 | C(COOCH₃)=COCH₃ | H | C₆H₅ | H |
| B.8 | C(COOCH₃)=NOCH₃ | H | C₆H₅ | H |
| B.9 | C(COOCH₃)=C-CH₃ | H | C₆H₅ | H |
| B.10 | C(CONHCH₃)=NOCH₃ | H | C₆H₅ | H |
| B.11 | N(COOCH₃)-OCH₃ | H | 4-F-C₆H₄ | H |
| B.12 | C(COOCH₃)=COCH₃ | H | 4-F-C₆H₄ | H |
| B.13 | C(COOCH₃)=NOCH₃ | H | 4-F-C₆H₄ | H |
| B.14 | C(COOCH₃)=C-CH₃ | H | 4-F-C₆H₄ | H |
| B.15 | C(CONHCH₃)=NOCH₃ | H | 4-F-C₆H₄ | H |
| B.16 | N(COOCH₃)-OCH₃ | H | CH₃ | CH₃ |
| B.17 | C(COOCH₃)=COCH₃ | H | CH₃ | CH₃ |
| B.18 | C(COOCH₃)=NOCH₃ | H | CH₃ | CH₃ |
| B.19 | C(COOCH₃)=C-CH₃ | H | CH₃ | CH₃ |
| B.20 | C(CONHCH₃)=NOCH₃ | H | CH₃ | CH₃ |
| B.21 | N(COOCH₃)-OCH₃ | H | C₆H₅ | CH₃ |
| B.22 | C(COOCH₃)=COCH₃ | H | C₆H₅ | CH₃ |
| B.23 | C(COOCH₃)=NOCH₃ | H | C₆H₅ | CH₃ |
| B.24 | C(COOCH₃)=C-CH₃ | H | C₆H₅ | CH₃ |
| B.25 | C(CONHCH₃)=NOCH₃ | H | C₆H₅ | CH₃ |
| B.26 | N(COOCH₃)-OCH₃ | H | 4-F-C₆H₄ | CH₃ |
| B.27 | C(COOCH₃)=COCH₃ | H | 4-F-C₆H₄ | CH₃ |
| B.28 | C(COOCH₃)=NOCH₃ | H | 4-F-C₆H₄ | CH₃ |
| B.29 | C(COOCH₃)=C-CH₃ | H | 4-F-C₆H₄ | CH₃ |
| B.30 | C(CONHCH₃)=NOCH₃ | H | 4-F-C₆H₄ | CH₃ |
| B.31 | N(COOCH₃)-OCH₃ | CH₃ | CH₃ | H |
| B.32 | C(COOCH₃)=COCH₃ | CH₃ | CH₃ | H |
| B.33 | C(COOCH₃)=NOCH₃ | CH₃ | CH₃ | H |
| B.34 | C(COOCH₃)=C-CH₃ | CH₃ | CH₃ | H |
| B.35 | C(CONHCH₃)=NOCH₃ | CH₃ | CH₃ | H |
| B.36 | N(COOCH₃)-OCH₃ | CH₃ | C₆H₅ | H |
| B.37 | C(COOCH₃)=COCH₃ | CH₃ | C₆H₅ | H |
| B.38 | C(COOCH₃)=NOCH₃ | CH₃ | C₆H₅ | H |
| B.39 | C(COOCH₃)=C-CH₃ | CH₃ | C₆H₅ | H |
| B.40 | C(CONHCH₃)=NOCH₃ | CH₃ | C₆H₅ | H |
| B.41 | N(COOCH₃)-OCH₃ | CH₃ | 4-F-C₆H₄ | H |
| B.42 | C(COOCH₃)=COCH₃ | CH₃ | 4-F-C₆H₄ | H |
| B.43 | C(COOCH₃)=NOCH₃ | CH₃ | 4-F-C₆H₄ | H |
| B.44 | C(COOCH₃)=C-CH₃ | CH₃ | 4-F-C₆H₄ | H |
| B.45 | C(CONHCH₃)=NOCH₃ | CH₃ | 4-F-C₆H₄ | H |
| B.46 | N(COOCH₃)-OCH₃ | CH₃ | CH₃ | CH₃ |
| B.47 | C(COOCH₃)=COCH₃ | CH₃ | CH₃ | CH₃ |
| B.48 | C(COOCH₃)=NOCH₃ | CH₃ | CH₃ | CH₃ |
| B.49 | C(COOCH₃)=C-CH₃ | CH₃ | CH₃ | CH₃ |
| B.50 | C(CONHCH₃)=NOCH₃ | CH₃ | CH₃ | CH₃ |
| B.51 | N(COOCH₃)-OCH₃ | CH₃ | C₆H₅ | CH₃ |
| B.52 | C(COOCH₃)=COCH₃ | CH₃ | C₆H₅ | CH₃ |
| B.53 | C(COOCH₃)=NOCH₃ | CH₃ | C₆H₅ | CH₃ |
| B.54 | C(COOCH₃)=C-CH₃ | CH₃ | C₆H₅ | CH₃ |
| B.55 | C(CONHCH₃)=NOCH₃ | CH₃ | C₆H₅ | CH₃ |
| B.56 | N(COOCH₃)-OCH₃ | CH₃ | 4-F-C₆H₄ | CH₃ |
| B.57 | C(COOCH₃)=COCH₃ | CH₃ | 4-F-C₆H₄ | CH₃ |
| B.58 | C(COOCH₃)=NOCH₃ | CH₃ | 4-F-C₆H₄ | CH₃ |
| B.59 | C(COOCH₃)=C-CH₃ | CH₃ | 4-F-C₆H₄ | CH₃ |
| B.60 | C(CONHCH₃)=NOCH₃ | CH₃ | 4-F-C₆H₄ | CH₃ |

**Tabelle C:**

| Nr. | **X** | **R**^{**2**} | **R**^{**3**} | **R**^{**4**} | **R**^{**a**} |
|---|---|---|---|---|---|
| C.1 | N(COOCH₃)-OCH₃ | H | H | CH₃ | H |
| C.2 | C(COOCH₃)=COCH₃ | H | H | CH₃ | H |
| C.3 | C(COOCH₃)=NOCH₃ | H | H | CH₃ | H |
| C.4 | C(COOCH₃)=C-CH₃ | H | H | CH₃ | H |
| C.5 | C(CONHCH₃)=NOCH₃ | H | H | CH₃ | H |
| C.6 | N(COOCH₃)-OCH₃ | H | H | C₆H₅ | H |
| C.7 | C(COOCH₃)=COCH₃ | H | H | C₆H₅ | H |
| C.8 | C(COOCH₃)=NOCH₃ | H | H | C₆H₅ | H |
| C.9 | C(COOCH₃)=C-CH₃ | H | H | C₆H₅ | H |
| C.10 | C(CONHCH₃)=NOCH₃ | H | H | C₆H₅ | H |
| C.11 | N(COOCH₃)-OCH₃ | H | H | 4-F-C₆H₄ | H |
| C.12 | C(COOCH₃)=COCH₃ | H | H | 4-F-C₆H₄ | H |
| C.13 | C(COOCH₃)=NOCH₃ | H | H | 4-F-C₆H₄ | H |
| C.14 | C(COOCH₃)=C-CH₃ | H | H | 4-F-C₆H₄ | H |
| C.15 | C(CONHCH₃)=NOCH₃ | H | H | 4-F-C₆H₄ | H |
| C.16 | N(COOCH₃)-OCH₃ | H | CH₃ | CH₃ | CH₃ |
| C.17 | C(COOCH₃)=COCH₃ | H | CH₃ | CH₃ | CH₃ |
| C.18 | C(COOCH₃)=NOCH₃ | H | CH₃ | CH₃ | CH₃ |
| C.19 | C(COOCH₃)=C-CH₃ | H | CH₃ | CH₃ | CH₃ |
| C.20 | C(CONHCH₃)=NOCH₃ | H | CH₃ | CH₃ | CH₃ |
| C.21 | N(COOCH₃)-OCH₃ | H | CH₃ | C₆H₅ | CH₃ |
| C.22 | C(COOCH₃)=COCH₃ | H | CH₃ | C₆H₅ | CH₃ |
| C.23 | C(COOCH₃)=NOCH₃ | H | CH₃ | C₆H₅ | CH₃ |
| C.24 | C(COOCH₃)=C-CH₃ | H | CH₃ | C₆H₅ | CH₃ |
| C.25 | C(CONHCH₃)=NOCH₃ | H | CH3 | C₆H₅ | CH3 |
| C.26 | N(COOCH₃)-OCH₃ | H | CH₃ | 4-F-C₆H₄ | CH₃ |
| C.27 | C(COOCH₃)=COCH₃ | H | CH₃ | 4-F-C₆H₄ | CH₃ |
| C.28 | C(COOCH₃)=NOCH₃ | H | CH₃ | 4-F-C₆H₄ | CH₃ |
| C.29 | C(COOCH₃)=C-CH₃ | H | CH₃ | 4-F-C₆H₄ | CH₃ |
| C.30 | C(CONHCH₃)=NOCH₃ | H | CH₃ | 4-F-C₆H₄ | CH₃ |
| C.31 | N(COOCH₃)-OCH₃ | H | CH₃ | C₆H₅ | Ethyl |
| C.32 | C(COOCH₃)=COCH₃ | H | CH₃ | C₆H₅ | Ethyl |
| C.33 | C(COOCH₃)=NOCH₃ | H | CH₃ | C₆H₅ | Ethyl |
| C.34 | C(COOCH₃)=C-CH₃ | H | CH₃ | C₆H₅ | Ethyl |
| C.35 | C(CONHCH₃)=NOCH₃ | H | CH₃ | C₆H₅ | Ethyl |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria*-Arten an Gemüse und Obst,
- *Botrytis cinerea* (Grauschimmel) an Erdbeeren, Gemüse, Zierpflanzen und Reben,
- *Cercospora arachidicola* an Erdnüssen,
- *Erysiphe cichoracearum* und *Sphaerotheca fuliginea* an Kürbisgewächsen,
- *Erysiphe graminis* (echter Mehltau) an Getreide,
- *Fusarium-* und *Verticillium*-Arten an verschiedenen Pflanzen,
- *Helminthosporium*-Arten an Getreide,
- *Myccsphaerella-*Arten an Bananen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora*-Arten an Hopfen und Gurken,
- *Puccinia*-Arten an Getreide,
- *Pyricularia oryzae* an Reis,
- *Rhizoctonia*-Arten an Baumwolle, Reis und Rasen,
- *Septoria nodorim* an Weizen,
- *Uncinula necator* an Reben,
- *Ustilago-*Arten an Getreide und Zuckerrohr, sowie
- *Venturia inaequalis* (Schorf) an Äpfeln.

Die Verbindungen I eignen sich außerdem zur Bekämpfung von Schadpilzen wie *Paecilomyces variotii* im Materialschutz (z.B. Holz, Papier, Dispersionen für den Ansprich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glössina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeürodes vaporariorum und Viteus vitifolii,
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchüs neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
v. 80 Gew.-Teile einer erfindungsgemäßen Verbindung werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (wirkstoffgehalt 80 Gew.-%).
VI. Man vermischt 90 Gew.-Teile einer erfindungsgemäßen Verbindung mit 10 Gew.-Teilen N-Methyl-a-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl)-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino)-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die fungizide Wirkung ist bevorzugt.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1 Herstellung von 2,2-Dimethoxy-1-phenyl-propan-1-on

In 14,8 g 1-Phenyl-propan-1,2-dion, gelöst in 27,5 ml Methanol, wurden 21,2 g Trimethoxymethan und 8,7 ml 25 gew.-%iger methanolischer HCl-Lösung gegeben. Das Reaktionsgemisch wurde 2 h bei 20 bis 25°C gerührt, dann das Lösungsmittel abdestilliert. Der Rückstand wurde in Methyl-tert.-butylether (MTBE) aufgenommen und die Lösung mit Wasser gewaschen. Die organischen Phasen wurden getrocknet und das Lösungsmittel abdestilliert. Man erhielt 18,2 g Produkt als helles Öl.

¹H-NMR (CDCl₃): 2,6.(s, 3H); 3,3 (s, 6H); 7,4 - 7,6 (m, 3H); 8,2 ppm (m, 2H).

### Beispiel 2 Herstellung von [1-(1,1-Dimethoxy-ethyl)-propenyl] -benzol

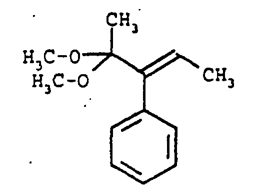

In 8,2 g Ethyltriphenylphosphoniumbromid, gelöst in 50 ml Tetrahydrofuran (THF) wurden bei 5°C eine Lösung von 2,5 g Kalium-tert.-butylat in 25 ml THF getropft. Dann wurde die Mischung etwa 10 min bei 5°C gerührt und tropfenweise mit einer Lösung von 3,9 g 2,2-Dimethoxy-1-phenyl-propan-1-on aus Beispiel 1 in 10 ml THF versetzt.Das Reaktionsgemisch wurde 72 h bei 20 bis 25°C gerührt. dann mit etwa 300 ml Wasser versetzt und mit Methyl-tert.-butylether (MTBE) extrahiert. Die organischen Phasen wurden mit Wasser gewaschen, getrocknet und vom Lösungsmittel befreit. Nach Chromatographie an Kieselgel (MTBE/Cyclohexan 1:10) wurden aus dem Rückstand 2,2 g Produkt als Isomerengemisch erhalten, das ohne Reinigung weiter umgesetzt werden kann.

### Beispiel 3 Herstellung von 3-Phenyl-pent-3-en-2-oxim

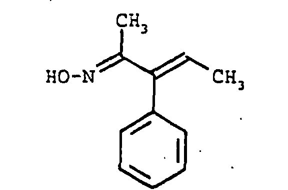

15,5 g Hydroxylammoniumhydrochlorid und 17,6 g Pyridin wurden in 140 ml Methanol vorgelegt. Dazu tropfte man'die Lösung von 30,7 g des in Beispiel 2 erhaltenen [1-(1,1-Dimethoxy-ethyl)-propenyl]-benzols in 140 ml Methanol und rührt 72 h bei 20 bis 25°C. Das Reaktionsgemisch wurde in etwa 1,5 Liter NaCl-Lösung aufgenommen und mit Methyl-tert.-butylether (MTBE) extrahiert. Die organischen Phasen wurden mit 5 gew.-%iger Salzsäure, dann mit wasser gewaschen. Nach Trocknen und Abdestillieren des Lösungsmittels erhielt man daraus 14,5 g Produkt als hellgelbes Pulver vom Fp. 99 - 101°C.

¹H-NMR (CDCl₃): 1,6 (d, 3H); 2,0 (s,3H); 6,2 (q, 1H) ; 7,0 - 7,4 (m, 5H); 8,5 ppm (s, 1H).

### Beispiel 4 Herstellung von (E)-2-Methoxyimino-2-(3-Phenyl-pent-3-en-2-iminooxymethyl)-phenylessigsäuremethylester

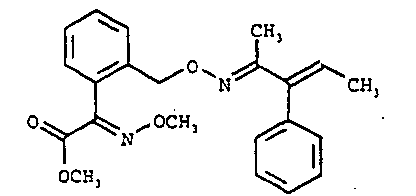

25 g (142 mmol) des in Beispiel 3 erhaltenen Oxims, gelöst in 100 ml Dimethylformamid (DMF), wurden mit 25 g 30 Gew.-%iger methanolischer Natriummethylat-Lösung versetzt. Nach 15 min Rühren bei 20 bis 25°C wurden 40,8 g (142 mmol) 2-Brommethyl-phenylglyoxylsäuremethylester-O-methyloxim, in 100 cnl DMF gelöst, zugetropft. Nach etwa 3 h Rühren bei 20 bis 25°C wurde das Reaktionsgemisch auf Eiswasser gegeben und mit Methyl-tert.-butylether (MTBE) extrahiert. Die organischen Phasen wurden gewaschen, getrocknet und vom Lösungsmittel befreit. Nach Chromatographie an Kieselgel (MTBE/Cyclohexan 1:9) wurden aus dem Rückstand 32 g der Titelverbindung erhalten.

¹H-NMR (CDCl₃): 1,6 (d, 3H) ; 3,8 (s, 3H); 4,0 (s, 3H); 5,0 (s, 2H); 6,2 (q, 1H); 7,1 - 7,5 ppm (m, 9H).

### Beispiel 5 Herstellung von (E)-2-Methoxyimino-2-[(3-Phenyl)-pent-3-en-2-iminooxymethyl]-phenyl-N-methylessigsäureamid

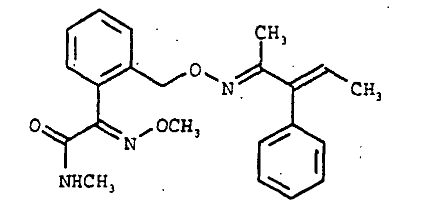

12,4 g (32,6 mmol) des in Beispiel 4 erhaltenen Esters wurden in 200 ml Tetrahydrofuran (THF) gelöst und mit 25,3 g 40 gew.-%iger wässriger Methylamin-Lösung tropfenweise versetzt. Nach 2 h Rühren bei 20 bis 25°C wurde die Lösung auf Eiswasser gegeben und mit Methyl-tert.-butylether (MTBE) extrahiert. Die organischen Phasen wurden gewaschen, getrocknet und vom Lösungsmittel befreit. Nach Chromatographie an Kieselgel (MTBE/Cyclohexan 1:3) wurden aus dem Rückstand 7,7 g Produkt erhalten.

¹H-MMR (CDCl₃): 1,6 (d, 3H); 1,8 (s, 3H); 2,85 (d, 3H); 3,9 (s, 3H); 4,95 (s, 2H); 6,1 (q, 1H); 6,7 (s, 1H); 7,1 - 7,5 ppm (m, 9H).

### Beispiel 6 Herstellung von (E)-2-Methoxyimino-2-[1-(3-Methyl-2-phenyl-oxiranyl)-ethylideniminooxymethyl]-phenyl-N-methylessigsäureamid

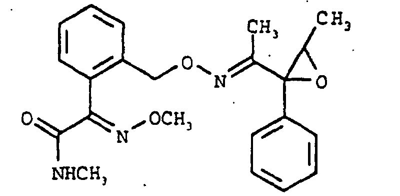

20,3 g (53,6 mmol) des in Beispiel 5 erhaltenen Amides wurden in 250 ml Methylenchlorid gelöst. Dazu wurden 24,4 g (77,7 mmol) 3-Chlorperbenzoesäure gegeben und die Reaktionsmischung 18 h bei 20 bis 25°C gerührt. Das Reaktionsgemisch wurde auf Wasser gegeben, mit Natriumthiosulfat- und Natriumhydroxid-Lösung, dann mit wasser gewaschen. Die organische Phase wurde abgetrennt und nach Trocknen vom Lösungsmittel befreit. Nach Chromatographie an Kieselgel (MTBE/Cyclohexan 1:3) wurden aus dem Rückstand 21,3 g Produkt als Öl erhalten.

¹H-NMR (CDCl₃): 1,0 (d, 3H); 1,8 (s, 3H); 2,8 (d, 3H) ; 3,7 (q, 1H); 4,0 (s, 3H); 5,0 (s, 2H); 6,7 (s, 1H) ; 6,7 (s, 1H); 7,2 - 7,5 ppm (m, 9H).

### Beispiel 7 Herstellung von (E)-2-Methoxyimino-2-[1-(1-phenyl-cyclopropyl)-ethylideniminooxymethyl]-phenylessigsäuremethylester

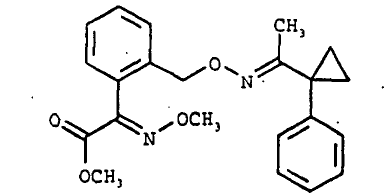

Zu 0,53 g Natriumhydrid in 30 ml wasserfreiem Dimethylformamid (DMF) tropfte man die Lösung von 3,5 g [1-(1-Phenyl-cyclopropyl)-ethyliden]-oxim in 10 ml DMF zu. Nach 2 h Rühren bei 60°C wurde bei 0°C 4,2 g 2-Brommethyl-phenylglyoxylsäuremethylester, in 20 ml DMF gelöst, zugetropft. Nach Rühren über Nacht bei 20 bis 25°C wurde die Lösung auf Eiswasser gegeben und mit Methyl-tert.-butylether (MTBE) extrahiert. Die organischen Phasen wurden gewaschen, getrocknet und vom Lösungsmittel befreit. Nach Chromatographie an Kieselgel (Toluol) wurden aus dem Rückstand 3 g der Titelverbindung als gelbe Kristalle mit dem Fp. 67 - 70°C erhalten.

¹H-NMR (CDCl₃): 0,9 (m, 2H) ; 1,2 (m, 2H) ; 1,8 (s, 3H) ; 3, 8 (s, 3H); 4,0 (s, 3H); 5,0 (s, 2H); 7,1 - 7,6 (m; 9H).

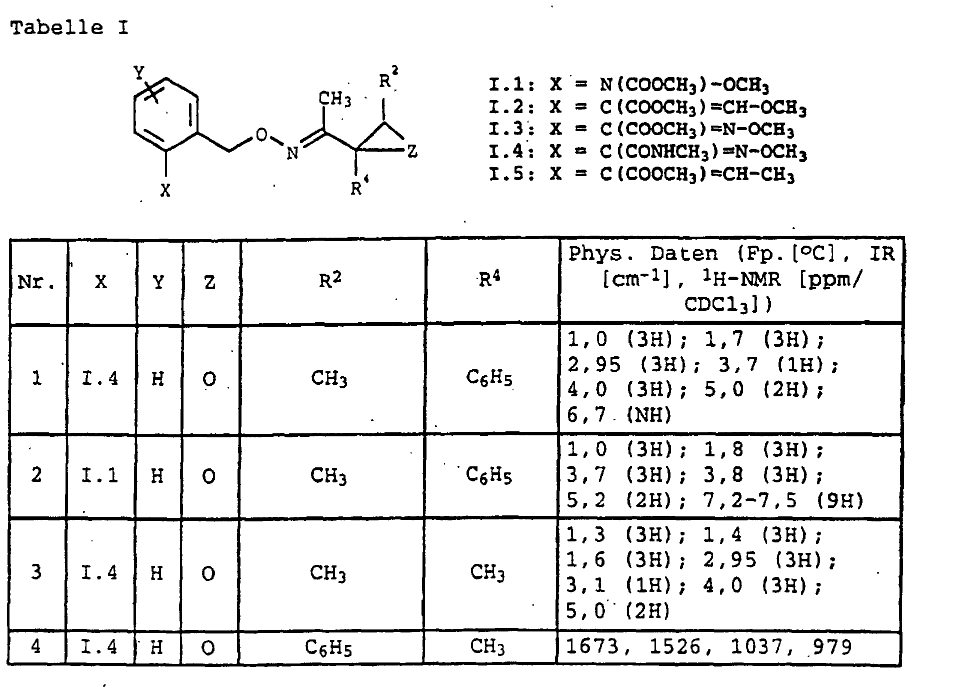

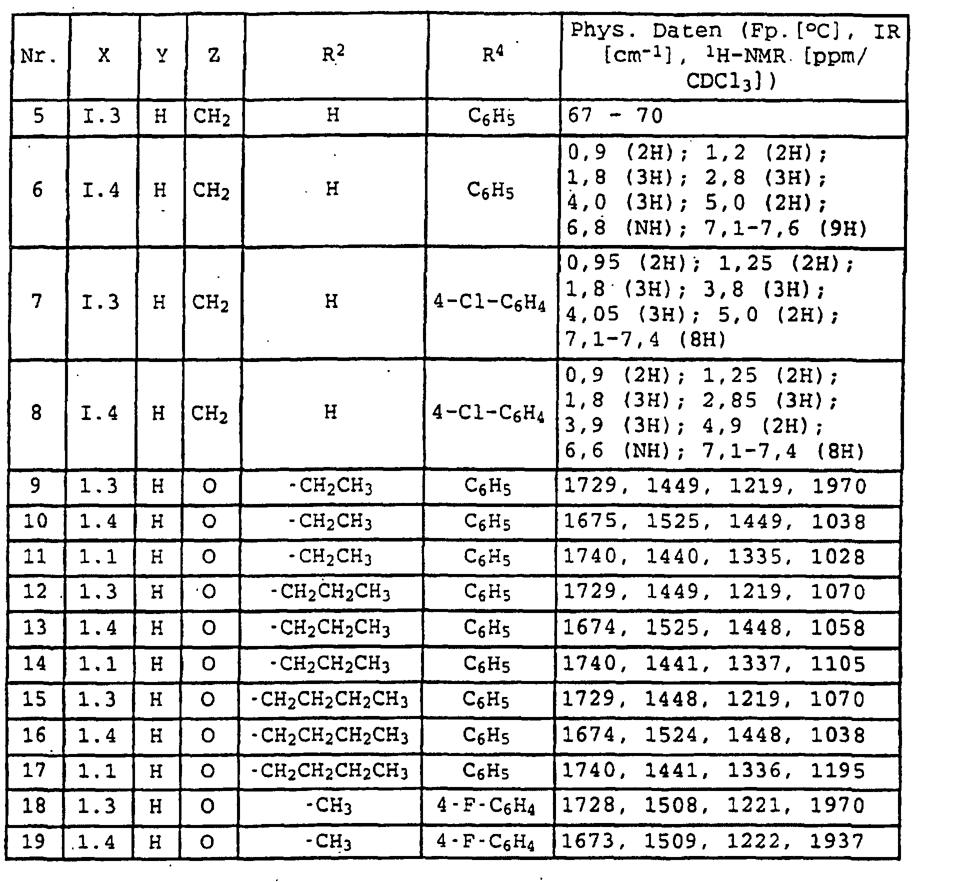

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 10%ige Emulsion in einem Gemisch aus 63 Gew.-% Cyclohexanon und 27 Gew.-% Emulgator aufbereitet und entsprechend der gewünschten Konzentration mit Wasser verdünnt.

Als Vergleichswirkstoffe dienten die aus EP-A 463 488 als Nrn. 56 der Tabellen I und II bekannten Verbindungen A und B:

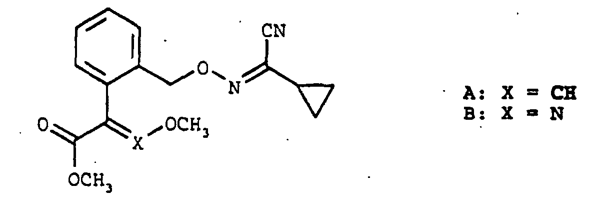

### Anwendungsbeispiel 1 - Wirksamkeit gegen Weizenmehltau

Blätter von in Töpfen gewachsenen Weizenkeimlingen der Sorte "Frühgold" wurden mit wäßriger Wirkstoffaufbereitung bis zur Tropfnässe besprüht und 24 Stunden nach dem Antrocknen des Spritzbelages mit Sporen des Weizenmehltaus (*Erysiphe graminis* forma specialis *tritici*) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung visuell in % Befall der gesamten Blattfläche ermittelt.

In diesem Test zeigten die mit 16 ppm der Verbindungen Nr. 1, 2, 3, 4, 9, 10, 11, 12, 13, 14 und 18 der Tabelle I behandelten Pflanzen nicht über 15 % Befall, während die mit 16 ppm der Vergleichsverbindungen A und B behandelten Pflanzen zu 40 % und die Unbehandelten zu 80 % befallen waren.

### Anwendungsbeispiel 2 - Wirksamkeit gegen Plasmopara viticola

Blätter von Topfreben der Sorte "Müller-Thurgau" wurden mit wäßriger Wirkstoffaufbereitung bis zur Tropfnässe besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoosporenaufschwemmung von *Plasmopara viticola* inokuliert. Danach wurden die Reben zunächst für 48 Stunden in einer wasserdampfgesättigten Kammer bei 24°C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In diesem Test zeigten die mit 16 ppm der Verbindungen Nr. 1, 4, 5, 6, 7, 8, 9, 10, 12, 13, 16, 18 und 19 der Tabelle I behandelten Pflanzen maximal 10 % Befall, während die mit 16 ppm der Vergleichsverbindung B behandelten Pflanzen zu 60 % und die Unbehandelten zu 80 % befallen waren.

### Anwendungsbeispiel 3 - Wirksamkeit gegen Pyricularia oryzae (protektiv)

Blätter von in Töpfen gewachsenen Reiskeimlingen der Sorte "Tai-Nong 67" wurden mit wäßriger Wirkstoffaufbereitung bis zur Tropfnässe besprüht. Am folgenden Tag wurden die Pflanzen mit einer wäßrigen Sporensuspension von *Pyricularia oryzae* inokuliert. Anschließend wurden die Versuchspflanzen in Klimakammern bei 22 bis 24°C und 95 bis 99 % relativer Luftfeuchtigkeit für 6 Tage aufgestellt. Dann wurde das Ausmaß der Befallsentwicklung auf den Blättern visuell ermittelt.

In diesem Test zeigten die mit 16 ppm der Verbindungen Nr. 1, 2, 4, 6, 7, 8, 9, 10, 11, 12, 13, 14, 15, 16, 18 und 19 der Tabelle I behandelten Pflanzen maximal 15 % Befall, während die mit 16 ppm der Vergleichsverbindung B behandelten Pflanzen zu 40 % und die Unbehandelten zu 80 % befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Benzyloxyimino-Verbindungen der Formel I, 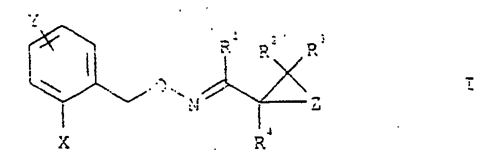 in der die Substituenten die folgenden Bedeutungen haben:
X N(COOCH₃)-OCH₃, C(COOCH₃)=CH-OCH₃, C(COOCH₃)=N-OCH₃, C(CONHCH₃)=N-OCH₃ oder C(COOCH₃)=CH-CH₃;
Y Wasserstoff, Halogen, C₁-C₄-Alkyl, C₁-C₄-Halogenalkyl oder C₁-C₄-Alkoxy;
Z Sauerstoff, C(R^{a})₂ oder NR^{a}, wobei
R^{a} für Wasserstoff oder C₁-C₄-Alkyl steht und im Fall C(R^{a})₂ gleich oder verschieden sein können;
R¹ Halogen, C₁-C₄-Alkyl, C₁-C₄-Halogenalkyl oder C₁-C₄-Alkoxy;
R² Wasserstoff, Halogen, C₁-C₄-Alkyl, C₁-C₄-Alkoxy oder gegebenenfalls durch übliche Gruppen substituiertes Phenyl;
R³ Wasserstoff, Halogen, C₁-C₄-Alkyl oder C₁-C₄-Alkoxy;
R⁴ C₁-C₄-Alkyl oder
Phenyl, das gegebenenfalls 1-3 Substituenten aus der folgenden Gruppe trägt: Halogen, C₁-C₄-Alkyl, C₁-C₄-Halogenalkyl, C₁-C₄-Alkoxy, C₁-C₄-Halogenalkoxy oder C(R^{b})=N-OR^{c}, wobei
R^{b} für Wasserstoff oder C₁-C₄-Alkyl und
R^{c} für C₁-C₄-Alkyl, C₃-C₄-Alkenyl oder C₃-C₄-Alkinyl steht,
wobei Z nicht für C(R^{a})₂ steht, wenn X C(COOCH₃)=CH-OCH₃ oder C(COOCH₃)=N-OCH₃ und R⁴ C₁-C₄-Alkyl bedeutet.

2. Verfahren zur Herstellung von Verbindungen der Formel I gemäß Anspruch 1, wobei Z für Sauerstoff und CR^{a} steht, **dadurch gekennzeichnet, daß** man ein Oxim der Formel II 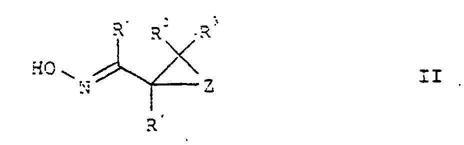 mit einer Benzylverbindung der Formel III, 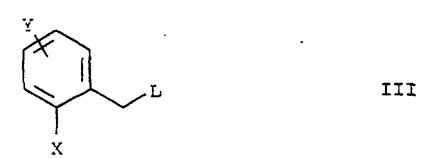 in der L für eine nucleophil austauschbare Gruppe steht, umsetzt.

3. Verfahren zur Herstellung von Verbindungen der Formel I gemäß Anspruch 1, in der Z für Sauerstoff steht, **dadurch gekennzeichnet, daß** man eine Benzylverbindung der Formel III gemäß Anspruch 2 mit einem Oxim der Formel IV 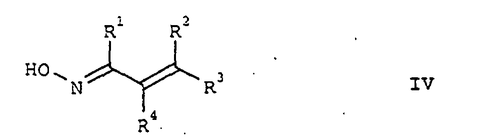 zu einem Oximether der Formel V 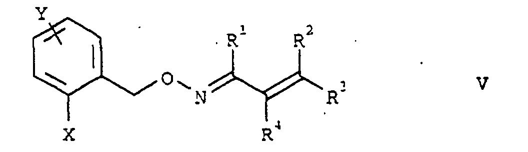 umsetzt, der anschließend durch Oxidation in eine Verbindung der Formel I übergeführt wird.

4. Verfahren zur Herstellung von Verbindungen der Formel I gemäß Anspruch 1, in der Z für NH steht, **dadurch gekennzeichnet, daß** man Verbindungen der Formel I, in der Z für Sauerstoff steht, mit Metallaziden umsetzt..

5. Verfahren zur Herstellung von Verbindungen der Formel I gemäß Anspruch 1, in der Z für N-C₁-C₄-Alkyl steht, **dadurch gekennzeichnet, daß** man Verbindungen der Formel I, in denen Z für NH steht, mit Alkylierungsmitteln umsetzt.

6. Zwischenprodukte der Formel II gemäß Anspruch 2, 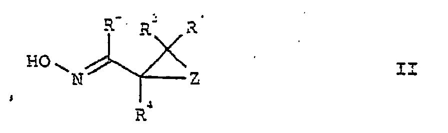 in der Z für Sauerstoff oder NR^{a} steht und die Substituenten R¹ bis R⁴ die in Anspruch 1 gegebenen Bedeutungen haben.

7. Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

8. Verwendung der Verbindungen I gemäß Anspruch 1 zur Herstellung eines zur Bekämpfung von tierischen Schädlingen oder Schadpilzen geeigneten Mittels.

9. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

10. Verfahren zur Bekämpfung von tierischen Schädlingen, **dadurch gekennzeichnet, daß** man die tierischen Schädlinge oder die vor ihnen zu schützenden Materialien, Pflanzen, den Boden oder Saatgüter mit einer wirksamen Menge einer Verbindung der allgemeinen Formel I gemäß Anspruch 1 behandelt.

## Claims

1. Benzyloxyimino compounds of the formula I, 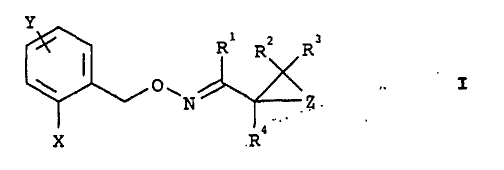 where:
X is N(COOCH₃)-OCH₃, C(COOCH₃)=CH-OCH₃, C(COOCH₃)=N-OCH₃, C(CONHCH₃)=N-OCH₃ or C(COOCH₃)=CH-CH₃;
Y is hydrogen, halogen, C₁-C₄-alkyl, C₁-C₄-haloalkyl or C₁-C₄-alkoxy;
Z is oxygen, C(R^{a})₂ or NR^{a}, where
R^{a} is hydrogen or C₁-C₄-alkyl and, in the case of C(R^{a})₂, can be identical or different;
R¹ is halogen, C₁-C₄-alkyl, C₁-C₄-haloalkyl or C₁-C₄-alkoxy;
R² is hydrogen, halogen, C₁-C₄-alkyl, C₁-C₄-alkoxy or is phenyl with or without substitution by customary groups;
R³ is hydrogen, halogen, C₁-C₄-alkyl or C₁-C₄-alkoxy;
R⁴ is C₁-C₄-alkyl or
phenyl with or without substitution by 1-3 substituents from the following group: halogen, C₁-C₄-alkyl, C₁-C₄-haloalkyl, C₁-C₄-alkoxy, C₁-C₄-haloalkoxy or C(R^{b})=N-OR^{c}, where
R^{b} is hydrogen or C₁-C₄-alkyl and
R^{c} is C₁-C₄-alkyl, C₃-C₄-alkenyl or C₃-C₄-alkynyl with the proviso that Z is not C(R^{a})₂ when X is C(COOCH₃)=CH-OCH₃ or C(COOCH₃)=N-OCH₃ and R⁴ is C₁-C₄-alkyl.

2. A process for preparing compounds of the formula I as claimed in claim 1 where Z is oxygen and CR^{a}, which comprises reacting an oxime of the formula II 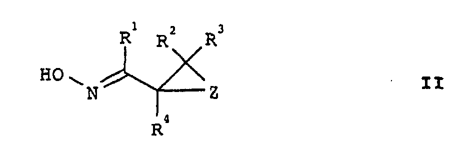 with a benzyl compound of the formula III, 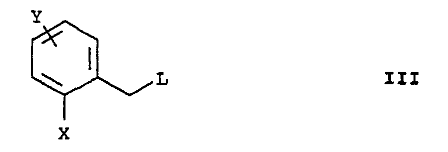 in which L is a nucleophilically replaceable group.

3. A process for preparing compounds of the formula I as claimed in claim 1 in which Z is oxygen, which comprises reacting a benzyl compound of the formula III as set forth in claim 2 with an oxime of the formula IV 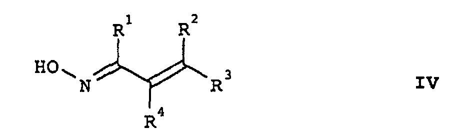 to give an oxime ether of the formula V 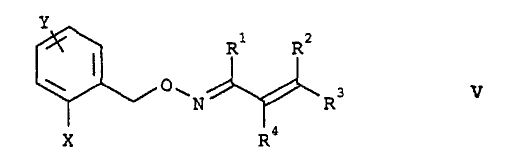 which is subsequently converted by oxidation into a compound of the formula I.

4. A process for preparing compounds of the formula I as claimed in claim 1 in which Z is NH, which comprises reacting compounds of the formula I in which Z is oxygen with metal azides.

5. A process for preparing compounds of the formula I as claimed in claim 1 in which Z is N-C₁-C₄-alkyl, which comprises reacting compounds of the formula I in which Z is NH with alkylating agents.

6. Intermediates of the formula II as set forth in claim 2 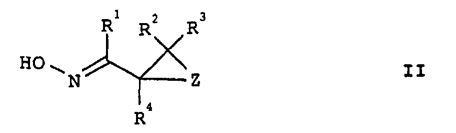 in which Z is oxygen or NR^{a} and the substituents R¹ to R⁴ are each as defined in claim 1.

7. A composition suitable for controlling animal pests or harmful fungi, comprising a solid or liquid carrier and a compound of the formula I as claimed in claim 1.

8. The use of the compounds I as claimed in claim 1 for preparing a composition suitable for controlling animal pests or harmful fungi.

9. A method for controling harmful fungi, which comprises treating the fungi or the materials, plants, the soil or seeds to be protected against attack by fungi with an effective amount of a compound of the formula I as claimed in claim 1.

10. A method for controling animal pests, which comprises treating the animal pests or the materials, plants, the soil or seeds to be protected against them with an effective amount of a compound of the formula I as claimed in claim 1.

## Revendications

1. Composés benzyloxyimino de formule I 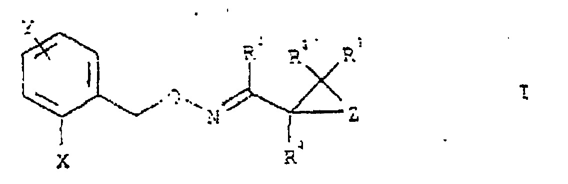 dans laquelle les substituants ont les significations suivantes :
x représente N(COOCH₃)-OCH₃, C(COOCH₃)=(CH-OCH₃), C(COOH₃)=N-OCH₃, C (CONHCH₃)=N-OCH₃ ou C(COOCH₃) =CH-CH₃ ;
Y représente un hydrogène, un halogène, un alkyle en C₁-C₄, un halogénoalkyle en C₁-C₄ ou un alcoxy en C₁-C₄ ;
z représente un oxygène, C(R^{a})₂ ou NR^{a}, où
R^{a} désigne un hydrogène ou un alkyle en C₁-C₄ et, dans le cas de C(R^{a})₂, peut être identique ou différent ;
R1 représente un halogène, un alkyle en C₁-C₄, un halogénoalkyle en C₁-C₄ ou un alcoxy en C₁-C₄ ;
R2 représente un hydrogène, un halogène, un alkyle en C₁-C₄, un alcoxy en C₁-C₄ ou un phényle éventuellement substitué par des groupes habituels ;
R³ représente un hydrogène, un halogène, un alkyle en C₁-C₄ ou un alcoxy en C₁-C₄;
R4 représente un alkyle en C₁-C₄ ou
un phényle portant éventuellement 1-3 substituants parmi le groupe suivant : un halogène, un alkyle en C₁-C₄, un halogénoalkyle en C₁-C₄, un alcoxy en C₁-C₄, un halogénoalcoxy en C₁-C₄ ou C(R^{b})=N-OR^{c}, où
R^{b} désigne un hydrogène ou un alkyle en C₁-C₄, et
R^{c} désigne un alkyle en C₁-C₄, un alcényle en C₃-C₄ ou un alcynyle en C₃-C₄,
où Z ne désigne pas C(R^{a})₂ si X représente C(COOCH₃)=CH-OCH₃ ou C(COOCH₃)=N-OCH₃ et R⁴ représente un alkyle en C₁-C₄.

2. Procédé de préparation de composés de formule (I) selon la revendication 1, dans laquelle Z désigne un oxygène et CR^{a}, **caractérisé en ce que** l'on fait réagir un oxime de formule II 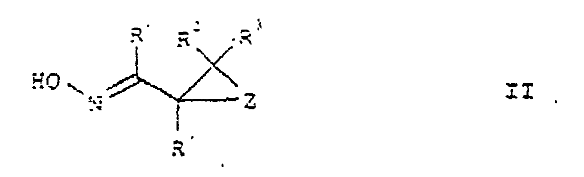 avec un composé benzylique de formule III, 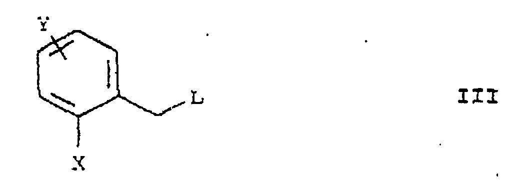 dans laquelle L désigne un groupe remplaçable de façon nucléophile.

3. Procédé de préparation de composés de formule I selon la revendication 1, dans laquelle Z désigne un oxygène, **caractérisé en ce que** l'on fait réagir un composé benzylique de formule III selon la revendication 2 avec un oxime de formule IV 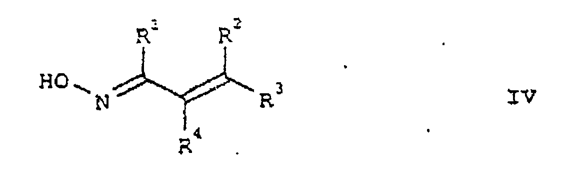 pour donner lieu à un oxime-éther de formule V 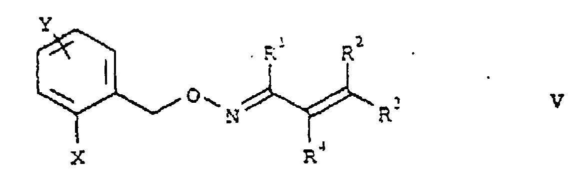 qui est ensuite transformé en un composé de formule I par oxydation.

4. Procédé de préparation de composés de formule I selon la revendication 1, dans laquelle Z désigne NH, **caractérisé en ce que** l'on fait réagir des composés de formule I, dans laquelle Z désigne un oxygène, avec des azotures métalliques.

5. Procédé de préparation de composés de formule I selon la revendication 1, dans laquelle Z désigne un N-C₁-C₄-alkyle, **caractérisé en ce que** l'on fait réagir des composés de formule I, dans laquelle Z désigne NH, avec des agents d'alkylation.

6. Intermédiaires de formule II selon la revendication 2, 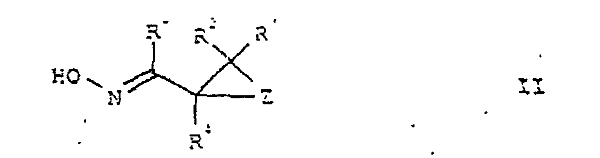 dans laquelle Z désigne un oxygène ou NR^{a} et les substituants R¹ à R⁴ ont les significations indiquées à la revendication 1.

7. Composition appropriée pour la lutte contre des parasites animaux ou des champignons nuisibles, comprenant un support solide ou liquide et un composé de formule générale I selon la revendication 1.

8. Utilisation des composés I selon la revendication 1 pour la préparation d'une composition appropriée pour la lutte contre des parasites animaux ou des champignons nuisibles.

9. Procédé de lutte contre des champignons nuisibles, **caractérisé en ce que** les champignons ou les matériaux, les plantes, les sols ou les semences à protéger vis-à-vis d'une infection fongique sont traités par une quantité efficace d'un composé de formule générale I selon la revendication 1.

10. Procédé de lutte contre des parasites animaux, **caractérisé en ce que** les parasites animaux ou les matériaux, les plantes, les sols ou les semences à protéger vis-à-vis de ceux-ci sont traités par une quantité efficace d'un composé de formule générale I selon la revendication 1.
